# EUROPEAN PATENT SPECIFICATION

(11) **EP 3 313 876 B1**
(45) Date of publication and mention of the grant of the patent: **22.01.2025**
(21) Application number: 16733394.7
(22) Date of filing: 23.06.2016
(51) Int. Cl.: C07K 16/00, C07K 16/28, C07K 16/46

(54) **MULTISPECIFIC ANTIGEN BINDING PROTEINS**
MULTISPEZIFISCHE ANTIGEN BINDENDE PROTEINE
PROTEINES MULTISPECIFIQUES LIANT DES ANTIGENES

(30) Priority: 23.06.2015 WO PCT/EP2015/064070; 28.12.2015 US 201562271491 P
(43) Date of publication of application: 02.05.2018
(73) Proprietor: Innate Pharma, 13009 Marseille (FR)
(72) Inventor: GAUTHIER, Laurent, 13008 Marseille (FR); ROSSI, Benjamin, 13008 Marseille (FR)
(74) Representative: Cabinet Becker et Associés
(86) International application number: PCT/EP2016/064528
(87) International publication number: WO 2016/207273

(56) References cited:
- WO-A1-2011/066501
- WO-A2-2005/000086
- WO-A2-2011/133886
- US-A1- 2010 256 338
- KONTERMANN ROLAND E: "Dual targeting strategies with bispecific antibodies", MABS, LANDES BIOSCIENCE, US, vol. 4, no. 2, 1 March 2012 (2012-03-01), pages 182 - 197, XP002698995, ISSN: 1942-0862, DOI: 10.4161/MABS.4.2.19000
- MATHIAS LINDH JØRGENSEN ET AL: "Expression of single-chain variable fragments fused with the Fc-region of rabbit IgG in Leishmania tarentolae", MICROBIAL CELL FACTORIES,, vol. 13, no. 1, 15 January 2014 (2014-01-15), pages 9, XP021175885, ISSN: 1475-2859, DOI: 10.1186/1475-2859-13-9
- ANNABELLE PATRICIA HERRINGTON-SYMES ET AL: "Antibody fragments: Prolonging circulation half-life special issue-antibody research", ADVANCES IN BIOSCIENCE AND BIOTECHNOLOGY, vol. 04, no. 05, 1 January 2013 (2013-01-01), pages 689 - 698, XP055282656, ISSN: 2156-8456, DOI: 10.4236/abb.2013.45090
- C. ROZAN ET AL: "Single-Domain Antibody-Based and Linker-Free Bispecific Antibodies Targeting Fc RIII Induce Potent Antitumor Activity without Recruiting Regulatory T Cells", MOLECULAR CANCER THERAPEUTICS, vol. 12, no. 8, 1 August 2013 (2013-08-01), pages 1481 - 1491, XP055156453, ISSN: 1535-7163, DOI: 10.1158/1535-7163.MCT-12-1012
- MULLER K M ET AL: "The first constant domain (CH1 and CL) of an antibody used as heterodimerization domain for bispecific miniantibodies", FEBS LETTERS, ELSEVIER, AMSTERDAM, NL, vol. 422, no. 2, 30 January 1998 (1998-01-30), pages 259 - 264, XP004261818, ISSN: 0014-5793, DOI: 10.1016/S0014-5793(98)00021-0
- KUFER P ET AL: "A revival of bispecific antibodies", TRENDS IN BIOTECHNOLOGY, ELSEVIER PUBLICATIONS, CAMBRIDGE, GB, vol. 22, no. 5, 1 May 2004 (2004-05-01), pages 238 - 244, XP004504363, ISSN: 0167-7799, DOI: 10.1016/J.TIBTECH.2004.03.006
- HOLLANDER NURIT: "Bispecific antibodies for cancer therapy", IMMUNOTHERAPY, FUTURE MEDICINE LTD, UK, vol. 1, no. 2, 1 March 2009 (2009-03-01), pages 211 - 222, XP001525697, ISSN: 1750-743X
- SHAN CHUNG ET AL: "Quantitative evaluation of fucose reducing effects in a humanized antibody on Fc[gamma] receptor binding and antibody-dependent cell-mediated cytotoxicity activities", MABS, vol. 4, no. 3, 1 May 2012 (2012-05-01), US, pages 326 - 340, XP055302095, ISSN: 1942-0862, DOI: 10.4161/mabs.19941
- ADAM W BARB ET AL: "NMR analysis demonstrates immunoglobulin G N-glycans are accessible and dynamic", NATURE CHEMICAL BIOLOGY, vol. 7, no. 3, 1 March 2011 (2011-03-01), GB, pages 147 - 153, XP055302088, ISSN: 1552-4450, DOI: 10.1038/nchembio.511

## Description

### FIELD OF THE INVENTION

Multispecific proteins that bind and can be used to specifically redirect effector cells to lyse a target cell of interest are provided. The proteins formats have utility in the treatment of disease.

### BACKGROUND

Bispecific antibodies binding two different epitopes and offer opportunities for increasing specificity, broadening potency, and utilizing novel mechanisms of action that cannot be achieved with a traditional monoclonal antibody. A variety of formats for bispecific antibodies that bind to two targets simultaneously have been reported. Cross-linking two different receptors using a bispecific antibody to inhibit a signaling pathway has shown utility in a number of applications (see, e.g., Jackman, et al., (2010) J. Biol. Chem. 285:20850-20859). Bispecific antibodies have also been used to neutralize two different receptors. In other approaches, bispecific antibodies have been used to recruit immune effector cells, where T-cell activation is achieved in proximity to tumor cells by the bispecific antibody which binds receptors simultaneously on the two different cell types (see Baeuerle, P. A., et al, (2009) Cancer Res 69(12):4941-4). Most such approaches involve bispecific antibodies that link the CD3 complex on T cells to a tumor-associated antigen. The most well-studied bispecific antibody formats are "BiTe" antibodies and "DART" antibodies which do not comprise Fc domains. However these antibodies are known to be difficult to produce, require lengthy cell development, have low productions yields and/or cannot be produced (based on published literature) as a homogenous protein composition. Notably, in order to fully activate a T-cell, the T-cell and a cluster of BiTEs must interact on the surface of a target cell. Due to the difficulties of finding antibody variable regions which are functional in the BiTE format, to date only a single immune cell receptor (CD3) has been targeted, in the CD19 x CD3 specific antibody blinatumamab. Bispecific antibodies developed to date also include those which link the CD3 complex on T cells to a tumor-associated antigen. In another example, a bispecific antibody having one arm which bound FcyRIII and another which bound to the HER2 receptor was developed for therapy of ovarian and breast tumors that overexpress the HER2 antigen.

However, despite the existence of a variety of formats for bispecific antibodies, there is therefore a need in the art for proteins with new and well-defined mechanisms of action that can bind two or more biological targets, and that have attractive properties for industrial development.

[D1] WO2011/133886 describes tetrameric antibodies in which both antigen binding domains are N-terminal to the Fc domain.

[D2] Kufer et al., 2004 Trends in Biotechnology 22(5): 238-244 describes Fab-based protein structures, including one format having an Fc domain and in which a problem of chain pairing and therefore of production aroses.

[D3] Rozan et al., 2013 Molecular Cancer Therapeutics 12(8): 1481-1491 discloses a bispecific antibody in which two single domain antibodies (sdAbs) derived from llamas are linked through a CH1 and CK domains pairing.

[D4] Müller et al., 1998 FEBS Letters 422(2): 259-264 describes a bispecific antibody in which two scFv are linked through a CH1 and CK domains pairing and a disulfide linkage.

[D5] Barb et al., 2011 Nature Chemical Biology 7(3): 147-153 discusses N297-linked glycosylation of antibodies.

[D6] Hollander et al., 2009 Immunotherapy 1(2):211-222 provides a review of different antibody formats.

[D7] Chung et al., 2012 mAbs 4(3): 326-340 discusses effect of fucose content on Fc gamma receptor binding.

[D8] WO2005/000086 describes tetradoma technology which results in a mixture of many species of antibody.

[D9] WO2011/066501 describes modified Fc domains.

[D10] Kontermann et al., 2012 mAbs 4:2; 182-197 reviews dual targeting strategies and provides an overview of the established bispecific antibody formats.

[D11] US 20100256338 discloses a full-length antibody where each heavy chain is connected at its CH3 domain to a single chain Fab.

[D12] Jorgensen et al., 2014 Microbial Cell Factories 13(9): 1-9 describes single chain variable fragments fused at their C-terminus to Fc regions of rabbit IgG.

[D13] Herrington-Symes et al. reviews recombinant and chemical modifications to improve the shortcomings of different antibody fragments.

### SUMMARY OF THE INVENTION

The present invention arises from the discovery of a functional protein format that permits a wide range of antibody variable regions to be readily used, having advantages in manufacturing by being adapted to standard recombinant production techniques and without the need for development of product-specific folding or purification technique. While the new protein formats can be used to bind any desired antigens by incorporation the desired variable regions, advantageous examples are provided where multispecific proteins can bind to one (or optionally two or three) antigens of interest on target cells (e.g. cells to be eliminated or depleted), and one, two or three immune effector cell activating receptors on immune cells (e.g. lymphocytes, NK cells, T cells, etc.), optionally where one of the activating receptors is human CD16A. In some examples, the proteins possess one antigen binding domain (ABD) formed by immunoglobulin variable regions thereby binding to a target antigen, and a dimeric Fc domain that comprises N-linked glycosylation and binds the activating receptor CD16A. In some examples, the proteins possess two antigen binding domains (ABDs) each formed by immunoglobulin variable regions, thereby binding to two antigens (e.g. different antigens), and a dimeric Fc domain that comprises N-linked glycosylation and binds the activating receptor CD16A; when such protein includes an ABD that binds an effector cell activating receptor other than CD16A, the protein will therefore bind to two effector cell activating receptors (i.e., CD16A and the effector cell activating receptor other than CD16A), thereby providing advantageous immune enhancing activity. Other exemplary multispecific proteins can possess three antigen binding domains formed by immunoglobulin variable regions; such protein can for example have one or two ABDs that bind a different effector cell activating receptor (e.g. which may or may not include CD16A), and one or two ABDs that binds a cancer antigen. When such a protein with three ABDs further include a dimeric Fc domain that comprises N-linked glycosylation and binds the activating receptor CD16A, the protein can for example have up to three ABDs that bind a cancer antigen, or one or two ABDs that bind a cancer antigen and one ABD that binds an effector cell activating receptor other than CD16A. Exemplary multispecific proteins can thus bind three antigens, wherein the antigens may be the same or different.

The present invention relates to the subject-matter defined in the appended claims.

The proteins are made of different polypeptide chains that each comprise at least one heavy or light chain variable domain fused to a human CH1 or Cκ constant domain (a V-(CH1/Cκ) unit), wherein the protein chains undergo CH1-Cκ dimerization and are bound to one another by non-covalent bonds and optionally further by disulfide bonds formed between respective CH1 and Cκ domain. Two of the chains comprise Fc domains, such that a dimeric Fc domain is formed.

In one aspect, provided is an isolated or purified heterotimeric protein that binds a first and second antigen, wherein the protein comprises three polypeptide chains each comprising a different V-(CH1/Cκ) unit, wherein two of chains further comprise an Fc domain fused to the C-terminus of the V-(CH1/Cκ) unit, whereby the chains are bound to one another by non-covalent bonds and optionally further by disulfide bonds between CH1 and Cκ domains, optionally, whereby the chains are further bound by non-covalent bonds between respective variable regions, CH1 and Cκ domains.

The variable and constant regions are selected and configured such that each chain will preferentially associate with its desired complementary partner chain. The resulting multimeric protein will therefore be simple to produce using conventional production methods using recombinant host cells. The choice of which VH, VL to associate with a CH1 and Cκ in a unit is based on affinity between the units to be paired so as to drive the formation of the desired multimer. The resulting multimer will be bound by non-covalent bonds between complementary VH and VL domains, by non-covalent bonds between complementary CH1 and Cκ domains, and optionally disulfide bonding between complementary CH1 and Cκ domains (and/or optionally further disulfide bonds between complementary hinge domains). VH-VL associations are stronger than VH-VH or VL-VL, consequently, as shown herein, one can place a VH or a VL next to either a CH1 or a Cκ, and the resulting V-C unit will partner preferably with its V-C counterpart. For example VH-Cκ will pair with VL-CH1 preferentially over VH-CH1. Additionally, by including an Fc domain, preferred chain pairing is further improved, as the two Fc-containing chains will be bound by non-covalent bonds between CH3 domains of the Fc domains. The different V-C combinations, further combined with Fc pairing thereby provides tools to make heteromultimeric proteins.

In one example, a multispecific protein is provided that binds to three antigens, wherein one of the antigens is human CD16. In one aspect, the protein comprises a first and a second polypeptide chain each comprising a variable domain fused to a CH1 or Cκ domain (a V-(CH1/Cκ) unit), in turn fused at its C-terminus to a human Fc domain, wherein the V-(CH1/Cκ) unit of the first chain has undergone CH1-Cκ dimerization with the V-(CH1/Cκ) unit of the second chain thereby forming a first antigen binding domain (ABD₁) and a dimeric Fc domain, wherein one of the polypeptide chains further comprises an antigen binding domain that forms a second antigen binding domain (ABD₂), and wherein the Fc domain binds to a human CD16 polypeptide. The Fc domain comprises N-linked glycosylation at residue N297 (Kabat EU numbering).

In one example, a multispecific protein is provided that binds to three antigens, wherein one of the antigens is human CD16. In one aspect, the protein comprises three polypeptide chains, each comprise a variable domain fused to a CH1 or Cκ domain (a V-(CH1/Cκ) unit), wherein a first (central) chain comprises two V-(CH1/Cκ) units and a human Fc domain interposed between the units, the second chain comprises one V-(CH1/Cκ) unit and a human Fc domain, and the third chain comprises one V-(CH1/Cκ) unit, wherein one of the V-(CH1/Cκ) units of the central chain has undergone CH1-Cκ dimerization with the V-(CH1/Cκ) unit of the second chain thereby forming a first antigen binding domain (ABD₁) and a dimeric Fc domain, and wherein the other of the V-(CH1/Cκ) units of the central chain has undergone CH1-Cκ dimerization with the V-(CH1/Cκ) unit of the third chain thereby forming a second antigen binding domain (ABD₂), and wherein the Fc domain binds to a human CD16 polypeptide. The Fc domain comprises N-linked glycosylation at residue N297 (Kabat EU numbering). In one example, the protein has a domain arrangement:

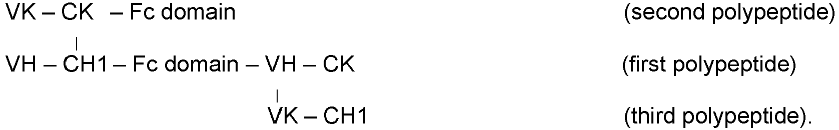

The central chain comprises an Fc domain interposed between the two V-(CH1/Cκ) units. The second or third polypeptide comprises an Fc domain, wherein the Fc domain is placed at the C-terminus of a V-(CH1/Cκ) unit in the second or third chain, wherein the Fc domains of the central chain and the Fc domain of the second or third chain associate within the heteromultimeric protein to form a dimeric Fc domain. In one aspect, the dimeric Fc domain binds human FcRn and human CD16 polypeptide. The Fc domain comprises N-linked glycosylation at residue N297 (Kabat EU numbering).

When a V-(CH1/Cκ) unit of one chain has undergone dimerization with a V-(CH1/Cκ) unit of another chain, the units will be bound by non-covalent bonds and optionally further by disulfide bond(s) between respective CH1 and Cκ domains (and further non-covalent bonds, as discussed above). The variable (V) domains and CH1/Cκ will be selected are configured such that each complementary pair of V-(CH1/Cκ) units collectively comprises one VH, one VL, one CH1 and one Cκ domain.

The multimeric polypeptide is composed of 3 different polypeptide chains in which 2 chains dimerize with a central chain based on CH1-CK heterodimerization. The multimer may be composed of a central (first) polypeptide chain comprising two immunoglobulin variable domains that are part of separate antigen binding domains (e.g., of different antigen specificities), with an Fc domain interposed between the two immunoglobulin variable domains on the polypeptide chain, and a CH1 or CK constant domain placed on the polypeptide chain adjacent to one of, or each of, the variable domain. A second additional polypeptide chain will then be configured which will comprise a first immunoglobulin variable domain and a CH1 or CK constant region selected so as to permit CH1-CK heterodimerization with the central polypeptide chain; the immunoglobulin variable domain will be selected so as to complement the variable domain of the central chain that is adjacent to the CH1 or CK domain, whereby the complementary variable domains form an antigen binding domain for a first antigen of interest. The antigen binding domain for the second and third antigens of interest can then be formed according to several configurations.

Provided in one aspect are multimeric proteins that bind specifically to three antigens of interest (where the antigens may be the same or different), comprising a central (first) polypeptide chain comprising at least two variable domains that are part of different antigen binding domains, a CH1 or Cκ constant region fused to the C-terminus of one of the variable domains (thereby forming a V-(CH1/Cκ) unit), and an Fc domain interposed between the two variable domains; and a second and/or third polypeptide chain that each comprise at least one V-(CH1/Cκ) unit, wherein the variable domain and CH1 or Cκ constant region of the V-(CH1/Cκ) unit of the second polypeptide chain (and, if present, third polypeptides) are complementary to the V and CH1 or Cκ constant region of the first polypeptide chain (but not to the V and CH1 or Cκ of the other of the second or third chain) such that the second (and, if present third polypeptide) chains preferentially form a CH1- Cκ heterodimer with the central chain, thereby forming a heterotrimer. The CH1-Cκ heterotrimers will be characterized by non-covalent bonds and optionally further by disulfide bond(s) formed between respective CH1 and Cκ domains). When the second polypeptide comprises an Fc domain (and where the CH1/Cκ - Fc domain comprise hinge domains), the protein can optionally further be characterized by a disulfide bond formed between hinge domains.

The multimeric, multispecific protein comprises a dimeric Fc domain that binds a human CD16A polypeptide.

Furthermore, despite that the subject multispecific proteins are bound by CD16, unexpectedly they do not induce or increase down-modulation or internalization of the antigen of interest, even when targeting antigens of interest known to be susceptible to down-modulation or internalization when bound by conventional antibodies (such as full length human IgG1's). Based thereon, the subject multispecific proteins should be well suited for targeting antigens of interest expressed by target cells, e.g., tumor or infected cells, including antigens which are known to be capable of undergoing down-modulation or internalization when bound by conventional antibodies (e.g. antibodies with human IgG1 Fc domains that retain CD16 binding). This is a huge therapeutic benefit since it is known in the art that antigen internalization can substantially impede the ability of conventional human IgG1 antibodies to mediate ADCC against a target cell. Thus, in one aspect, a multispecific protein (or an ABD thereof) binds an antigen expressed by target cell that is known to internalize upon binding to a conventional antibody (e.g. monoclonal monospecific human IgG1), wherein the multispecific protein causes less (or does not cause) induction or increase in internalization of the antigen compared to a conventional antibody.

The multispecific antibody are designed to bind to human CD16 and therefore can mediate target cell lysis via CD16, optionally in addition to other activating receptors on an effector cell.

Optionally in any aspect, fusions or linkages on the same polypeptide chain between different domains (e.g., between V domains and CH1 or Cκ domains, between CH1 or Cκ domains and Fc domains, between Fc domains and V domains) may occur via intervening amino acid sequences, for example via a hinge region or linker peptide.

Optionally in any aspect, one or two of the antigens of interest is a cancer antigen, viral antigen or bacterial antigen, and one or two of the antigens of interest is a polypeptide expressed on the surface of an immune effector cell.

In one aspect provided is a protein as described herein for use for the treatment of cancer.

### BRIEF DESCRIPTION OF THE FIGURES

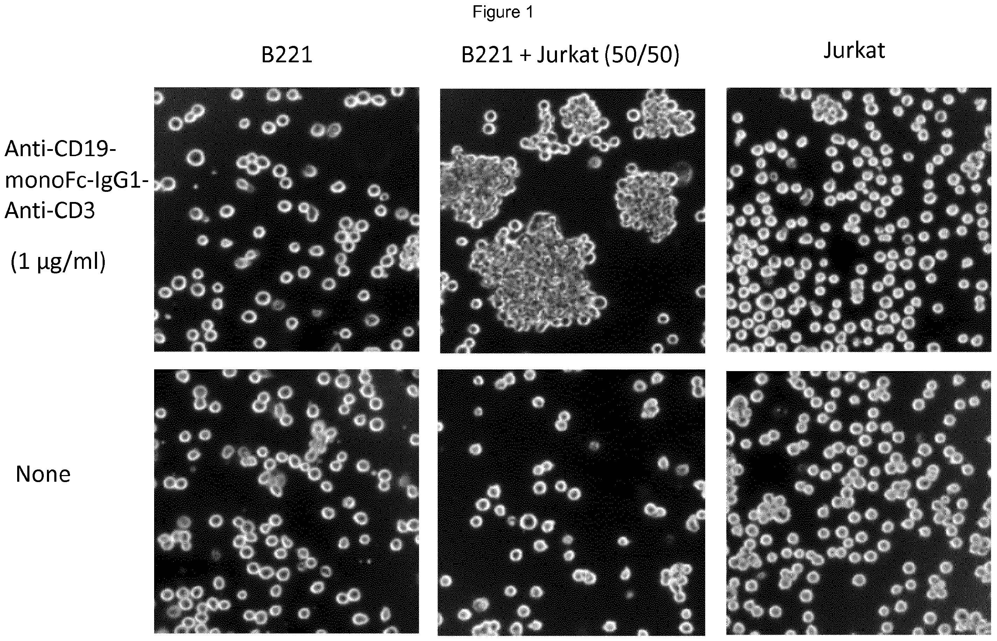

**Figure 1** shows that Anti-CD19-F1-Anti-CD3 does not cause T/B cell aggregation in the presence of B221 (CD19) or JURKAT (CD3) cell lines when separate, but it does cause aggregation of cells when both B221 and JURKAT cells are co-incubated.

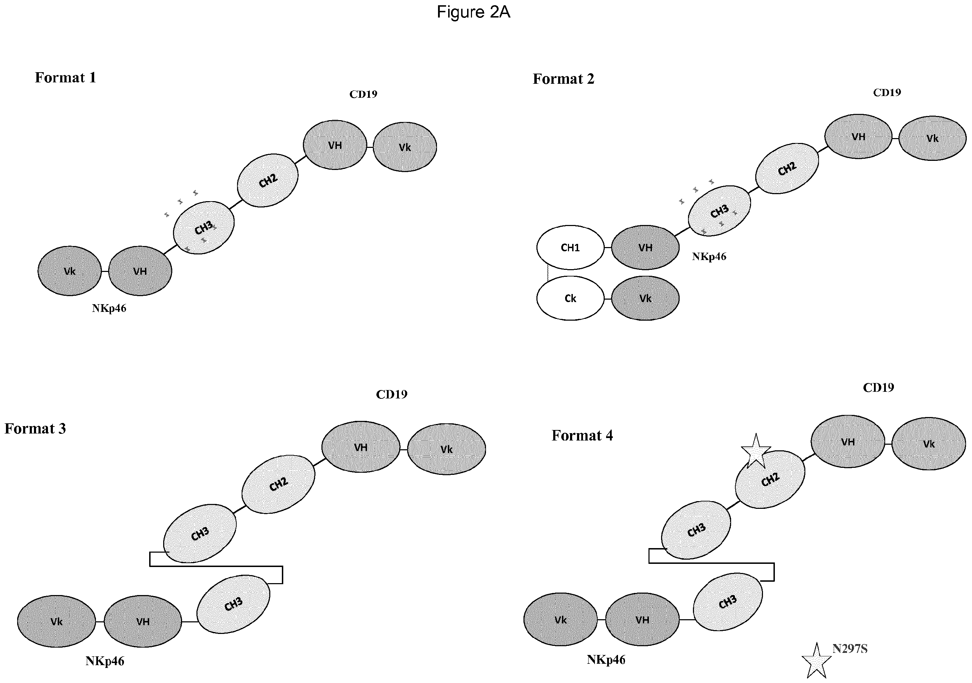

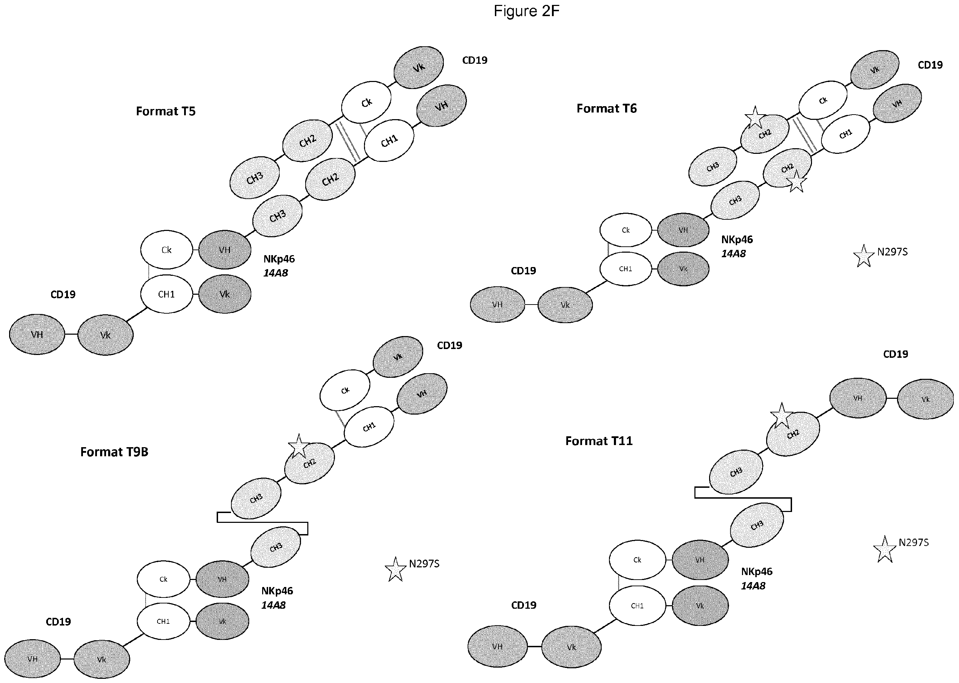

**Figures 2A** to **2E** show different domain arrangements of bispecific proteins produced. Figure 2F shows different domain arrangements proteins with three immunogloblin ABDs.

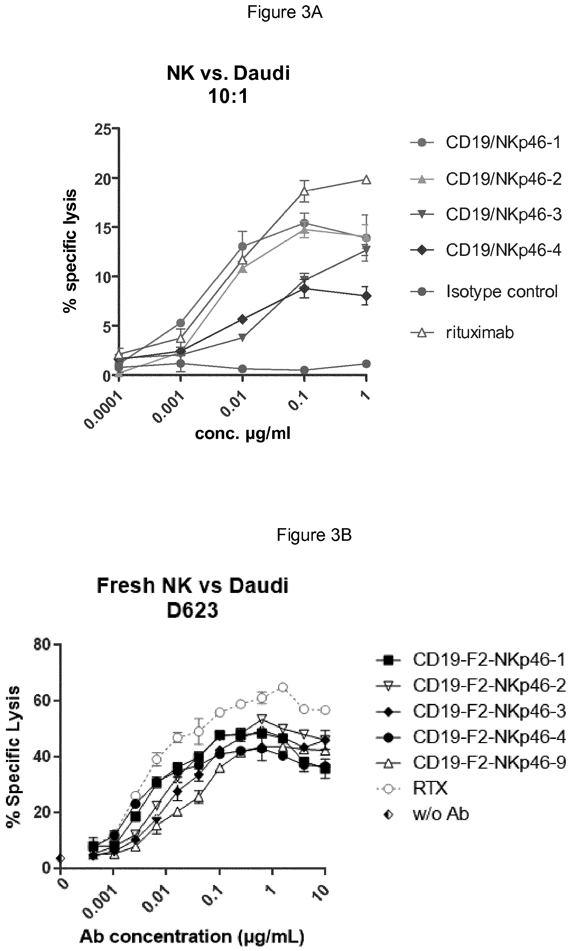

**Figures 3A** and **3B** respectively demonstrate that bispecific F1 and F2 format proteins having NKp46 binding region based on NKp46-1, NKp46-2, NKp46-3 or NKp46-4 are able to direct resting NK cells to their CD19-positive Daudi tumor target cells, while isotype control antibody did not lead to the elimination of the Daudi cells. Rituximab (RTX) served as the positive control of ADCC, where the maximal response obtained with RTX (at 10 µg/ml in this assay) was 21.6% specific lysis.

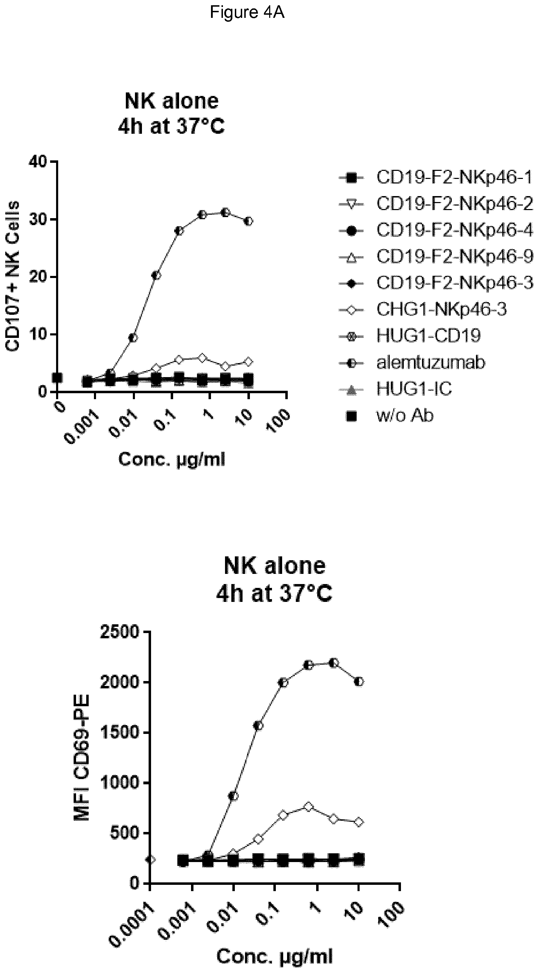

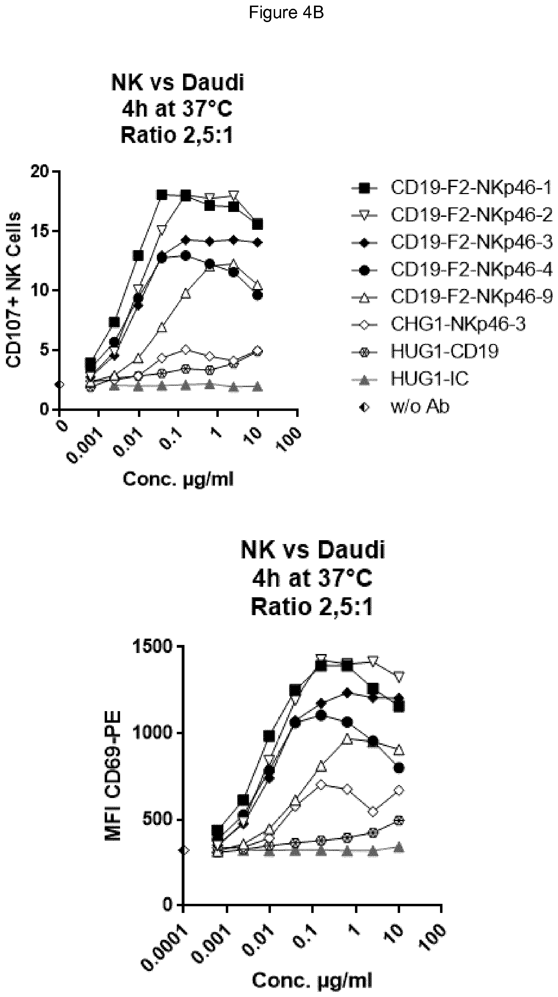

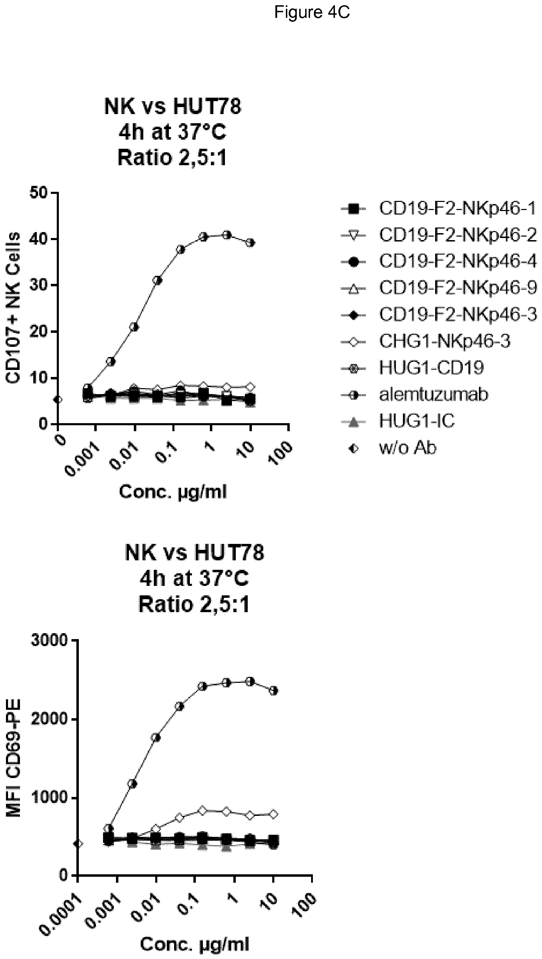

**Figure 4A** shows that bispecific antibodies having NKp46 and CD19 binding regions in an F2 format protein do not activate resting NK cells in the absence of target cells; by contrast full length anti-NKp46 antibodies as well as positive control alemtuzumab did activate NK cells. **Figure 4B** shows that bispecific anti-NKp46 x anti-CD19 antibodies (including each of the NKp46-1, NKp46-2, NKp46-3 or NKp46-4 binding domains) activated resting NK cells in presence of Daudi target cells, while full-length anti-CD19 showed at best only very low activation of NK cells and neither full-length anti-NKp46 antibodies nor alemtuzumab elicited a substantial increase in activation beyond what was observed in the presence of NK cells alone. **Figure 4C** shows that in the presence of CD19-negative HUT78 cells, none of the bispecific anti-NKp46 x anti-CD19 antibodies (including each of the NKp46-1, NKp46-2, NKp46-3 or NKp46-4 variable regions) activated NK cells. However, the full-length anti-NKp46 antibodies and alemtuzumab resulted in detectable activation of NK cells, i.e., at a similar level observed in presence of NK cells alone. Isotype control antibody did not induce activation.

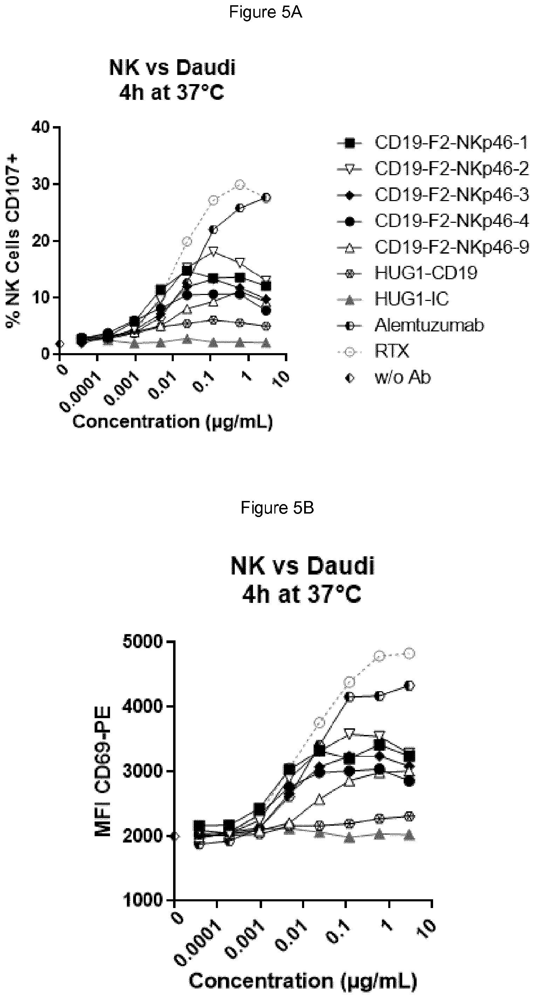

**Figures 5A** and **5B** shows that at low effector:target ratios of 1:1 each of the tested bispecific anti-NKp46 x anti-CD19 antibodies activated NK cells in the presence of Daudi cells, and that bispecific anti-NKp46 x anti-CD19 antibodies were far more potent (better elicited lysis of target cells) than a control anti-CD19 antibody as well as a full-length human IgG1 ADCC inducing antibody.

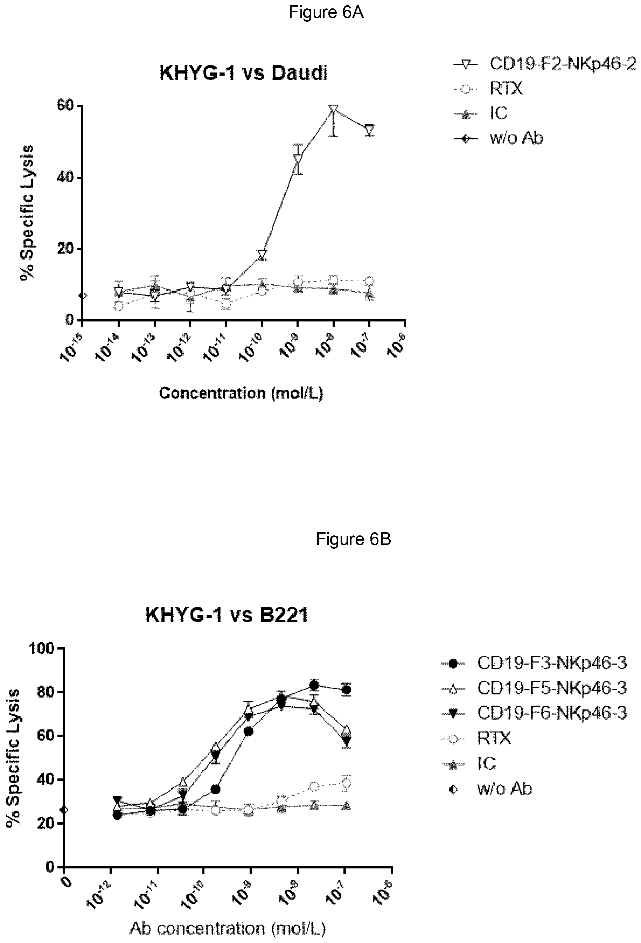

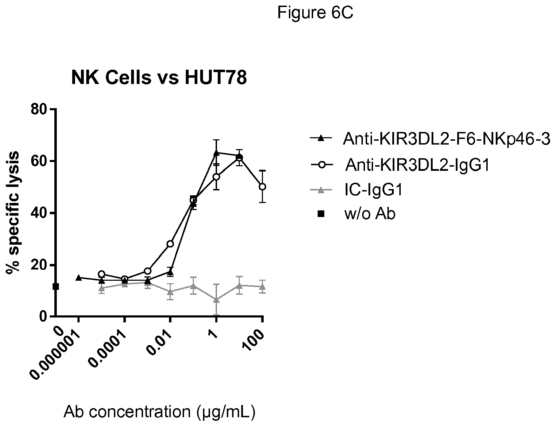

**Figures 6A** and **6B** show that each NKp46 x CD19 bispecific protein (Format F3, F5 and F6) induced specific lysis of Daudi (Figure 6A) or B221 (Figure 6B) cells by human KHYG-1 CD16-negative hNKp46-positive NK cell line, while rituximab and human IgG1 isotype control (IC) antibodies did not. **Figure 6C** shows that a NKp46 x KIR3DL2 bispecific protein (Format F6) induced specific lysis of HUT78 tumor cells via NKp46 binding (without CD16 binding) comparably to a conventional IgG1 antibody with the same anti-KIR3DL2 variable regions.

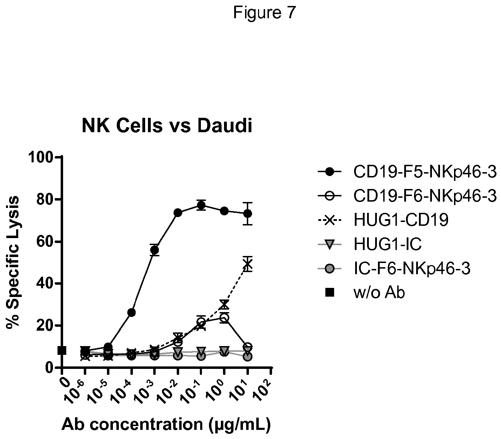

**Figure 7** shows a NKp46 x CD19 bispecific protein in F5 format whose Fc domain binds CD16 is far more potent in mediating Daudi target cell lysis than a full-length IgG1 anti-CD19 antibody or a F6 format bispecific protein. The figure also shows that a bispecific anti-CD19 in F6 format whose Fc domain does not bind CD16 was as potent in mediating NK cell lysis of Daudi target cells as the full-length IgG1 anti-CD19 antibody, which is unexpected considering that the control IgG1 anti-CD19 antibody binds CD19 bivalently. At comparable levels of target cell lysis, CD19-F5-NKp46-3 was at least 1000 times more potent than the full-length anti-CD19 IgG1.

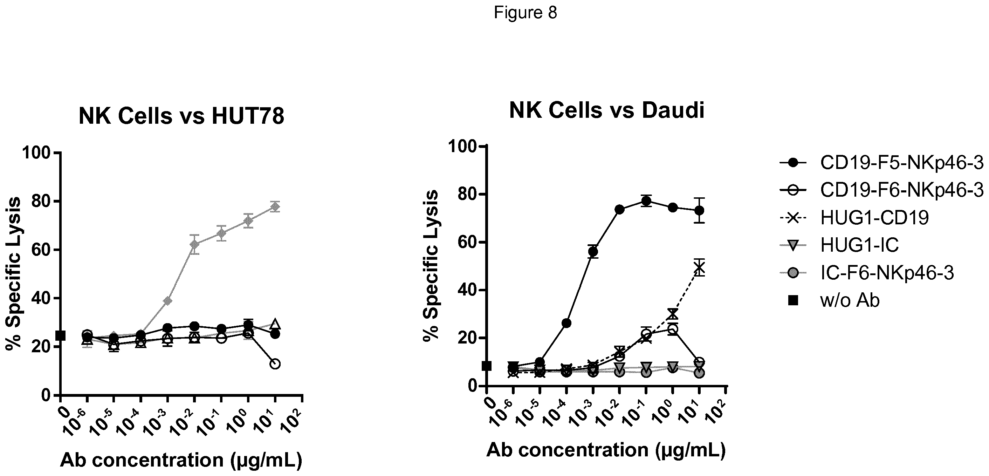

**Figure 8** shows the results of cytotoxicity assays using fresh NK cells (Daudi cell in the right hand panel and HUT78 cells in the left hand panel); the CD19-F6-NKp46-3 whose Fc domain does not bind CD16 due to a N297 substitution has as mode of action NKp46 triggering when NK cells encounter the target cell, while the CD19-F5-NKp46-3 bispecific protein demonstrated a far higher potency in mediating cytotoxicity toward Daudi cells. Neither the F5 nor F6 proteins mediated any NK cell cytotoxicity towards HUT78 cells.

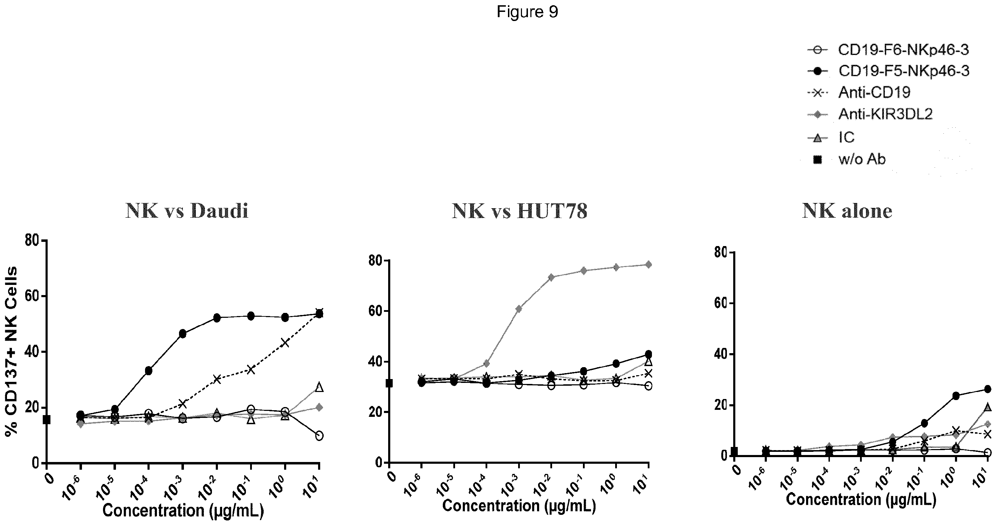

**Figure 9** shows the results of flow cytometry staining of NK cell surface markers showed a strong upregulation of CD137 on the surface of NK cells by F5 proteins (Left-most panel: NK cells vs. Daudi; middle panel: NK cells vs. HUT78; right-most panel: NK cells alone). The full-length anti-CD19 IgG1 antibody that binds CD16 also showed CD137 upregulation, but to a far lesser extent than the CD19-F5-NKp46-3 protein. The CD19-F6-NKp46-3 which functions via NKp46 but not CD16 did not show any CD137 upregulation.

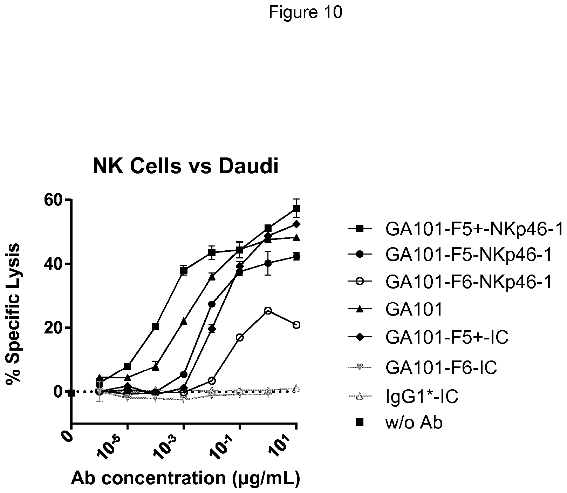

Figure 10 shows the results of cytotoxicity assays which compared the ability of the GA101-F5+-NKp46-1 bispecific protein to a comparison antibody (GA101) containing the same variable regions to lyse Daudi cells. The results therein show that the GA101-F5⁺-NKp46-1 bispecific protein possesses far higher potency (approximately 10-fold increase in EC₅₀) in mediating cytotoxicity toward Daudi cells than GA101.

### DETAILED DESCRIPTION OF THE INVENTION

### Definitions

As used in the specification, "a" or "an" may mean one or more. As used in the claim(s), when used in conjunction with the word "comprising", the words "a" or "an" may mean one or more than one.

Where "comprising" is used, this can optionally be replaced by "consisting essentially of", more optionally by "consisting of'.

As used herein, the term "antigen binding domain" or "ABD" refers to a domain comprising a three-dimensional structure capable of immunospecifically binding to an epitope. Thus, in one aspect, said domain can comprise a hypervariable region, optionally a VH and/or VL domain of an antibody chain, optionally at least a VH domain. In another aspect, the binding domain may comprise at least one complementarity determining region (CDR) of an antibody chain.

The term "antibody" herein is used in the broadest sense and specifically includes full-length monoclonal antibodies, polyclonal antibodies, multispecific antibodies (e.g., bispecific antibodies), and antibody fragments and derivatives, so long as they exhibit the desired biological activity. Various techniques relevant to the production of antibodies are provided in, e.g., Harlow, et al., ANTIBODIES: A LABORATORY MANUAL, Cold Spring Harbor Laboratory Press, Cold Spring Harbor, N.Y., (1988). An "antibody fragment" comprises a portion of a full-length antibody, e.g. antigen-binding or variable regions thereof. Examples of antibody fragments include Fab, Fab', F(ab)₂, F(ab')₂, F(ab)₃, Fv (typically the VL and VH domains of a single arm of an antibody), single-chain Fv (scFv), dsFv, Fd fragments (typically the VH and CH1 domain), and dAb (typically a VH domain) fragments; VH, VL, VhH, and V-NAR domains; minibodies, diabodies, triabodies, tetrabodies, and kappa bodies (see, e.g., III et al., Protein Eng 1997;10: 949-57); camel IgG; IgNAR; and multispecific antibody fragments formed from antibody fragments, and one or more isolated CDRs or a functional paratope, where isolated CDRs or antigen-binding residues or polypeptides can be associated or linked together so as to form a functional antibody fragment. Various types of antibody fragments have been described or reviewed in, e.g., Holliger and Hudson, Nat Biotechnol 2005; 23, 1126-1136; WO2005040219, and published U.S. Patent Applications 20050238646 and 20020161201.

The term "antibody derivative", as used herein, comprises a full-length antibody or a fragment of an antibody, e.g. comprising at least antigen-binding or variable regions thereof, wherein one or more of the amino acids are chemically modified, e.g., by alkylation, PEGylation, acylation, ester formation or amide formation or the like. This includes, but is not limited to, PEGylated antibodies, cysteine-PEGylated antibodies, and variants thereof.

The term "hypervariable region" when used herein refers to the amino acid residues of an antibody that are responsible for antigen binding. The hypervariable region generally comprises amino acid residues from a "complementarity-determining region" or "CDR" (e.g. residues 24-34 (L1), 50-56 (L2) and 89-97 (L3) in the light-chain variable domain and 31-35 (H1), 50-65 (H2) and 95-102 (H3) in the heavy-chain variable domain; Kabat et al. 1991) and/or those residues from a "hypervariable loop" (e.g. residues 26-32 (L1), 50-52 (L2) and 91-96 (L3) in the light-chain variable domain and 26-32 (H1), 53-55 (H2) and 96-101 (H3) in the heavy-chain variable domain; Chothia and Lesk, J. Mol. Biol 1987;196:901-917). Typically, the numbering of amino acid residues in this region is performed by the method described in Kabat et al., supra. Phrases such as "Kabat position", "variable domain residue numbering as in Kabat" and "according to Kabat" herein refer to this numbering system for heavy chain variable domains or light chain variable domains. Using the Kabat numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (residue 52a according to Kabat) after residue 52 of CDR H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

By "framework" or "FR" residues as used herein is meant the region of an antibody variable domain exclusive of those regions defined as CDRs. Each antibody variable domain framework can be further subdivided into the contiguous regions separated by the CDRs (FR1, FR2, FR3 and FR4).

By "constant region" as defined herein is meant an antibody-derived constant region that is encoded by one of the light or heavy chain immunoglobulin constant region genes. By "constant light chain" or "light chain constant region" as used herein is meant the region of an antibody encoded by the kappa (Cκ) or lambda (C 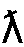 ) light chains. The constant light chain typically comprises a single domain, and as defined herein refers to positions 108-214 of Cκ, or C 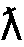 , wherein numbering is according to the EU index (Kabat et al., 1991, Sequences of Proteins of Immunological Interest, 5th Ed., United States Public Health Service, National Institutes of Health, Bethesda). By "constant heavy chain" or "heavy chain constant region" as used herein is meant the region of an antibody encoded by the mu, delta, gamma, alpha, or epsilon genes to define the antibody's isotype as IgM, IgD, IgG, IgA, or IgE, respectively. For full length IgG antibodies, the constant heavy chain, as defined herein, refers to the N-terminus of the CH1 domain to the C-terminus of the CH3 domain, thus comprising positions 118-447, wherein numbering is according to the EU index.

By "Fab" or "Fab region" as used herein is meant the polypeptide that comprises the VH, CH1, VL, and CL immunoglobulin domains. Fab may refer to this region in isolation, or this region in the context of a polypeptide, multispecific polypeptide or ABD, or any other aspects as outlined herein.

By "single-chain Fv" or "scFv" as used herein are meant antibody fragments comprising the VH and VL domains of an antibody, wherein these domains are present in a single polypeptide chain. Generally, the Fv polypeptide further comprises a polypeptide linker between the VH and VL domains which enables the scFv to form the desired structure for antigen binding. Methods for producing scFvs are well known in the art. For a review of methods for producing scFvs see Pluckthun in The Pharmacology of Monoclonal Antibodies, vol. 113, Rosenburg and Moore eds. Springer-Verlag, New York, pp. 269-315 (1994).

By "Fv" or "Fv fragment" or "Fv region" as used herein is meant a polypeptide that comprises the VL and VH domains of a single antibody.

By "Fc" or "Fc region", as used herein is meant the polypeptide comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM, Fc may include the J chain. For IgG, Fc comprises immunoglobulin domains Cγ2 (CH2) and Cγ3 (CH3) and the hinge between Cγ1 and Cγ2. Although the boundaries of the Fc region may vary, the human IgG heavy chain Fc region is usually defined to comprise residues C226, P230 or A231 to its carboxyl-terminus, wherein the numbering is according to the EU index. Fc may refer to this region in isolation, or this region in the context of an Fc polypeptide, as described below. By "Fc polypeptide" or "Fc-derived polypeptide" as used herein is meant a polypeptide that comprises all or part of an Fc region. Fc polypeptides include but are not limited to antibodies, Fc fusions and Fc fragments. Also, Fc regions according to the invention include variants containing at least one modification that alters (enhances or diminishes) an Fc associated effector function. Also, Fc regions according to the invention include chimeric Fc regions comprising different portions or domains of different Fc regions, e.g., derived from antibodies of different isotype or species.

By "variable region" as used herein is meant the region of an antibody that comprises one or more Ig domains substantially encoded by any of the VL (including Vκ and VA) and/or VH genes that make up the light chain (including κ and A) and heavy chain immunoglobulin genetic loci respectively. A light or heavy chain variable region (VL and VH) consists of a "framework" or "FR" region interrupted by three hypervariable regions referred to as "complementarity determining regions" or "CDRs". The extent of the framework region and CDRs have been precisely defined, for example as in Kabat (see "Sequences of Proteins of Immunological Interest," E. Kabat et al., U.S. Department of Health and Human Services, (1983)), and as in Chothia. The framework regions of an antibody, that is the combined framework regions of the constituent light and heavy chains, serves to position and align the CDRs, which are primarily responsible for binding to an antigen.

The term "specifically binds to" means that an antibody or polypeptide can bind preferably in a competitive binding assay to the binding partner, as assessed using either recombinant forms of the proteins, epitopes therein, or native proteins present on the surface of isolated target cells. Competitive binding assays and other methods for determining specific binding are further described below and are well known in the art.

The term "affinity", as used herein, means the strength of the binding of an antibody or polypeptide to an epitope. The affinity of an antibody is given by the dissociation constant K_{D}, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant K_{A} is defined by 1/K_{D}. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One preferred and standard method well known in the art for determining the affinity of mAbs is the use of surface plasmon resonance (SPR) screening (such as by analysis with a BIAcore^{™} SPR analytical device).

By "amino acid modification" herein is meant an amino acid substitution, insertion, and/or deletion in a polypeptide sequence. An example of amino acid modification herein is a substitution. By "amino acid modification" herein is meant an amino acid substitution, insertion, and/or deletion in a polypeptide sequence. By "amino acid substitution" or "substitution" herein is meant the replacement of an amino acid at a given position in a protein sequence with another amino acid. For example, the substitution Y50W refers to a variant of a parent polypeptide, in which the tyrosine at position 50 is replaced with tryptophan. A "variant" of a polypeptide refers to a polypeptide having an amino acid sequence that is substantially identical to a reference polypeptide, typically a native or "parent" polypeptide. The polypeptide variant may possess one or more amino acid substitutions, deletions, and/or insertions at certain positions within the native amino acid sequence.

"Conservative" amino acid substitutions are those in which an amino acid residue is replaced with an amino acid residue having a side chain with similar physicochemical properties. Families of amino acid residues having similar side chains are known in the art, and include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. 12, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

An "isolated" molecule is a molecule that is the predominant species in the composition wherein it is found with respect to the class of molecules to which it belongs (i.e., it makes up at least about 50% of the type of molecule in the composition and typically will make up at least about 70%, at least about 80%, at least about 85%, at least about 90%, at least about 95%, or more of the species of molecule, e.g., peptide, in the composition). Commonly, a composition of a polypeptide will exhibit 98%, 98%, or 99% homogeneity for polypeptides in the context of all present peptide species in the composition or at least with respect to substantially active peptide species in the context of proposed use.

In the context herein, "treatment" or "treating" refers to preventing, alleviating, managing, curing or reducing one or more symptoms or clinically relevant manifestations of a disease or disorder, unless contradicted by context. For example, "treatment" of a patient in whom no symptoms or clinically relevant manifestations of a disease or disorder have been identified is preventive or prophylactic therapy, whereas "treatment" of a patient in whom symptoms or clinically relevant manifestations of a disease or disorder have been identified generally does not constitute preventive or prophylactic therapy.

The term "internalization", used interchangeably with "intracellular internalization", refers to the molecular, biochemical and cellular events associated with the process of translocating a molecule from the extracellular surface of a cell to the intracellular surface of a cell. The processes responsible for intracellular internalization of molecules are well-known and can involve, *inter alia,* the internalization of extracellular molecules (such as hormones, antibodies, and small organic molecules); membrane-associated molecules (such as cell-surface receptors); and complexes of membrane-associated molecules bound to extracellular molecules (for example, a ligand bound to a transmembrane receptor or an antibody bound to a membrane-associated molecule). Thus, "inducing and/or increasing internalization" refers to events wherein intracellular internalization is initiated and/or the rate and/or extent of intracellular internalization is increased.

As used herein, the phrase "NK cells" refers to a sub-population of lymphocytes that is involved in non-conventional immunity. NK cells can be identified by virtue of certain characteristics and biological properties, such as the expression of specific surface antigens including CD56 and/or NKp46 for human NK cells, the absence of the alpha/beta or gamma/delta TCR complex on the cell surface, the ability to bind to and kill cells that fail to express "self" MHC/HLA antigens by the activation of specific cytolytic machinery, the ability to kill tumor cells or other diseased cells that express a ligand for NK activating receptors, and the ability to release protein molecules called cytokines that stimulate or inhibit the immune response. Any of these characteristics and activities can be used to identify NK cells, using methods well known in the art. Any subpopulation of NK cells will also be encompassed by the term NK cells. Within the context herein "active" NK cells designate biologically active NK cells, including NK cells having the capacity of lysing target cells or enhancing the immune function of other cells. NK cells can be obtained by various techniques known in the art, such as isolation from blood samples, cytapheresis, tissue or cell collections, etc. Useful protocols for assays involving NK cells can be found in Natural Killer Cells Protocols (edited by Campbell KS and Colonna M). Humana Press, pp. 219-238 (2000).

As used herein, "T cells" refers to a sub-population of lymphocytes that mature in the thymus, and which display, among other molecules T cell receptors on their surface. T cells can be identified by virtue of certain characteristics and biological properties, such as the expression of specific surface antigens including the TCR, CD4 or CD8, the ability of certain T cells to kill tumor or infected cells, the ability of certain T cells to activate other cells of the immune system, and the ability to release protein molecules called cytokines that stimulate or inhibit the immune response. Any of these characteristics and activities can be used to identify T cells, using methods well known in the art. Within the context herein, "active" or "activated" T cells designate biologically active T cells, more particularly T cells having the capacity of cytolysis or of stimulating an immune response by, e.g., secreting cytokines. Active cells can be detected in any of a number of well-known methods, including functional assays and expression-based assays such as the expression of cytokines such as TNF-alpha.

As used herein, an agent that has "agonist" activity at a cell surface receptor (e.g. an activating receptor) is an agent that can cause or increase signalling by the receptor, e.g., an ability of the receptor to activate or transduce an intracellular signaling pathway. Changes in signaling activity can be measured, for example, by assays designed to measure changes in receptor signaling pathways, e.g. by monitoring phosphorylation of signal transduction components, assays to measure the association of certain signal transduction components with other proteins or intracellular structures, or in the biochemical activity of components such as kinases, or assays designed to measure expression of reporter genes under control of receptor-sensitive promoters and enhancers, or indirectly by a downstream effect mediated by the receptor (e.g. activation of specific cytolytic machinery in NK or T cells). Reporter genes can be naturally occurring genes (e.g. monitoring cytokine production) or they can be genes artificially introduced into a cell. Other genes can be placed under the control of such regulatory elements and thus serve to report the level of receptor signaling.

### Producing polypeptides

The antigen binding domains (ABDs) described herein can be readily derived from any of a variety of immunoglobulin or non-immunoglobulin scaffolds, for example affibodies based on the Z-domain of staphylococcal protein A, engineered Kunitz domains, monobodies or adnectins based on the 10th extracellular domain of human fibronectin III, anticalins derived from lipocalins, DARPins (designed ankyrin repeat domains, multimerized LDLR-A module, avimers or cysteine-rich knottin peptides. See, e.g., Gebauer and Skerra (2009) Current Opinion in Chemical Biology 13:245-255.

Immunoglobulin ABDs can be obtained from variable domains derived from antibodies (from immunoglobulin chains), for example in the form of associated V_{L} and V_{H} domains found on two polypeptide chains, or a single chain antigen binding domain such as a scFv, a V_{H} domain, a V_{L} domain, a dAb, a V-NAR domain or a V_{H}H domain. In certain advantageous proteins formats disclosed herein that directly enable the use of a wide range of variable regions from Fab or scFv without substantial further requirements for pairing and/or folding, the an antigen binding domain (e.g,. ABD, and ABD₂) can also be readily derived from antibodies as a Fab or scFv.

Typically, antibodies are initially obtained by immunization of a non-human animal, e.g., a mouse, rat, guinea pig or rabbit, with an immunogen comprising a polypeptide, or a fragment or derivative thereof, typically an immunogenic fragment, for which it is desired to obtain antibodies (e.g. a human polypeptide). The step of immunizing a non-human mammal with an antigen may be carried out in any manner well known in the art for stimulating the production of antibodies in a mouse (see, for example, E. Harlow and D. Lane, Antibodies: A Laboratory Manual., Cold Spring Harbor Laboratory Press, Cold Spring Harbor, NY (1988). Human antibodies may also be produced by using, for immunization, transgenic animals that have been engineered to express a human antibody repertoire (Jakobovitz et Nature 362 (1993) 255), or by selection of antibody repertoires using phage display methods. For example, a XenoMouse (Abgenix, Fremont, CA) can be used for immunization. A XenoMouse is a murine host that has had its immunoglobulin genes replaced by functional human immunoglobulin genes. Thus, antibodies produced by this mouse or in hybridomas made from the B cells of this mouse, are already humanized. The XenoMouse is described in United States Patent No. 6,162,963. Antibodies may also be produced by selection of combinatorial libraries of immunoglobulins, as disclosed for instance in (Ward et al. Nature, 341 (1989) p. 544). Phage display technology (McCafferty et al (1990) Nature 348:552-553) can be used to produce antibodies from immunoglobulin variable (V) domain gene repertoires from unimmunized donors. See, e.g., Griffith et al (1993) EMBO J. 12:725- 734; US 5,565,332; US 5,573,905; US 5,567,610; and US 5,229,275). When combinatorial libraries comprise variable (V) domain gene repertoires of human origin, selection from combinatorial libraries will yield human antibodies.

Additionally, a wide range of antibodies are available in the scientific and patent literature, including DNA and/or amino acid sequences, or from commercial suppliers. Antibodies will typically be directed to a pre-determined antigen. Examples of antibodies include antibodies that recognize an antigen expressed by a target cell that is to be eliminated, for example a proliferating cell or a cell contributing to a disease pathology. Examples include antibodies that recognize tumor antigens, microbial (e.g. bacterial or parasite) antigens or viral antigens.

Variable domains and/or antigen binding domains can be selected based on the desired cellular target, and may include for example cancer antigens, bacterial or viral antigens, etc. As used herein, the term "bacterial antigen" includes, but is not limited to, intact, attenuated or killed bacteria, any structural or functional bacterial protein or carbohydrate, or any peptide portion of a bacterial protein of sufficient length (typically about 8 amino acids or longer) to be antigenic. Examples include gram-positive bacterial antigens and gram-negative bacterial antigens. In some aspects, the bacterial antigen is derived from a bacterium selected from the group consisting of Helicobacter species, in particular Helicobacter pyloris; Borrelia species, in particular Borrelia burgdorferi; Legionella species, in particular Legionella pneumophilia; Mycobacteria s species, in particular M. tuberculosis, M. avium, M. intracellulare, M. kansasii, M. gordonae; Staphylococcus species, in particular Staphylococcus aureus; Neisseria species, in particular N. gonorrhoeae, N. meningitidis; Listeria species, in particular Listeria monocytogenes; Streptococcus species, in particular S. pyogenes, S. agalactiae; S. faecalis; S. bovis, S. pneumonae; anaerobic Streptococcus species; pathogenic Campylobacter species; Enterococcus species; Haemophilus species, in particular Haemophilus influenzae; Bacillus species, in particular Bacillus anthracis; Corynebacterium species, in particular Corynebacterium diphtheriae; Erysipelothrix species, in particular Erysipelothrix rhusiopathiae; Clostridium species, in particular C. perfringens, C. tetani; Enterobacter species, in particular Enterobacter aerogenes, Klebsiella species, in particular Klebsiella 1S pneumoniae, Pasteurella species, in particular Pasteurella multocida, Bacteroides species; Fusobacterium species, in particular Fusobacterium nucleatum; Streptobacillus species, in particular Streptobacillus moniliformis; Treponema species, in particular Treponema pertenue; Leptospira; pathogenic Escherichia species; and Actinomyces species, in particular Actinomyces israeli.

As used herein, the term "viral antigen" includes, but is not limited to, intact, attenuated or killed whole virus, any structural or functional viral protein, or any peptide portion of a viral protein of sufficient length (typically about 8 amino acids or longer) to be antigenic. Sources of a viral antigen include, but are not limited to viruses from the families: Retroviridae (e.g., human immunodeficiency viruses, such as HIV-1 (also referred to as HTLV-III, LAV or HTLV-III/LAV, or HIV-III; and other isolates, such as HIV-LP; Picornaviridae (e.g., polio viruses, hepatitis A virus; enteroviruses, human Coxsackie viruses, rhinoviruses, echoviruses); Calciviridae (e.g., strains that cause gastroenteritis); Togaviridae (e.g., equine encephalitis viruses, rubella viruses); Flaviviridae (e.g., dengue viruses, encephalitis viruses, yellow fever viruses); Coronaviridae (e.g., coronaviruses); Rhabdoviridae (e.g., vesicular stomatitis viruses, rabies viruses); Filoviridae (e.g., Ebola viruses); Paramyxoviridae (e.g., parainfluenza viruses, mumps virus, measles virus, respiratory syncytial virus); Orthomyxoviridae (e.g., influenza viruses); Bunyaviridae (e.g., Hantaan viruses, bunya viruses, phleboviruses and Nairo viruses); Arenaviridae (hemorrhagic fever viruses); Reoviridae (e.g., reoviruses, orbiviruses and rotaviruses); Bornaviridae; Hepadnaviridae (Hepatitis B virus); Parvoviridae (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus; Poxviridae (variola viruses, vaccinia viruses, pox viruses); and Iridoviridae (e.g., African swine fever virus); and unclassified viruses (e.g., the agent of delta hepatitis (thought to be a defective satellite of hepatitis B virus), Hepatitis C; Norwalk and related viruses, and astroviruses). Alternatively, a viral antigen may be produced recombinantly.

As used herein, the terms "cancer antigen" and "tumor antigen" are used interchangeably and refer to antigens that are differentially expressed by cancer cells or are expressed by non-tumoral cells (e.g. immune cells) having a pro-tumoral effect (e.g. an immunosuppressive effect), and can thereby be exploited in order to target cancer cells. Cancer antigens are antigens which can potentially stimulate apparently tumor-specific immune responses. Some of these antigens are encoded, although not necessarily expressed, or expressed at lower levels or less frequently, by normal cells. These antigens can be characterized as those which are normally silent (i.e., not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), fusion proteins resulting from internal deletions or chromosomal translocations. Still other cancer antigens can be encoded by viral genes such as those carried on RNA and DNA tumor viruses. Still other cancer antigens can be expressed on immune cells capable of contributing to or mediating a pro-tumoral effect, e.g. cell that contributes to immune evasion, a monocyte or a macrophage, optionally a suppressor T cell, regulatory T cell, or myeloid-derived suppressor cell.

The cancer antigens are usually normal cell surface antigens which are either over-expressed or expressed at abnormal times, or are expressed by a targeted population of cells. Ideally the target antigen is expressed only on proliferative cells (e.g., tumor cells) or pro-tumoral cells (e.g. immune cells having an immunosuppressive effect), however this is rarely observed in practice. As a result, target antigens are in many cases selected on the basis of differential expression between proliferative/disease tissue and healthy tissue. Example of cancer antigens include: Receptor Tyrosine Kinase-like Orphan Receptor 1 (ROR1), Crypto, CD4, CD20, CD30, CD19, CD38, CD47, Glycoprotein NMB, CanAg, Her2 (ErbB2/Neu), a Siglec family member, for example CD22 (Siglec2) or CD33 (Siglec3), CD79, CD138, CD171, PSCA, L1-CAM, PSMA (prostate specific membrane antigen), BCMA, CD52, CD56, CD80, CD70, E-selectin, EphB2, Melanotransferrin, Mud 6 and TMEFF2. Examples of cancer antigens also include Immunoglobulin superfamily (IgSF) such as cytokine receptors, Killer-Ig Like Receptor, CD28 family proteins, for example, Killer-Ig Like Receptor 3DL2 (KIR3DL2), B7-H3, B7-H4, B7-H6, PD-L1, IL-6 receptor. Examples also include MAGE, MART-1/Melan-A, gp100, major histocompatibility complex class I-related chain A and B polypeptides (MICA and MICB), adenosine deaminase-binding protein (ADAbp), cyclophilin b, colorectal associated antigen (CRC)-C017-1A/GA733, protein tyrosine kinase 7(PTK7), receptor protein tyrosine kinase 3 (TYRO-3), nectins (e.g. nectin-4), major histocompatibility complex class I-related chain A and B polypeptides (MICA and MICB), proteins of the UL16-binding protein (ULBP) family, proteins of the retinoic acid early transcript-1 (RAET1) family, carcinoembryonic antigen (CEA) and its immunogenic epitopes CAP-1 and CAP-2, etv6, aml1, prostate specific antigen (PSA), T-cell receptor/CD3-zeta chain, MAGE-family of tumor antigens, GAGE-family of tumor antigens, anti-Müllerian hormone Type II receptor, delta-like ligand 4 (DLL4), DR5, ROR1 (also known as Receptor Tyrosine Kinase-Like Orphan Receptor 1 or NTRKR1 (EC 2.7.10.1), BAGE, RAGE, LAGE-1, NAG, GnT-V, MUM-1, CDK4, MUC family, VEGF, VEGF receptors, Angiopoietin-2, PDGF, TGF-alpha, EGF, EGF receptor, members of the human EGF-like receptor family, e.g., HER-2/neu, HER-3, HER-4 or a heterodimeric receptor comprised of at least one HER subunit, gastrin releasing peptide receptor antigen, Muc-1, CA125, integrin receptors, αvß3 integrins, α5ß1 integrins, αIIbß3-integrins, PDGF beta receptor, SVE-cadherin, IL-8 receptor, hCG, IL-6 receptor, CSF1R (tumor-associated monocytes and macrophages), α-fetoprotein, E-cadherin, α-catenin, ß-catenin and γ-catenin, p120ctn, PRAME, NY-ESO-1, cdc27, adenomatous polyposis coli protein (APC), fodrin, Connexin 37, Ig-idiotype, p15, gp75, GM2 and GD2 gangliosides, viral products such as human papillomavirus proteins, imp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2, although this is not intended to be exhaustive. In one aspect, the antigen of interest is an antigen (e.g. any one of the antigens listed above) capable of undergoing intracellular internalization, for example when bound by a conventional human IgG1 antibody, either in the presence of absence of Fcy receptor cells. In one aspect, the antigen of interest is a CD19 or CD20 polypeptide; in one aspect, the multispecific protein comprises a VH and/or VL, or a scFv, or another ABD, that binds CD19 or CD20 comprising an amino acid sequence which is at least 60%, 70%, 80%, 85%, 90% or 95% identical to the sequence of the anti-CD19 or anti- CD20 respective VH, VL or scFv described in the Examples herein, or comprises the heavy and light chain CDR1, -2 and -3 of the anti-CD19 or anti-CD20 heavy and light chain variable regions disclosed herein. In one aspect, the multispecific protein competes for binding to a human CD19 or CD20 polypeptide with an antibody, or a F5 or T6 protein, comprising the respective anti-CD19 or anti-CD20 VH, VL or scFv disclosed in the Examples herein.

In one aspect, the ABD that binds an antigen of interest is derived from (e.g. comprises the hypervariable region of, or comprises one, two, three, four, five or six of the CDRs of) a parental antibody that binds an antigen of interest (e.g. a murine antibody, a human antibody) which, when bound to its antigenic target (the antigen of interest on cells), increases or induces down-modulation or intracellular internalization of the antigen of interest. In one aspect, the antigen of interest is a cancer antigen, e.g. one of the cancer antigens listed above known to internalize (e.g. Immunoglobulin superfamily (IgSF) members, for example cytokine receptor α or β chains, Killer-Ig Like Receptors, CD28 family proteins, B7-H3, B7-H4, B7-H6, KIR3DL2, PTK7, ROR1, L1-CAM, Siglec family members, EGF receptor and EGF-like receptor family members, EGFR, HER-2, integrins, anti-Müllerian hormone Type II receptor, CSF-1R, and others) In one aspect, the antigen target is a polypeptide present on an immune cell capable of mediating a pro-tumoral effect, e.g. a monocyte or a macrophage, optionally a suppressor T cell, regulatory T cell, or myeloid-derived suppressor cell.

In exemplary aspects, an ABD, variable domain or pair of complementary variable domains will bind an antigen expressed by a target cell that is to be eliminated (e.g., a tumor antigen, microbial (e.g. bacterial or parasitic) antigen, viral antigen, or antigen expressed on an immune cell that is contributing to inflammatory or autoimmune disease, and another ABD, variable domain or pair of complementary variable domains will bind to an antigen expressed on an immune cell, for example an immune effector cell, e.g. a cell surface receptor of an effector cells such as a T or NK cell. Examples of antigens expressed on immune cells, optionally immune effector cells, include antigens expressed on a member of the human lymphoid cell lineage, e.g. a human T cell, a human B cell or a human natural killer (NK) cell, a human monocyte, a human neutrophilic granulocyte or a human dendritic cell. Advantageously, such cells will have either a cytotoxic or an apoptotic effect on a target cell that is to be eliminated (e.g., that expresses a tumor antigen, microbial antigen, viral antigen, or antigen expressed on an immune cell that is contributing to inflammatory or autoimmune disease). Especially advantageously, the human lymphoid cell is a cytotoxic T cell or NK cell which, when activated, exerts a cytotoxic effect on the target cell. According to this aspect, then, the cytotoxic activity of the human effector cells is recruited. According to another aspect, the human effector cell is a member of the human myeloid lineage.

Antigens expressed on an immune cell to which antibodies of fragments that make up multispecific protein can bind also include NK and/or T cell receptors, e.g. any molecule on the surface of NK cells or T cells, respectively, that can serve to direct the NK or T cell to the intended target cell to be eliminated, and preferably to permit the NK and/or T cell to mediate the elimination or lysis of the target cell. Examples include, e.g., members of the immunoglobulin superfamily, members of the killer-cell immunoglobulin-like receptor (KIR) family, the leukocyte immunoglobulin-like receptors (LILR) family, or the lectin family or the NK cell lectin-like receptor family. Activity can be measured for example by bringing target cells and effector cells into contact in presence of the multispecific polypeptide. Optionally the immune cell receptor is an immune effector cell activating receptor, e.g. an activating NK cell or T cell receptor. As used herein, the terms "activating NK cell receptor" and "activating T cell receptor" refers to any molecule on the surface of NK cells or T cells, respectively, that, when stimulated, causes a measurable increase in any property or activity known in the art as associated with NK cell or T cell activity, respectively, such as cytokine (for example IFN-γ or TNF-α) production, increases in intracellular free calcium levels, the ability to lyse target cells in a redirected killing assay as described, e.g. elsewhere in the present specification, or the ability to stimulate NK cell or T cell proliferation, respectively. The term "activating NK receptor" includes but is not limited to DNAX accessory molecule-1 (DNAM-1), 2B4, activating forms of KIR proteins (for example KIR2DS receptors, KIR2DS2, KIR2DS4), NKG2D, NKp30, CD137, CD69, NKp80, NKp44, NKp46, IL-2R, IL-12R, IL-15R, IL-18R and IL-21R. In one aspect, the activating NK cell receptor is a receptor other than an Fcy receptor. In one aspect, the activating NK cell receptor is a receptor other than NKp46.

Activation of cytotoxic T cells may occur via binding of the CD3 antigen as effector antigen on the surface of the cytotoxic T cell by a multispecific (e.g. bispecific) polypeptide of this aspect. The human CD3 antigen is present on both helper T cells and cytotoxic T cells. Human CD3 denotes an antigen which is expressed on T cells as part of the multimolecular T cell complex and which comprises three different chains: CD3-epsilon, CD3-delta and CD3-gamma. Other effector cell antigens that can be bound by an ABD are the human CD8 antigen, the human CD2 antigen, the human CD28 antigen or the human CD25 antigen.

In one aspect, the multispecific protein comprises one ABD that binds specifically to CD8, and one ABD that bind to CD3. In one aspect, the multispecific protein comprises one ABD that binds specifically to an activating receptor present on effector NK cells, and one ABD that bind to an activating receptor present on effector T cells. In one aspect, the multispecific comprises one ABD that binds to a cancer antigen, a viral antigen or a bacterial antigen.

The ABDs or variable domains which are incorporated into the polypeptides can be tested for any desired activity prior to inclusion in a polypeptide. Once appropriate antigen binding domains having desired specificity and/or activity are identified, DNA encoding each variable domain can be placed, in suitable arrangements, in an appropriate expression vector(s), together with DNA encoding any elements such as an enzymatic recognition tag, or CH2 and CH3 domains and any other optional elements (e.g. DNA encoding a linker or hinge region) for transfection into an appropriate host(s). The host is then used for the recombinant production of the polypeptide chains that make up the multispecific protein.

An ABD or variable region derived from an antibody will generally comprise at minimum a hypervariable region sufficient to confer binding activity when present in the multimeric polypeptide. It will be appreciated that an ABD or variable region may comprise linker elements (e.g. linker peptides, constant domain derived sequences, hinges, or fragments thereof, each of which can be placed between a variable domain and a CH1, Cκ, CH2 or CH3 domain, or between other domains as needed).

In any aspect, ABDs or variable regions can be obtained from a humanized antibody in which residues from a complementary-determining region (CDR) of a human antibody are replaced by residues from a CDR of the original antibody (the parent or donor antibody, e.g. a murine or rat antibody) while maintaining the desired specificity, affinity, and capacity of the original antibody. The CDRs of the parent antibody, some or all of which are encoded by nucleic acids originating in a non-human organism, are grafted in whole or in part into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536. An antigen binding domain can thus have non-human hypervariable regions or CDRs and human frameworks region sequences (optionally with back mutations).

Polypeptide chains will be arranged in one or more expression vectors so as to produce the polypeptides having the desired domains operably linked to one another. The host cell may be of mammalian origin or may be selected from COS-1, COS-7, HEK293, BHK21, CHO, BSC-1, Hep G2, 653, SP2/0, 293, HeLa, myeloma, lymphoma, yeast, insect or plant cells, or any derivative, immortalized or transformed cell thereof.

The polypeptide can then be produced in an appropriate host cell or by any suitable synthetic process and brought into contact under appropriate conditions for the multimeric (e.g. dimer or trimer) polypeptide to form.

### Polypeptide configurations

An isolated hetero-multimeric protein that binds a first and second antigen of interest can be prepared according to different configurations, in each case involving at least a central (first) polypeptide chain and a second polypeptide chain, and a third polypeptide chain.

The first (central) polypeptide chain will provide one variable domain that will, together with a complementary variable domain on a second polypeptide chain, form an antigen binding domain specific for one (e.g. a first) antigen of interest. The first (central) polypeptide chain will also provide a second variable domain that will be paired with a complementary variable domain to form an antigen binding domain specific for another (e.g. a second) antigen of interest; the variable domain that is complementary to the second variable domain is placed on a third polypeptide chain. The second and third polypeptide chains will associate with the central polypeptide chain by CH1-Cκ heterodimerization, forming non-covalent interactions and optionally further interchain disulfide bonds between respective hinge domains and between complementary CH1 and CK domains, with a single multimeric polypeptide being formed so long as CH/Cκ and VH/VK domains are chosen to give rise to a sole dimerization configuration. In a trimer, or when polypeptides are constructed for preparation of a trimer, there will generally be one polypeptide chain that comprises a non-naturally occurring VH-Cκ or VL-CH1 domain arrangement.

The first (central) polypeptide chain comprises a first variable domain (V) fused to a CH1 of CL constant region (e.g. the V domain is fused at its C-terminus to the N-terminus of a CH1 or CK constant region), a second variable domain, and an Fc domain interposed between the first and second variable domains may have the Examples of domain arrangement for the first polypeptide include:

| |
|---|
| (VH or VK) - CH1 - Fc domain - VH - CH1 |
| (VH or VK) - CK - Fc domain - VK - CK |
| (VH or VK) - CK - Fc domain - VK - CH1 |
| (VH or VK) - CH1 - Fc domain - VH - CK |
| (VH or VK) - CH1 - Fc domain - VK - CH1 |
| (VH or VK) - CK - Fc domain - VH - CK |
| (VH or VK) - CK - Fc domain - VH - CH1 |
| (VH or VK) - CH1 - Fc domain - VK - CK |

A second polypeptide chain comprises a first variable domain (V) fused (e.g. at its C-terminus) to a CH1 or CL (e.g. CK) constant region selected to be complementary to the CH1 or CL constant region of the first polypeptide chain such that the first and second polypeptides form a CH1-CL (e.g., CH1-CK) heterodimer. The second polypeptide chain further comprises an Fc domain fused to the C- terminus of the CH1 or CL domain. Examples of domain arrangement for the second polypeptide include

| |
|---|
| (VH or VK) - (CH1) - Fc domain |
| (VH or VK) - (CK) - Fc domain |

A third polypeptide chain has the domain arrangement:

| |
|---|
| (VH or VK) - (CH1 or (CK) |
| (VH or VK) - (CH1 or (CK) - scFV |

### Heterotrimers with two ABDs and a dimeric Fc

Heterotrimeric proteins are formed by using a central (first) polypeptide chain comprising a first variable domain (V) fused to a first CH1 or CK constant region, a second variable domain (V) fused to a second CH1 or CK constant region, and an Fc domain interposed between the first and second variable domains (i.e. the Fc domain is interposed between the first and second (V-(CH1/CK)) units. For example, a central polypeptide chain for use in a heterotrimeric protein has the domain arrangements (N- to C- terminal) as follows:

Vₐ₁ - (CH1 or CK)ₐ - Fc domain - Vₐ₂ - (CH1 or CK)_{b}.

A second polypeptide chain comprises a domain arrangement (N- to C- terminal):

V_{b1} - (CH1 or CK)_{c} - Fc domain

such that the (CH1 or CK)_{c} dimerizes with the (CH1 or CK)ₐ on the central chain, and the Vₐ₁ and V_{b1} form an antigen binding domain.

A third polypeptide chain comprises a domain arrangement (N- to C- terminal):

V_{b2} - (CH1 or CK)_{d},

such that the (CH1 or CK)_{d} dimerizes with the (CH1 or CK)_{b} unit on the central chain, and the Vₐ₂ and V_{b2} form an antigen binding domain.

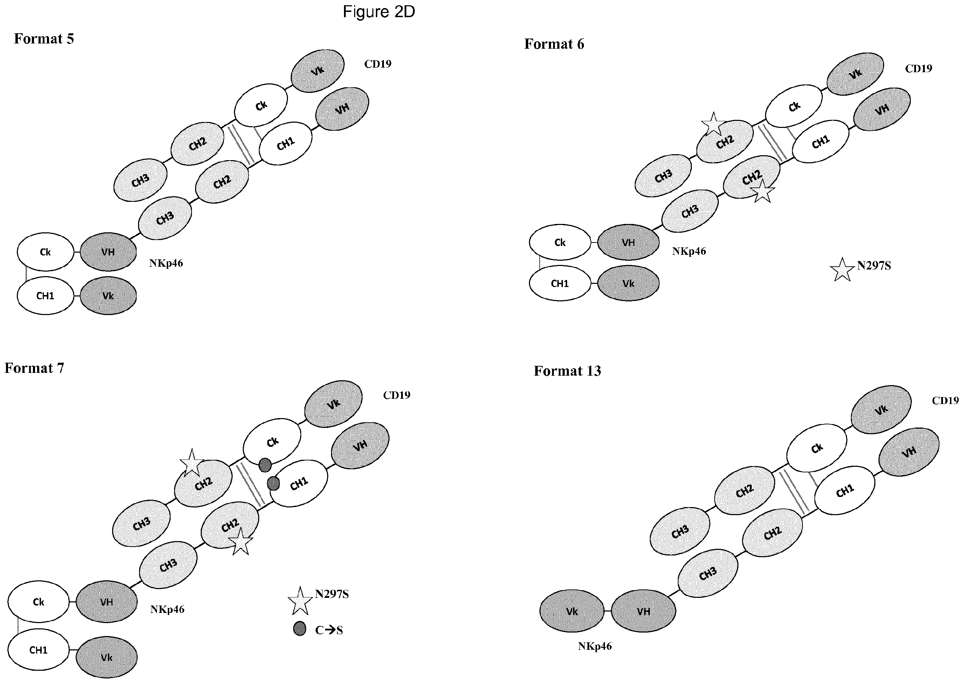

An example of a configuration of a resulting heterotrimer with a dimeric Fc domain (also shown as format 5 in Figure 2D) has a domain arrangement:

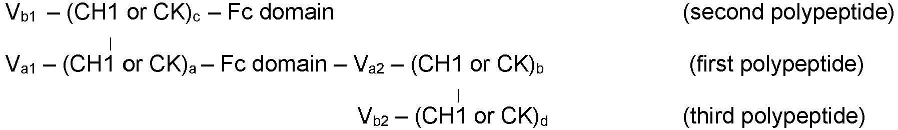

Thus, in a configuration of a trimer polypeptide, the first polypeptide can have two variable domains that each form an antigen binding domain with a variable domain on a separate polypeptide chain (i.e. the variable domain of the second and third chains), the second polypeptide chain has one variable domain, and the third polypeptide has one variable domain, and wherein the trimer has a dimeric Fc domain that binds CD16.

Examples of domain arrangement for the trimeric bispecific polypeptide formed from include:

| | | |
|---|---|---|
| 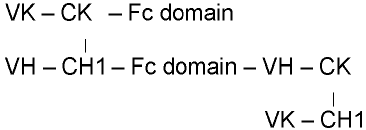 | (second polypeptide) | |
| | (first polypeptide) | |
| | (third polypeptide) | |
| 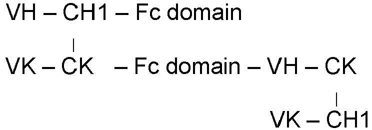 | | (second polypeptide) |
| | | (first polypeptide) |
| | | (third polypeptide) |
| 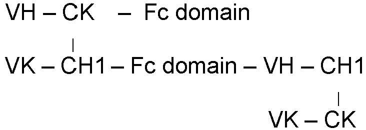 | | (second polypeptide) |
| | | (first polypeptide) |
| | | (third polypeptide) |

### Heterotrimers with three ABDs and a dimeric Fc)

Heterotrimeric proteins can for example be formed by using a central (first) polypeptide chain comprising a first variable domain (V) fused to a first CH1 or CK constant region, a second variable domain (V) fused to a second CH1 or CK constant region, and an Fc domain interposed between the first and second variable domains (i.e. the Fc domain is interposed between the first and second (V-(CH1/CK)) units. For example, a central polypeptide chain for use in a heterotrimeric protein has the domain arrangements (N- to C- terminal) as follows:

V₁ - (CH1 or CK)ₐ - Fc domain - V₂ - (CH1 or CK)_{b}.

A second polypeptide chain comprises a domain arrangement (N- to C- terminal):

V₁ - (CH1 or CK)_{c}- Fc domain

such that the (CH1 or CK)_{c} dimerizes with the (CH1 or CK)ₐ on the central chain, and the Vₐ₁ and V_{b1} form an antigen binding domain.

A third polypeptide chain can then comprise a domain arrangement (N- to C- terminal):

V₂ - (CH1 or CK)_{d} - scFv,

such that the (CH1 or CK)_{d} dimerizes with the (CH1 or CK)_{b} unit on the central chain, and the Vₐ₂ and V_{b2} form an antigen binding domain.

An example of a configuration of a resulting heterotrimer with a dimeric Fc domain (also shown as format T5 in Figure 2F) has a domain arrangement:

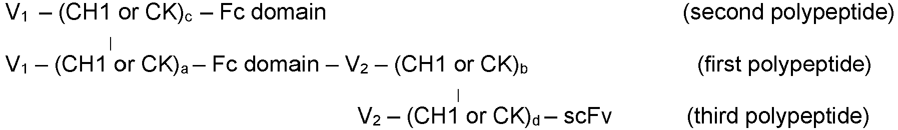

In any of the polypeptide chains herein, a hinge region will typically be present on a polypeptide chain between a CH1 domain and a CH2 domain of an Fc domain, and/or can be present between a CK domain and a CH2 domain. A hinge region can optionally be replaced for example by a suitable linker peptide.

The proteins domains described in the present disclosure can optionally be specified as being from N- to C- terminal. Protein arrangements of the disclosure for purposes of illustration are shown from N-terminus (on the left) to C-terminus. Domains can be referred to as fused to one another (e.g. a domain can be said to be fused to the C-terminus of the domain on its left, and/or a domain can be said to be fused to the N-terminus of the domain on its right).

The proteins domains described in the present disclosure can be fused to one another directly or via intervening amino acid sequences. For example, a CH1 or CK domain will be fused to an Fc domain via a linker peptide, optionally a hinge region or a fragment thereof. In another example, a VH or VK domain will be fused to a CH3 domain via a linker peptide. VH and VL domains linked to another in tandem will be fused via a linker peptide (e.g. as an scFv). VH and VL domains linked to an Fc domain will be fused via a linker peptide. Two polypeptide chains will be bound to one another (indicated by "_{|}") by non-covalent bonds and optionally further by interchain disulfide bonds formed between cysteine residues within complementary CH1 and CK domains.

It will be appreciated that in any aspect herein, a VK domain can be replaced by a Vλ variable domain.

In any of the domain arrangements, the Fc domain comprises a CH2-CH3 unit (a full length CH2 and CH3 domain). In heterotrimers comprising two chains with Fc domains (a dimeric Fc domain), the CH3 domain will be capable of CH3-CH3 dimerization (e.g. a wild-type CH3 domain). Optionally the multimeric polypeptide is capable of binding to human FcRn with intermediate affinity, e.g. retains binding to FcRn but has decreased binding to a human FcRn receptor compared to a full-length wild type human IgG1 antibody. The Fc moiety may further comprise one or more amino acid modifications, e.g. in the CH2 domain, that decreases further (e.g. abolishes) binding to one or more Fcy receptors.

In one aspect of any of the polypeptides or methods herein, the CH3 domain comprises an amino acid substitution at 1, 2, 3, 4, 5, 6 or 7 of the positions L351, T366, L368, P395, F405, T407 (or Y407) and/or K409 (EU numbering as in Kabat).

When two variable regions that form an antigen binding domain are placed on the same polypeptide chain they are typically linked together by a linker of sufficient length to enable the ABD to fold in such a way as to permit binding to the antigen for which the ABD is intended to bind, e.g., they can form a scFv. Examples of linkers include, for example, linkers comprising glycine and serine residues, e.g., the amino acid sequence GEGTSTGS(G₂S)₂GGAD. In another specific aspect, the VH domain and VL domains of an svFv are linked together by the amino acid sequence (G₄S)₃.

An ABD can be linked to a constant domain or Fc domain via a linker (e.g. a flexible polypeptide linker) that permits the ABD to be positioned such that it binds to its target antigen and exhibits the desired functionality, e.g. it possesses a sufficient range of motion relative to the rest of the multispecific protein (the Fc domain and/or other ABD) and thereby mediates signaling at a cell surface activating receptor. Examples of linkers include, for example, linkers derived from antibody hinge regions, an amino sequence RTVA, or linkers comprising glycine and serine residues, e.g., the amino acid sequence GEGTSTGS(G₂S)₂GGAD. In another specific aspect, the V_{H} domain and V_{L} domains of a scFv are linked together by the amino acid sequence (GaS)s. Such linkers can be used particularly advantageously to link and ABD to a constant region or Fc domain when the ABD comprises two variable regions that are placed on the same polypeptide chain (e.g., an scFv)

Any of the peptide linkers contained in the subject multispecific proteins may comprise a length of at least 4 residues, at least 5 residues, at least 10 residues, at least 15 residues, at least 20 residues, at least 25 residues, at least 30 residues or more. In other aspects, the linkers comprise a length of between 2-4 residues, between 2-4 residues, between 2-6 residues, between 2-8 residues, between 2-10 residues, between 2-12 residues, between 2-14 residues, between 2-16 residues, between 2-18 residues, between 2- 20 residues, between 2-22 residues, between 2-24 residues, between 2-26 residues, between 2-28 residues, between 2-30 residues, between 2 and 50 residues, or between 10 and 50 residues.

An ABD (e.g. an immunoglobulin variable region) can optionally be linked to a constant domain or Fc domain via a flexible linker (e.g. polypeptide linker) that leads to less structural rigidity or stiffness (e.g. between or amongst the ABD and Fc domain) compared to a conventional (e.g. wild-type full length human IgG) antibody. For example, the multispecific protein may have a structure or a flexible linker between the ABD and constant domain or Fc domain that permits an increased range of domain motion a compared to ABD in a conventional (e.g. wild-type full length human IgG) antibody. In particular, the structure or a flexible linker can be configured to confer on the antigen binding sites greater intrachain domain movement compared to antigen binding sites in a conventional human IgG1 antibody. Rigidity or domain motion/interchain domain movement can be determined, e.g., by computer modeling, electron microscopy, spectroscopy such as Nuclear Magnetic Resonance (NMR), X-ray crystallography (B-factors), or Sedimentation Velocity Analytical ultracentrifugation (AUC) to measure or compare the radius of gyration of proteins comprising the linker or hinge. A test protein or linker may have lower rigidity relative to a comparator protein if the test protein has a value obtained from one of the tests described in the previous sentence differs from the value of the comparator, e.g., an IgG1 antibody or a hinge, by at least 5%, 10%, 25%, 50%, 75%, or 100%. A person of skill in the art would be able to determine from the tests whether a test protein has at lower rigidity to that of another protein, respectively, by interpreting the results of these tests.

In one aspect, the multispecific protein may have a structure or a flexible linker between the ABD and constant domain or Fc domain that permits two ABDs to have a spacing between said two ABDs comprising less than about 80 angstroms, less than about 60 angstroms or ranges from about 40-60 angstroms.

In one aspect, a CH1 or CK domain is linked or fused to an Fc domain via a linker that comprises a fragment of a CH1 domain and/or hinge region. For example, a N-terminal amino acid sequence of CH1 can be fused to a variable domain in order to mimic as closely as possible the natural structure of a wild-type antibody. In one aspect, the linker comprises an amino acid sequence from a hinge domain or an N-terminal CH1 amino acid. The sequence can be, for example, between 2-4 residues, between 2-4 residues, between 2-6 residues, between 2-8 residues, between 2-10 residues, between 2-12 residues, between 2-14 residues, between 2-16 residues, between 2-18 residues, between 2- 20 residues, between 2-22 residues, between 2-24 residues, between 2-26 residues, between 2-28 residues, or between 2-30 residues. In one aspect, linker comprises or consists of the amino acid sequence RTVA.

In one aspect, a CH1 or CK domain is linked or fused to an Fc domain via a hinge region (or fragment thereof) derived form a hinge domain of a human IgG1 antibody. For example a hinge domain may comprise the amino acid sequence: T-H-T-C-S-S-C-P-A-P-E-L-L (one letter code), or an amino acid sequence at least 60%, 70%, 80% or 90% identical thereto, optionally wherein one or both cysteines are deleted or substituted by a different amino acid residue.

In one aspect, the hinge region (or fragment thereof) is derived from a Cµ2-C Cµ3 hinge domain of a human IgM antibody. For example a hinge domain may comprise the amino acid sequence: N-A-S-S-M-C-V-P-S-P-A-P-E-L-L (one letter code), or an amino acid sequence at least 60%, 70%, 80% or 90% identical thereto, optionally wherein one or both cysteines are deleted or substituted by a different amino acid residue.

Polypeptide chains that dimerize and associate with one another via non-covalent bonds may or may not additionally be bound by an interchain disulfide bond formed between respective CH1 and Cκ domains, and/or between respective hinge domains on the chains. CH1, Cκ and/or hinge domains (or other suitable linking amino acid sequences) can optionally be configured such that interchain disulfide bonds are formed between chains such that the desired pairing of chains is favored and undesired or incorrect disulfide bond formation is avoided. For example, when two polypeptide chains to be paired each possess a CH1 or Cκ adjacent to a hinge domain, the polypeptide chains can be configured such that the number of available cysteines for interchain disulfide bond formation between respective CH1/Cκ-hinge segments is reduced (or is entirely eliminated). For example, the amino acid sequences of respective CH1, Cκ and/or hinge domains can be modified to remove cysteine residues in both the CH1/Cκ and the hinge domain of a polypeptide; thereby the CH1 and Cκ domains of the two chains that dimerize will associate via non-covalent interaction(s).

In another example, the CH1 or Cκ domain adjacent (e.g., N-terminal to) a hinge domain comprises a cysteine capable of interchain disulfide bond formation, and the hinge domain which is placed at the C-terminus of the CH1 or Cκ comprises a deletion or substitution of one or both cysteines of the hinge (e.g. Cys 239 and Cys 242, as numbered for human IgG1 hinge according to Kabat). In one aspect, the hinge region (or fragment thereof) comprise the amino acid sequence: T-H-T-S-P-P-S-P-A-P-E-L-L (one letter code), or an amino acid sequence at least 60%, 70%, 80% or 90% identical thereto.

In another example, the CH1 or Cκ domain adjacent (e.g., N-terminal to) a hinge domain comprises a deletion or substitution at a cysteine residue capable of interchain disulfide bond formation, and the hinge domain placed at the C-terminus of the CH1 or Cκ comprises one or both cysteines of the hinge (e.g. Cys 239 and Cys 242, as numbered for human IgG1 hinge according to Kabat). In one aspect, the hinge region (or fragment thereof) comprises the amino acid sequence: T-H-T-C-S-S-C-P-A-P-E-L-L (one letter code), or an amino acid sequence at least 60%, 70%, 80% or 90% identical thereto.

In another example, a hinge region is derived from an IgM antibody. In such aspects, the CH1/CK pairing mimics the Cµ2 domain homodimerization in IgM antibodies. For example, the CH1 or Cκ domain adjacent (e.g., N-terminal to) a hinge domain comprises a deletion or substitution at a cysteine capable of interchain disulfide bond formation, and an IgM hinge domain which is placed at the C-terminus of the CH1 or Cκ comprises one or both cysteines of the hinge. In one aspect, the hinge region (or fragment thereof) comprises the amino acid sequence: T-H-T-C-S-S-C-P-A-P-E-L-L (one letter code), or an amino acid sequence at least 60%, 70%, 80% or 90% identical thereto.

Constant region domains can be derived from any suitable human antibody, including, the constant heavy (CH1) and light (Cκ) domains, hinge domains, CH2 and CH3 domains. "CH1" generally refers to positions 118-220 according to the EU index as in Kabat. "CH2" generally refers to positions 237-340 according to the EU index as in Kabat, and "CH3" generally refers to positions 341-447 according to the EU index as in Kabat.

A "hinge" or "hinge region" or "antibody hinge region" herein refers to the flexible polypeptide or linker between the first and second constant domains of an antibody. Structurally, the IgG CH1 domain ends at EU position 220, and the IgG CH2 domain begins at residue EU position 237. Thus for an IgG the hinge generally includes positions 221 (D221 in IgG1) to 236 (G236 in IgG1), wherein the numbering is according to the EU index as in Kabat. References to specific amino acid residues within constant region domains found within the polypeptides shall be, unless otherwise indicated or as otherwise dictated by context, be defined according to Kabat, in the context of an IgG antibody.

CH2 and CH3 domains which may be present in the subject antibodies or multispecific proteins can be derived from any suitable antibody. Such CH2 and CH3 domains can be used as wild-type domains or may serve as the basis for a modified CH2 or CH3 domain. Optionally the CH2 and/or CH3 domain is of human origin or may comprise that of another species (e.g., rodent, rabbit, non-human primate) or may comprise a modified or chimeric CH2 and/or CH3 domain, e.g., one comprising residues from different CH2 or CH3 domains, e.g., from different antibody isotypes or species antibodies .

In certain aspects herein where binding to CD16A is desired, a CH2 and/or CH3 domain (or Fc domain comprising same) may be wild-type domains or may comprise one or more amino acid modifications (e.g. amino acid substitutions) which increase binding to human CD16 and optionally another receptor such as FcRn. Optionally, the modifications will not substantially decrease or abolish the ability of the Fc-derived polypeptide to bind to neonatal Fc receptor (FcRn), e.g. human FcRn. Typical modifications include modified human IgG1-derived constant regions comprising at least one amino acid modification (e.g. substitution, deletions, insertions), and/or altered types of glycosylation, e.g., hypofucosylation. Such modifications can affect interaction with Fc receptors: FcγRI (CD64), FcγRII (CD32), and FcγRIII (CD16). FcγRI (CD64), FcγRIIA (CD32A) and FcγRIII (CD 16) are activating (i.e., immune system enhancing) receptors while FcγRIIB (CD32B) is an inhibiting (i.e., immune system dampening) receptor. A modification may, for example, increase binding of the Fc domain to FcγRIIIa on effector (e.g. NK) cells and/or decrease binding to FcγRIIB. Examples of modifications are provided in PCT publication no. WO2014/044686. Specific mutations in IgG1 which affect (enhance) FcγRIIIa or FcRn binding are also set forth below.

| Isotype | Species | Modification | Effector Function | Effect of Modification |
|---|---|---|---|---|
| IgG1 | Human | T250Q/M428L | Increased binding to FcRn | Increased half-life |
| IgG1 | Human | 1M252Y/S254T/T256E + H433K/N434F | Increased binding to FcRn | Increased half-life |
| IgG1 | Human | E333A | Increased binding to FcγRIIIa | Increased ADCC and CDC |
| IgG1 | Human | S239D/A330L/I332E | Increased binding to FcγRIIIa | Increased ADCC |
| IgG1 | Human | P257I/Q311 | Increased binding to FcRn | Unchanged half-life |
| IgG1 | Human | S239D/I332E/G236A | Increased FcγRIIa/FcγRIIb ratio | Increased macrophage phagocytosis |

In some aspects, the multispecific protein comprises a variant Fc region comprise at least one amino acid modification (for example, possessing 1, 2, 3, 4, 5, 6, 7, 8, 9, or more amino acid modifications) in the CH2 and/or CH3 domain of the Fc region, wherein the modification enhances binding to a human CD16 polypeptide. In other aspects, the multispecific protein comprises at least one amino acid modification (for example, 1, 2, 3, 4, 5, 6, 7, 8, 9, or more amino acid modifications) in the CH2 domain of the Fc region from amino acids 237-341, or within the lower hinge-CH2 region that comprises residues 231-341. In some aspects, the multispecific protein comprises at least two amino acid modifications (for example, 2, 3, 4, 5, 6, 7, 8, 9, or more amino acid modifications), wherein at least one of such modifications is within the CH3 region and at least one such modifications is within the CH2 region. Encompassed also are amino acid modifications in the hinge region. In one aspect, encompassed are amino acid modifications in the CH1 domain, optionally in the upper hinge region that comprises residues 216-230 (Kabat EU numbering). Any suitable functional combination of Fc modifications can be made, for example any combination of the different Fc modifications which are disclosed in any of United States Patents Nos. US, 7,632,497; 7,521,542; 7,425,619; 7,416,727; 7,371,826; 7,355,008; 7,335,742; 7,332,581; 7,183,387; 7,122,637; 6,821,505 and 6,737,056; and/or in PCT Publications Nos. WO2011/109400; WO 2008/105886; WO 2008/002933; WO 2007/021841; WO 2007/106707; WO 06/088494; WO 05/115452; WO 05/110474; WO 04/1032269; WO 00/42072; WO 06/088494; WO 07/024249; WO 05/047327; WO 04/099249 and WO 04/063351; and/or in Lazar et al. (2006) Proc. Nat. Acad. Sci. USA 103(11): 405-410; Presta, L.G. et al. (2002) Biochem. Soc. Trans. 30(4):487-490; Shields, R.L. et al. (2002) J. Biol. Chem. 26; 277(30):26733-26740 and Shields, R.L. et al. (2001) J. Biol. Chem. 276(9):6591-6604).

In some aspects, the multispecific protein comprises an Fc domain comprising at least one amino acid modification (for example, 1, 2, 3, 4, 5, 6, 7, 8, 9, or more amino acid modifications) relative to a wild-type Fc region, such that the molecule has an enhanced binding affinity for human CD16 relative to the same molecule comprising a wild-type Fc region, optionally wherein the variant Fc region comprises a substitution at any one or more of positions 221, 239, 243, 247, 255, 256, 258, 267, 268, 269, 270, 272, 276, 278, 280, 283, 285, 286, 289, 290, 292, 293, 294, 295, 296, 298, 300, 301, 303, 305, 307, 308, 309, 310, 311, 312, 316, 320, 322, 326, 329, 330, 332, 331, 332, 333, 334, 335, 337, 338, 339, 340, 359, 360, 370, 373, 376, 378, 392, 396, 399, 402, 404, 416, 419, 421, 430, 434, 435, 437, 438 and/or 439 (Kabat EU numbering).

In one aspect, the multispecific protein comprises an Fc domain comprising at least one amino acid modification (for example, 1, 2, 3, 4, 5, 6, 7, 8, 9, or more amino acid modifications) relative to a wild-type Fc region, such that the molecule has enhanced binding affinity for human CD16 relative to a molecule comprising a wild-type Fc region, optionally wherein the variant Fc region comprises a substitution at any one or more of positions 239, 298, 330, 332, 333 and/or 334 (e.g. S239D, S298A, A330L, I332E, E333A and/or K334A substitutions), optionally wherein the variant Fc region comprises a substitution at residues S239 and I332, e.g. a S239D and I332E substitution (Kabat EU numbering).

In some aspects, the multispecific protein comprises an Fc domain comprising altered glycosylation patterns that increase binding affinity for human CD16. Such carbohydrate modifications can be accomplished by, for example, by expressing a nucleic acid encoding the multispecific protein in a host cell with altered glycosylation machinery. Cells with altered glycosylation machinery are known in the art and can be used as host cells in which to express recombinant antibodies to thereby produce an antibody with altered glycosylation. See, for example, Shields, R.L. et al. (2002) J. Biol. Chem. 277:26733-26740; Umana et al. (1999) Nat. Biotech. 17:176-1, as well as, European Patent No: EP 1,176,195; PCT Publications WO 06/133148; WO 03/035835; WO 99/54342. In one aspect, the multispecific protein contains one or more hypofucosylated constant regions. Such multispecific protein may comprise an amino acid alteration or may not comprise an amino acid alteration and/or may be expressed or synthesized or treated under conditions that result in hypofucosylation. In one aspect, a multispecific protein composition comprises a multispecific protein described herein, wherein at least 20, 30, 40, 50, 60, 75, 85, 90, 95% or substantially all of the antibody species in the composition have a constant region comprising a core carbohydrate structure (e.g. complex, hybrid and high mannose structures) which lacks fucose. In one aspect, provided is a multispecific protein composition which is free of antibodies comprising a core carbohydrate structure having fucose. The core carbohydrate will preferably be a sugar chain at Asn297.

Optionally, a multispecific protein comprising a dimeric Fc domain can be characterized by having a binding affinity to a human CD16 polypeptide that is within 1-log of that of a conventional human IgG1 antibody, e.g., as assessed by surface plasmon resonance.

In one aspect, the multispecific protein comprising a dimeric Fc domain engineered to enhance Fc receptor binding can be characterized by having a binding affinity to a human CD16 polypeptide that is at least 1-log greater than that of a conventional or wild-type human IgG1 antibody, e.g., as assessed by surface plasmon resonance.

Optionally a multispecific protein comprising a dimeric Fc domain can be characterized by a Kd for binding (monovalent) to a human CD16 polypeptide of less than 10⁻⁵ M (10 µmolar), optionally less than 10⁻⁶ M (1 µmolar), as assessed by surface plasmon resonance (e.g. as in Example 13, SPR measurements performed on a Biacore T100 apparatus (Biacore GE Healthcare), with bispecific antibodies immobilized on a Sensor Chip CM5 and serial dilutions of soluble CD16 polypeptide injected over the immobilized bispecific antibodies.

In one aspect, the invention provides methods of making a heterotrimeric protein (e.g. any heterotrimeric protein described herein), comprising:
(a) providing a first nucleic acid encoding a first polypeptide chain described herein (e.g., a polypeptide chain comprising a first variable domain (V) fused to a first CH1 or CK constant region, a second variable domain fused to a second CH1 or CK constant region, and an Fc domain interposed between the first and second (V-CH1/CK) units);
(b) providing a second nucleic acid encoding a second polypeptide chain described herein (e.g., a polypeptide chain comprising a variable domain (V) fused at its C-terminus to a CH1 or CK constant region selected to be complementary to the first CH1 or CK constant region of the first polypeptide chain such that the first and second polypeptides form a CH1-CK heterodimer in which the first variable domain of the first polypeptide chain and the variable domain of the second polypeptide form an antigen binding domain, and an Fc domain;
(c) providing a third nucleic acid comprising a third polypeptide chain described herein (e.g., a polypeptide chain comprising a variable domain fused at its C-terminus to a CH1 or CK constant region, wherein the CH1 or CK constant region is selected to be complementary to the second variable domain and second CH1 or CK constant region of the first polypeptide chain such that the first and third polypeptides form a CH1-CK heterodimer in which the second variable domain of the first polypeptide and the variable domain of the third polypeptide form an antigen binding domain; and
(d) expressing said first, second and third nucleic acids in a host cell to produce a protein comprising said first, second and third polypeptide chains, respectively; and recovering a heterotrimeric protein comprising a dimeric Fc domain capable of binding human CD16. Optionally, the heterotrimeric protein produced represents at least 20%, 25% or 30% of the total proteins prior to purification. Optionally step (d) comprises loading the protein produced onto an affinity purification support, optionally an affinity exchange column, optionally a Protein-A support or column, and collecting the heterotrimeric protein; and/or loading the protein produced (e.g., the protein collected following loading onto an affinity exchange or Protein A column) onto an ion exchange column; and collecting the heterotrimeric fraction. In one aspect, one of the antigen binding domains binds NKp46 and the other binds an antigen of interest. In one aspect, the second or the third polypeptide further comprises and Fc domain or fragment thereof fused to the C-terminus of the CH1 or CK domain (e.g. via a hinge domain or linker).

In one aspect, the present disclosure provides a method for identifying or evaluating candidate variable regions for use in a heterotrimeric protein, comprising the steps of:
a) providing a plurality of nucleic acid pairs, wherein each pair includes one nucleic acid encoding a heavy chain candidate variable region and one nucleic acid encoding a light chain candidate variable region, for each of a plurality of heavy and light chain variable region pairs (e.g., obtained from different antibodies binding the same or different antigen(s) of interest);
b) for each of the plurality nucleic acid pairs, making a heterotrimeric protein by:
   (i) producing a first nucleic acid encoding a first polypeptide chain comprising one of the heavy or light chain candidate variable domains (V) fused to a first CH1 or CK constant region, a second variable domain fused to a second CH1 or CK constant region, and an Fc domain interposed between the first and second (V-CH1/CK) units);
   (ii) producing a second nucleic acid encoding a second polypeptide chain comprising the other of the heavy or light chain candidate variable domains (V) fused at its C-terminus to a CH1 or CK constant region selected to be complementary to the first CH1 or CK constant region of the first polypeptide chain such that the first and second polypeptides form a CH1-CK heterodimer in which the heavy and light chain candidate variable domains form an antigen binding domain, and an Fc domain;
   (iii) producing a third nucleic acid encoding a third polypeptide chain comprising a variable domain fused at its C-terminus to a CH1 or CK constant region, wherein the CH1 or CK constant region is selected to be complementary to the second variable domain and second CH1 or CK constant region of the first polypeptide chain such that the first and third polypeptides form a CH1-CK heterodimer in which the second variable domain of the first polypeptide and the variable domain of the third polypeptide form an antigen binding domain; and
   (iv) expressing said nucleic acids encoding the first and second polypeptide chains in a host cell to produce said first and second polypeptide chains, respectively; and recovering a heterodimeric protein comprising a dimeric Fc domain capable of binding human CD16; and
c) evaluating the plurality of heterodimeric proteins produced for a biological activity of interest, e.g., an activity disclosed herein. In one aspect, the second or the third polypeptide further comprises and Fc domain or fragment thereof fused to the C-terminus of the CH1 or CK domain (e.g. via a hinge domain or linker). Optionally, the heterotrimeric protein produced represents at least 20%, 25% or 30% of the total proteins prior to purification. Optionally the recovering step in (iii) loading the protein produced onto an affinity purification support, optionally an affinity exchange column, optionally a Protein-A support or column, and collecting the heterotrimeric protein; and/or loading the protein produced (e.g., the protein collected following loading onto an affinity exchange or Protein A column) onto an ion exchange column; and collecting the heterotrimeric fraction.

In the methods for identifying or evaluating candidate variable regions, it will be appreciated that the candidate variable regions can be for example from an anti-activating receptor antibody or from an antigen that binds an antigen of interest. It will also be appreciated that the position of the respective ABDs for the candidate variable region pair and the other variable region pair can be inverted. For example, in a trimeric protein the methods can be modified such that the heavy and light chain candidate variable domains are formed by the second V region of the first polypeptide and the V region of the second polypeptide, and the other variable region pair are formed by the first V region of the first polypeptide and the V region of the third polypeptide.

In one aspect, the second variable domain of the first polypeptide and the variable domain of the third polypeptide form an antigen binding domain that binds an activating receptor on an immune cell.

Furthermore, by providing a panel of different multispecific protein formats that all can be produced in standard cell lines and standardized methods with high yields, yet have different properties (e.g. conformational flexibility, spacing between two antigen binding domains, etc.) that can affect functional activity of the protein, the protein formats of the disclosure can be used in a panel to screen proteins configurations or formats to identify the most effective configurations or formats for a given antigen of interest, or combination of first and second antigen of interest. Different protein formats may access or engage their antigen targets differently.

In one aspect, the present disclosure provides a library of at least 5, 10, 20, 30, 50 hetero-multimeric proteins of the disclosure, wherein the proteins share domain arrangements but differ in the amino acid sequence of the variable domain of one or both of their antigen binding domains.

In one aspect, the present disclosure provides a library of at least 2, 3, 4, 5 or 10 hetero-multimeric proteins of the disclosure, wherein the proteins share the amino acid sequence of the variable domain of one or both of their antigen binding domains, but differ in domain arrangements.

In one aspect of the any of the aspects herein, recovering a heterotrimer protein can comprise introducing the protein to a solid phase so as to immobilize the protein. The immobilized protein can then subsequently be eluted. Generally, the solid support may be any suitable insoluble, functionalized material to which the proteins can be reversibly attached, either directly or indirectly, allowing them to be separated from unwanted materials, for example, excess reagents, contaminants, and solvents. Examples of solid supports include, for example, functionalized polymeric materials, e.g., agarose, or its bead form Sepharose^{®}, dextran, polystyrene and polypropylene, or mixtures thereof; compact discs comprising microfluidic channel structures; protein array chips; pipet tips; membranes, e.g., nitrocellulose or PVDF membranes; and microparticles, e.g., paramagnetic or non-paramagnetic beads. In some aspects, an affinity medium will be bound to the solid support and the protein will be indirectly attached to solid support via the affinity medium. In one aspect, the solid support comprises a protein A affinity medium or protein G affinity medium. A "protein A affinity medium" and a "protein G affinity medium" each refer to a solid phase onto which is bound a natural or synthetic protein comprising an Fc-binding domain of protein A or protein G, respectively, or a mutated variant or fragment of an Fc-binding domain of protein A or protein G, respectively, which variant or fragment retains the affinity for an Fc-portion of an antibody. Protein A and Protein G are bacterial cell wall proteins that have binding sites for the Fc portion of mammalian IgG. The capacity of these proteins for IgG varies with the species. In general, IgGs have a higher affinity for Protein G than for Protein A, and Protein G can bind IgG from a wider variety of species. The affinity of various IgG subclasses, especially from mouse and human, for Protein A varies more than for Protein G. Protein A can, therefore, be used to prepare isotypically pure IgG from some species. When covalently attached to a solid matrix, such as cross-linked agarose, these proteins can be used to capture and purify antigen-protein complexes from biochemical solutions. Commercially available products include, e.g., Protein G, A or L bonded to agarose or sepharose beads, for example EZview^{™} Red Protein G Affinity Gel is Protein G covalently bonded to 4% Agarose beads (Sigma Aldrich Co); or POROS^{®} A, G, and CaptureSelect^{®} HPLC columns (Invitrogen Inc.).Affinity capture reagents are also described, for example, in the Antibody Purification Handbook, Biosciences, publication No. 18-1037-46, Edition AC).

Once the multispecific protein is produced it can be assessed for biological activity. In one aspect, where a protein binds an antigen on a target cell to be eliminated and an activating receptor on an effector cell, a multispecific protein is capable of inducing activation of an immune effector cell (e.g. an NK cell, a T cell) when the protein is incubated in the presence of the effector cell and a target cell that expresses the antigen of interest). In one aspect, a multispecific protein is capable of inducing signaling at an immune effector cell activating receptor when the protein is incubated in the presence of the effector cell and a target cell that expresses the antigen of interest). Optionally, effector cell activation or signaling in characterized by increased expression of a cell surface marker of activation, e.g. CD107, CD69, etc. Activity can be measured for example by bringing target cells and effector cells into contact with one another, in presence of the multispecific polypeptide. In one example, aggregation of target cells and effector cells is measured. In another example, the multispecific protein may, for example, be assessed for the ability to cause a measurable increase in any property or activity known in the art as associated with NK cell activity, respectively, such as marker of cytotoxicity (CD107) or cytokine production (for example IFN-γ or TNF-α), increases in intracellular free calcium levels, the ability to lyse target cells in a redirected killing assay, etc. In one aspect of any of the methods of identifying, evaluating or making a protein, the method comprises a step of evaluating the multispecific protein for its ability to induce or increase the activity immune cells that express an activating receptor bound by an ABD multispecific protein (e.g. a marker of activation or cytotoxicity, cytokine production, ability to lyse a target cell, etc.), when incubated in the presence of the immune cells and target cells expressing an antigen of interest bound by an ABD of the multispecific protein (e.g. the cancer antigen). In one aspect, the immune cells express NKp46, CD16 and/or CD137. In one aspect, the cells are NKp46⁺ NK cells. In one aspect, the immune cells are CD16⁺ cells. In one aspect, the immune cells are CD137⁺ cells.

In the presence of target cells (target cells expressing the antigen of interest) and effector cells that express the activating receptor bound by the protein, the multispecific protein will be capable of causing an increase in a property or activity associated with effector (e.g. NK cell, T cell) cell activity (e.g. activation of NK cell cytotoxicity, CD107 expression, IFNγ production) *in vitro.* For example, a multispecific protein of the disclosure can be selected for the ability to increase an NK or T cell activity by more than about 20%, preferably with at least about 30%, at least about 40%, at least about 50%, or more compared to that achieved with the same effector: target cell ratio with the same NK or T cells and target cells that are not brought into contact with the multispecific protein, as measured by an assay of NK or T cell activity, e.g., a marker of activation of NK cell cytotoxicity, CD107 or CD69 expression, IFNy production, a classical *in vitro* chromium release test of cytotoxicity. Examples of protocols for activation and cytotoxicity assays are described in the Examples herein, as well as for example, in Pessino et al, J. Exp. Med, 1998, 188 (5): 953-960; Sivori et al, Eur J Immunol, 1999. 29:1656-1666; Brando et al, (2005) J. Leukoc. Biol. 78:359-371; EI-Sherbiny et al, (2007) Cancer Research 67(18):8444-9; and Nolte-'t Hoen et al, (2007) Blood 109:670-673).

In one aspect, evaluating heterotrimeric proteins for a characteristic of interest comprises evaluating the proteins for one or more properties selected from the group consisting of: binding to an antigen of interest, binding to an antigen on an immune effector cell, binding to a tumor, viral or bacterial antigen, binding to an FcRn receptor, binding to human CD16 and/or other Fc-domain mediated effector function(s), agonistic or antagonistic activity at a polypeptide to which the multimeric proteins binds, ability to modulate the activity (e.g. cause the death of) a cell expressing the antigen of interest, ability to direct a lymphocyte to a cell expressing the antigen of interest, ability to activate a lymphocyte in the presence and/or absence of a cell expressing the antigen of interest, NK cell activation, stability or half-life *in vitro* or *in vivo,* production yield, purity within a composition, and susceptibility to aggregate in solution.

In one aspect, the present disclosure provides a method for identifying or evaluating a protein, comprising the steps of:
(a) providing nucleic acid(s) encoding a protein described herein ;
(b) expressing said nucleic acid(s) in a host cell to produce said protein, respectively; and recovering said protein; and
(c) evaluating the protein produced for a biological activity of interest, e.g., an activity disclosed herein, the ability to mediate the lysis of target cells (that express antigen of interest). In one aspect, a plurality of different multispecific proteins are produced and evaluated.

In one aspect, the step (c) comprises:
(i) testing the ability of the protein to cause effector cells (e.g. NK cells, T cells) that express an activating receptor bound by the protein to mediate the lysis of target cells, when incubated with such effector cells in the presence of target cells (that express antigen of interest). Optionally, step (i) is followed by a step comprising: selecting a protein (e.g., for further development, for use as a medicament) that mediates the lysis of target cells.

In one aspect, a multispecific protein described herein that comprises and ABD that binds an activating receptor on an immune effector cell can for example be characterized by:
(a) being capable of inducing effector cells (e.g. T cell; NK cells) that express the activating receptor bound by the ABD of the multispecific protein to lyse target cells, when incubated in the presence of the effector cells and target cells; and
(b) lack of agonist activity at the activating receptor bound by the ABD when incubated with activating receptor-expressing effector cells in the absence of target cell; where the multispecific protein is capable of binding to CD16, the effector cells are CD16-negative cells, e.g. CD16- NK cells. Optionally, the effector cells are purified effector cells.

### Uses of compounds

In one aspect, provided is any of the compounds defined herein, particularly the inventive multispecific proteins or antibodies and/or cells which express same for use for the treatment, prevention or diagnosis of a disease in a mammal in need thereof. Provided also are any of the compounds defined above for use as a medicament or an active component or active substance in a medicament. In a further aspect the invention provides methods for preparing a pharmaceutical composition containing a compound as defined herein, to provide a solid or a liquid formulation for administration orally, topically, or by injection. Such a method or process at least comprises the step of mixing the compound with a pharmaceutically acceptable carrier.

The polypeptides described herein can be used to prevent or treat disorders that can be treated with antibodies, such as cancers, solid and non-solid tumors, hematological malignancies, infections such as viral or microbial/bacterial infections, and inflammatory or autoimmune disorders.

In one aspect, the an antigen of interest expressed on the surface of a malignant cell of a type cancer selected from the group consisting of: carcinoma, including that of the bladder, head and neck, breast, colon, kidney, liver, lung, ovary, prostate, pancreas, stomach, cervix, thyroid and skin, including squamous cell carcinoma; hematopoietic tumors of lymphoid lineage, including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias and promyelocytic leukemia; tumors of mesenchymal origin, including fibrosarcoma and rhabdomyosarcoma; other tumors, including neuroblastoma and glioma; tumors of the central and peripheral nervous system, including astrocytoma, neuroblastoma, glioma, and schwannomas; tumors of mesenchymal origin, including fibrosarcoma, rhabdomyosarcoma, and osteosarcoma; and other tumors, including melanoma, xeroderma pigmentosum, keratoacanthoma, seminoma, thyroid follicular cancer and teratocarcinoma, hematopoietic tumors of lymphoid lineage, for example T-cell and B-cell tumors, including but not limited to T-cell disorders such as T-prolymphocytic leukemia (T-PLL), including of the small cell and cerebriform cell type; large granular lymphocyte leukemia (LGL) preferably of the T-cell type; Sézary syndrome (SS); Adult T-cell leukemia lymphoma (ATLL); aid T-NHL hepatosplenic lymphoma; peripheral/post-thymic T cell lymphoma (pleomorphic and immunoblastic subtypes); angio immunoblastic T-cell lymphoma; angiocentric (nasal) T-cell lymphoma; anaplastic (Ki 1+) large cell lymphoma; intestinal T-cell lymphoma; T-lymphoblastic; and lymphoma/leukemia (T-Lbly/T-ALL).

In one asppect, the inventive multispecific polypeptides described herein can be used to prevent or treat a cancer selected from the group consisting of: carcinoma, including that of the bladder, head and neck, breast, colon, kidney, liver, lung, ovary, prostate, pancreas, stomach, cervix, thyroid and skin, including squamous cell carcinoma; hematopoietic tumors of lymphoid lineage, including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias and promyelocytic leukemia; tumors of mesenchymal origin, including fibrosarcoma and rhabdomyosarcoma; other tumors, including neuroblastoma and glioma; tumors of the central and peripheral nervous system, including astrocytoma, neuroblastoma, glioma, and schwannomas; tumors of mesenchymal origin, including fibrosarcoma, rhabdomyosarcoma, and osteosarcoma; and other tumors, including melanoma, xeroderma pigmentosum, keratoacanthoma, seminoma, thyroid follicular cancer and teratocarcinoma. Other exemplary disorders that can be treated according to the invention include hematopoietic tumors of lymphoid lineage, for example T-cell and B-cell tumors, including but not limited to T-cell disorders such as T-prolymphocytic leukemia (T-PLL), including of the small cell and cerebriform cell type; large granular lymphocyte leukemia (LGL) preferably of the T-cell type; Sézary syndrome (SS); Adult T-cell leukemia lymphoma (ATLL); aid T-NHL hepatosplenic lymphoma; peripheral/post-thymic T cell lymphoma (pleomorphic and immunoblastic subtypes); angio immunoblastic T-cell lymphoma; angiocentric (nasal) T-cell lymphoma; anaplastic (Ki 1+) large cell lymphoma; intestinal T-cell lymphoma; T-lymphoblastic; and lymphoma/leukaemia (T-Lbly/T-ALL).

In another aspect, the invention provides a multispecific protein for use for restoring or potentiating the activity of immune effector cells (e.g. NK cells or T cells) in a patient in need thereof (e.g. a patient having a cancer, or a viral, parasite or bacterial infection). In one aspect, this use is directed at increasing the activity of lymphocytes expressing the activating receptor bound by the multispecific protein.

In another aspect the subject multispecific proteins may be for use in combination with immune cells, particularly NK cells, derived from a patient who is to be treated or from a different donor, and these NK cells to be administered to a patient in need thereof such as a patient having a disease in which increased lymphocyte (e.g. NK cell) activity is beneficial or which is caused or characterized by insufficient NK cell activity, such as a cancer, or a viral or microbial, e.g., bacterial or parasite infection. As NK cells (unlike CAR-T cells) do not express TCRs, these NK cells, even those derived from different donors will not induce a GVHD reaction. (See e.g., Glienke et al., "Advantages and applications of CAR-expressing natural killer cells", Front. Pharmacol. 6, Art. 21:1-6 (2015); Hermanson and Kaufman, Front. Immunol. 6, Art. 195:1-6 (2015))

In one aspect, the multispecific protein of the disclosure is for use for a treatment in a therapeutically effective amount. A therapeutically effective amount may be any amount that has a therapeutic effect in a patient having a disease or disorder (or promotes, enhances, and/or induces such an effect in at least a substantial proportion of patients with the disease or disorder and substantially similar characteristics as the patient).

The multispecific proteins of the disclosure can be included in kits. The kits may optionally further contain any number of polypeptides and/or other compounds, e.g., 1, 2, 3, 4, or any other number of multispecific proteins and/or other compounds. It will be appreciated that this description of the contents of the kits is not limiting in any way. For example, the kit may contain other types of therapeutic compounds. Optionally, the kits also include instructions for using the polypeptides, e.g., detailing the herein-described methods.

The invention also provides pharmaceutical compositions comprising the subject multispecific proteins and optionally other compounds as defined above. A multispecific protein and optionally another compound may be to be administered in purified form together with a pharmaceutical carrier as a pharmaceutical composition. The form depends on the intended mode of administration and therapeutic or diagnostic application. The pharmaceutical carrier can be any compatible, nontoxic substance suitable to deliver the compounds to the patient. Pharmaceutically acceptable carriers are well known in the art and include, for example, aqueous solutions such as (sterile) water or physiologically buffered saline or other solvents or vehicles such as glycols, glycerol, oils such as olive oil or injectable organic esters, alcohol, fats, waxes, and inert solids A pharmaceutically acceptable carrier may further contain physiologically acceptable compounds that act for example to stabilize or to increase the absorption of the compounds Such physiologically acceptable compounds include, for example, carbohydrates, such as glucose, sucrose or dextrans, antioxidants, such as ascorbic acid or glutathione, chelating agents, low molecular weight proteins or other stabilizers or excipients One skilled in the art would know that the choice of a pharmaceutically acceptable carrier, including a physiologically acceptable compound, depends, for example, on the route of administration of the composition Pharmaceutically acceptable adjuvants, buffering agents, dispersing agents, and the like, may also be incorporated into the pharmaceutical compositions. Non-limiting examples of such adjuvants include by way of example inorganic and organic adjuvants such as alum, aluminum phosphate and aluminum hydroxide, squalene, liposomes, lipopolysaccharides, double stranded (ds) RNAs, single stranded(s-s) DNAs, and TLR agonists such as unmethylated CpG's.

Multispecific proteins according to the invention can be administered parenterally. Preparations of the compounds for parenteral administration must be sterile. Sterilization is readily accomplished by filtration through sterile filtration membranes, optionally prior to or following lyophilization and reconstitution. The parenteral route for administration of compounds is in accord with known methods, e.g. injection or infusion by intravenous, intraperitoneal, intramuscular, intraarterial, or intralesional routes. The compounds may be administered continuously by infusion or by bolus injection. A typical composition for intravenous infusion could be made up to contain 100 to 500 ml of sterile 0.9% NaCl or 5% glucose optionally supplemented with a 20% albumin solution and 1 mg to 10 g of the compound, depending on the particular type of compound and its required dosing regimen. Methods for preparing parenterally administrable compositions are well known in the art.

### EXAMPLES

### Example 1

### Generation of anti-huNKp46 antibodies

Balb/c mice were immunized with a recombinant human NKp46 extracellular domain recombinant-Fc protein. Mice received one primo-immunization with an emulsion of 50 µg NKp46 protein and Complete Freund Adjuvant, intraperitoneally, a 2nd immunization with an emulsion of 50 µg NKp46 protein and Incomplete Freund Adjuvant, intraperitoneally, and finally a boost with 10 µg NKp46 protein, intravenously. Immune spleen cells were fused 3 days after the boost with X63.Ag8.653 immortalized B cells, and cultured in the presence of irradiated spleen cells.

Primary screen: Supernatant (SN) of growing clones were tested in a primary screen by flow cytometry using a cell line expressing the human NKp46 construct at the cell surface. Briefly, for FACS screening, the presence of reactive antibodies in supernatants was revealed by Goat anti-mouse polyclonal antibody (pAb) labeled with PE.

A panel of antibodies that bound NKp46 was selected, produced and their variable regions sequenced and these antibodies and derivatives thereof further evaluated for their activity in the context of a bispecific molecule.

### Example 2:

### Identification of a bispecific antibody format that binds FcRn but not FcγR for targeting effector cell receptors

Experiments were conducted with the objective being the development of a new bispecific protein format that places an Fc domain on a polypeptide together with an anti-NKp46 binding domain and an anti-target antigen binding domain. Such bispecific proteins should bind to NKp46 monovalently via its anti-NKp46 binding domain. The monomeric Fc domain should retain at least partial binding to the human neonatal Fc receptor (FcRn), yet not substantially bind human CD16 and/or other human Fcy receptors. Consequently, such bispecific proteins should not induce Fcy-mediated (e.g. CD16-mediated) target cell lysis.

### Example 2-1 Construction and binding analysis of Anti-CD19-IgG1-Fcmono-Anti-CD3

Since no anti-NKp46 bispecific antibody has been produced that could indicate whether such a protein could be functional, CD3 was used as a model antigen in place of NKp46 in order to investigate the possible functionality of a new monovalent bispecific protein format prior to targeting NK cells via NKp46.

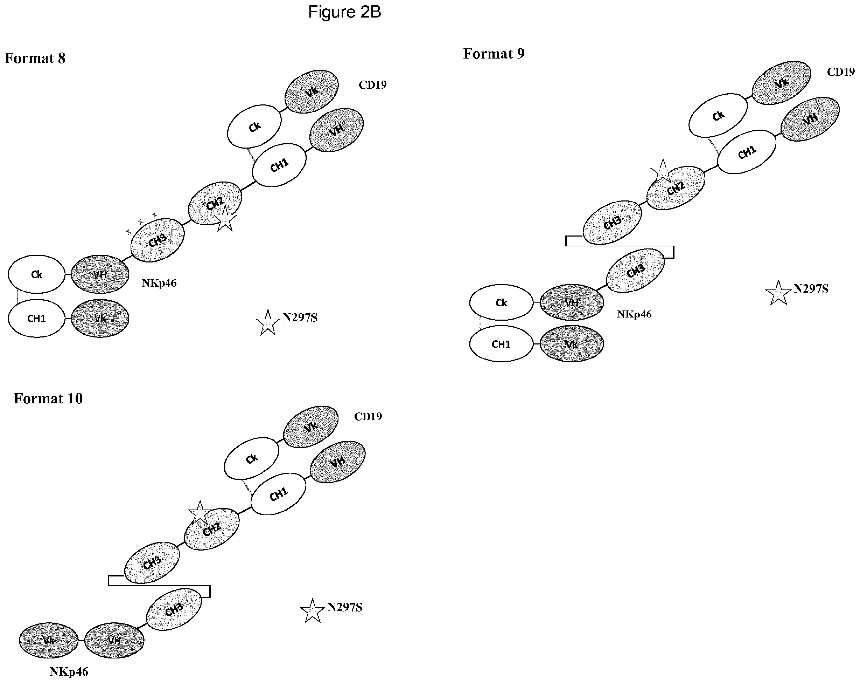

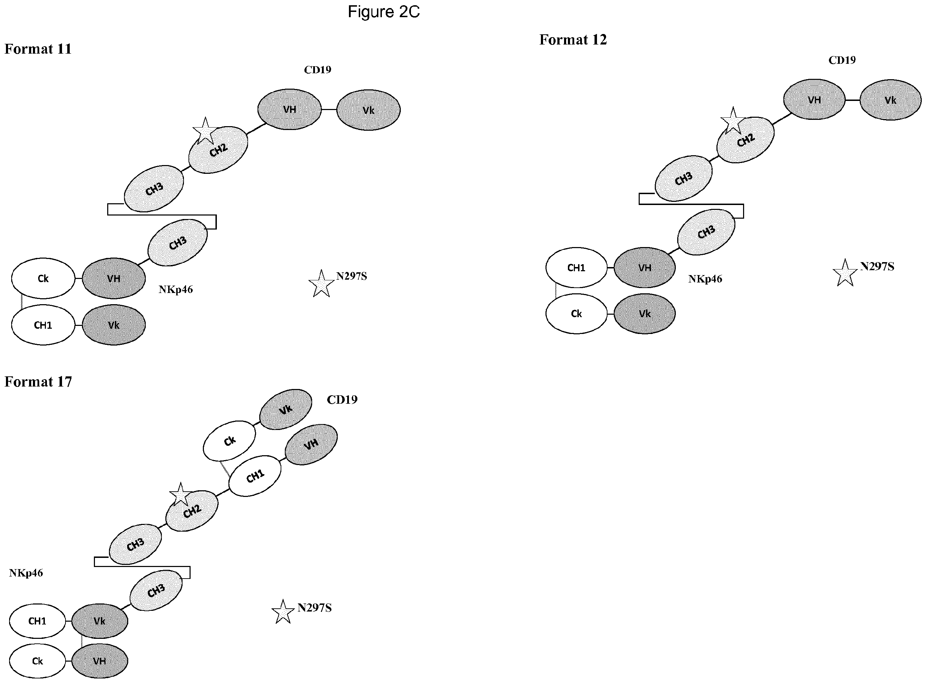

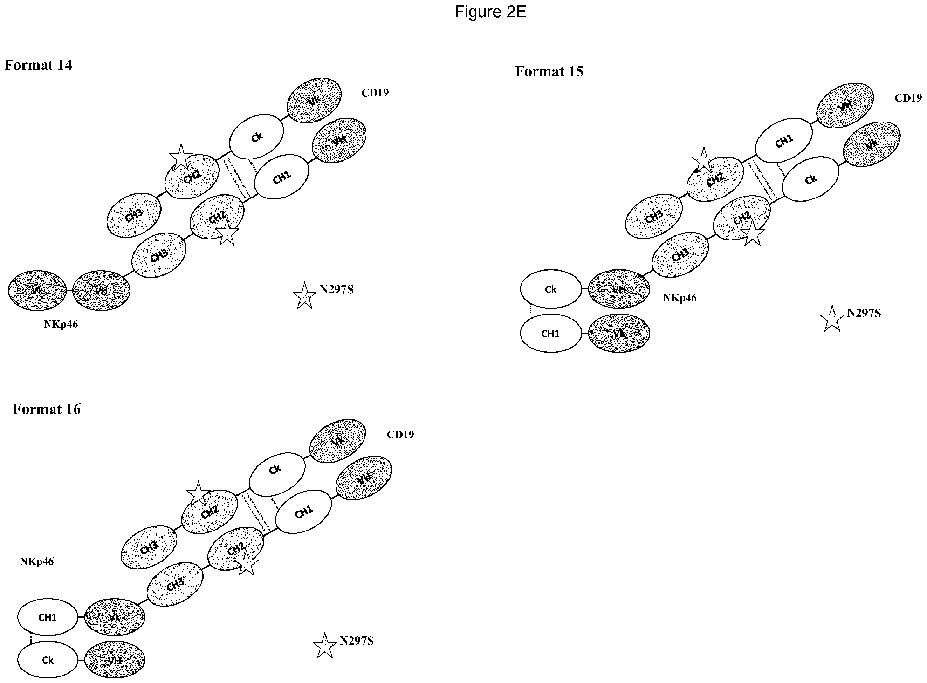

A bispecific Fc-based on a scFv specific for tumor antigen CD19 (anti-CD19 scFv) and a scFv specific for activating receptor CD3 on a T cell (anti-CD3 scFv) was used to assess FcRn binding and CD19-binding functions of a new monomeric bispecific polypeptide format. The domain arrangement of the final polypeptide is referred to as the "F1" format (the star in the CH2 domain indicates an optional N297S mutation, not included in the polypeptide tested here). (See **Figure 2**)

A bispecific monomeric Fc-containing polypeptide was constructed based on a scFv specific for the tumor antigen CD19 (anti-CD19 scFv) and a scFv specific for an activating receptor CD3 on a T cell (anti-CD3 scFv). The CH3 domain incorporated the mutations (EU numbering) L351 K, T366S, P395V, F405R, T407A and K409Y. The polypeptide has domains arranged as follows: anti-CD19-CH2-CH3-anti-CD3. A DNA sequence coding for a CH3/VH linker peptide having the amino acid sequence STGS was also designed in order to insert a specific Sall restriction site at the CH3-VH junction.

This CH3 domain incorporated the mutations (EU numbering) L351 K, T366S, P395V, F405R, T407A and K409Y. The selected CH2 domain was a wild-type CH2. DNA and amino acid sequences for the monomeric CH2-CH3 Fc portion and the anti-CD19 are shown below.The light chain and heavy chain DNA and amino acid sequences for the anti-CD19 scFv were as follows:
Anti-CD19-VK 
GACATTCAGCTGACCCAATCTCCAGCTTCTTTGGCTGTGTCTCTAGGGCAGAGGGCCACCATCTCCTG
CAAGGCCAGCCAAAGTGTTGATTATGATGGTGATAGTTATTTGAACTGGTACCAACAGATACCAGGAC
AGCCACCCAAACTCCTCATCTATGATGCATCCAATCTAGTATCTGGGATTCCACCCAGGTTTAGTGGC
AGTGGGTCTGGGACAGACTTCACCCTCAACATCCATCCTGTGGAGAAGGTGGATGCTGCAACCTATCA
CTGTCAGCAAAGTACTGAGGACCCTTGGACGTTCGGTGGAGGCACCAAGCTGGAAATCAAA (SEQ ID
NO: 3)
Anti-CD19-VK 
DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIK (SEQ ID NO: 4)
 Anti-CD19-VH 
CAGGTTCAGCTGCAGCAGTCTGGGGCTGAGCTGGTGCGGCCTGGGTCCTCAGTGAAGATTTCCTGCAA
AGCATCTGGCTACGCATTCAGTAGCTACTGGATGAACTGGGTGAAGCAGAGGCCTGGACAGGGTCTTG
AGTGGATTGGACAGATTTGGCCTGGAGATGGTGATACTAACTACAACGGAAAGTTCAAGGGCAAGGCC
ACACTGACTGCAGACGAATCCTCCAGCACAGCCTACATGCAGCTCAGCAGCCTGGCCTCTGAGGACTC
TGCGGTCTATTTCTGTGCAAGACGAGAAACGACCACTGTCGGGCGTTATTACTATGCTATGGACTACT
GGGGTCAAGGAACCACAGTCACCGTCTCCTCA (SEQ ID NO: 5)
Anti-CD19-VH 
QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSS
 (SEQ ID NO: 6)

The DNA and amino acid sequences for the monomeric CH2-CH3 Fc portion and final bispecific polypeptide were as follows:
IgG1-Fcmono (the last K was removed in that construct) 
GCACCTGAACTCCTGGGGGGACCGTCAGTCTTCCTCTTCCCCCCAAAACCCAAGGACACCCTCATGAT
CTCCCGGACCCCTGAGGTCACATGCGTGGTGGTGGACGTGAGCCACGAAGACCCTGAGGTCAAGTTCA
ACTGGTACGTGGACGGCGTGGAGGTGCATAATGCCAAGACAAAGCCGCGGGAGGAGCAGTACAACAGC
ACGTACCGTGTGGTCAGCGTCCTCACCGTCCTGCACCAGGACTGGCTGAATGGCAAGGAGTACAAGTG
CAAGGTCTCCAACAAAGCCCTCCCAGCCCCCATCGAGAAAACCATCTCCAAAGCCAAAGGGCAGCCCC
GAGAACCACAGGTGTACACCAAGCCCCCATCCCGGGAGGAGATGACCAAGAACCAGGTCAGCCTGTCC
TGCCTGGTCAAAGGCTTCTATCCCAGCGACATCGCCGTGGAGTGGGAGAGCAATGGGCAGCCGGAGAA
CAACTACAAGACCACGGTTCCCGTGCTGGACTCCGACGGCTCCTTCCGCCTCGCTAGCTACCTCACCG
 
TGGACAAGAGCAGGTGGCAGCAGGGGAACGTCTTCTCATGCTCCGTGATGCATGAGGCTCTGCACAAC
CACTACACGCAGAAGAGCCTCTCCCTGTCCCCGGGG (SEQ ID NO : 7)
IgG1-Fcmono* (*the last K residue was removed in that construct) 
APELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTKPPSREEMTKNQVSLS
CLVKGFYPSDIAVEWESNGQPENNYKTTVPVLDSDGSFRLASYLTVDKSRWQQGNVFSCSVMHEALHN
HYTQKSLSLSPG (SEQ ID NO: 8)
Anti-CD19-F1-Anti-CD3 Complete sequence (mature protein)

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKGGGGSGGGGSGGGGSQVQLQQSGAE
LVRPGSSVKISCKASGYAFSSYWMNWVKQRPGQGLEWIGQIWPGDGDTNYNGKFKGKATLTADESSST
AYMQLSSLASEDSAVYFCARRETTTVGRYYYAMDYWGQGTTVTVSSGGGSSAPELLGGPSVFLFPPKP
KDTLMISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTKPPSREEMTKNQVSLSCLVKGFYPSDIAVEWES
NGQPENNYKTTVPVLDSDGSFRLASYLTVDKSRWQQGNVFSCSVMHEALHNHYTQKSLSLSPGSTGSD
IKLQQSGAELARPGASVKMSCKTSGYTFTRYTMHWVKQRPGQGLEWIGYINPSRGYTNYNQKFKDKAT
LTTDKSSSTAYMQLSSLTSEDSAVYYCARYYDDHYCLDYWGQGTTLTVSSVEGGSGGSGGSGGSGGVD
DIQLTQSPAIMSASPGEKVTMTCRASSSVSYMNWYQQKSGTSPKRWIYDTSKVASGVPYRFSGSGSGT
SYSLTISSMEAEDAATYYCQQWSSNPLTFGAGTKLELK (SEQ ID NO: 9)

### Cloning and the production of the recombinant proteins

Coding sequences were generated by direct synthesis and/or by PCR. PCR was performed using the PrimeSTAR MAX DNA polymerase (Takara, #R045A) and PCR products were purified from 1% agarose gel using the NucleoSpin gel and PCR clean-up kit (Macherey-Nagel, #740609.250). Once purified the PCR products were quantified prior to the In-Fusion ligation reaction which was performed as described in the manufacturer's protocol (ClonTech, #ST0345). The plasmids were obtained after a miniprep preparation run on an EVO200 (Tecan) using the Nucleospin 96 plasmid kit (Macherey-Nagel, #740625.4). Plasmids were then sequenced for sequence confirmation before to transfecting the CHO cell line.

CHO cells were grown in the CD-CHO medium (Invitrogen) complemented with phenol red and 6 mM GlutaMax. The day before the transfection, cells were counted and seeded at 175,000 cells/ml. For the transfection, cells (200.000 cells/transfection) were prepared as described in the AMAXA SF cell line kit (AMAXA, #V4XC-2032) and nucleofected using the DS137 protocol with the Nucleofector 4D device. All the transfections were performed using 300 ng of verified plasmids. After transfection, cells were seeded into 24 well plates in prewarmed culture medium. After 24 hours, hygromycin B was added in the culture medium (200 µg/ml). Protein expression was monitored after one week in culture. Cells expressing the proteins were then sub-cloned to obtain the best producers. Sub-cloning was performed using 96 flat-bottom well plates in which the cells are seeded at one cell per well into 200 µl of culture medium complemented with 200 µg/ml of hygromycin B. Cells were left for three weeks before testing the clone's productivity.

Recombinant proteins which contain an IgG1-Fc fragment were purified using Protein-A beads (- rProteinA Sepharose fast flow, GE Healthcare). Briefly, cell culture supernatants were concentrated, clarified by centrifugation and injected onto Protein-A columns to capture the recombinant Fc containing proteins. Proteins were eluted at acidic pH (citric acid 0.1M pH 3), and the eluate immediately neutralized using TRIS-HCL pH 8.5 and dialyzed against 1X PBS. Recombinant scFvs which contain a "six his" tag were purified by affinity chromatography using Cobalt resin. Other recombinant scFvs were purified by size exclusion chromatography (SEC).

### Example 2-2: Binding analysis of Anti-CD19-IgG1-Fcmono-Anti-CD3 to B221, JURKAT, HUT78 and CHO cell lines

Cells were harvested and stained with the cell supernatant of the anti-CD19-F1-anti-CD3 producing cells during 1 H at 4°C. After two washes in staining buffer (PBS1X / BSA 0.2% / EDTA 2mM), cells were stained for 30 min at 4°C with goat anti-human (Fc)-PE antibody (IM0550 Beckman Coulter - 1/200). After two washes, stainings were conducted on a BD FACS Canto II and analyzed using the FlowJo software.

CD3 and CD19 expression were also controlled by flow cytometry: Cells were harvested and stained in PBS1X / BSA 0.2% / EDTA 2mM buffer during 30 min at 4°C using 5µl of the anti-CD3-APC and 5µl of the anti-CD19-FITC antibodies. After two washes, stainings were conducted on a BD FACS Canto II and analyzed using the FlowJo software.

The results of these experiments revealed that the Anti-CD19-F1-Anti-CD3 protein binds to CD3 cell lines (HUT78 and JURKAT cell lines) and to the CD19 cell line (B221 cell line) but not to the CHO cell line which was used as a negative control.

### Example 2-3: T- and B- cell aggregation by purified Anti-CD19-F1-Anti-CD3

Purified Anti-CD19-F1-Anti-CD3 was tested in a T/B cell aggregation assay to evaluate whether the antibody promotes the aggregation of CD19 and CD3 expressing cells.

The results of this assay are shown in **Figure 1****.** The top panel shows that Anti-CD19-F1-Anti-CD3 does not cause aggregation in the presence of B221 (CD19) or JURKAT (CD3) cell lines, but it does cause aggregation of cells when both B221 and JURKAT cells are co-incubated, indicating that the bispecific antibody is functional. The lower panel shows the results of the control experiment conducted without antibody.

### Example 2-4: Binding of bispecific monomeric Fc polypeptide to FcRn

### Affinity study by Surface Plasmon Resonance (SPR)

### Biacore T100 general procedure and reagents

SPR measurements were performed on a Biacore T100 apparatus (Biacore GE Healthcare) at 25°C. In all Biacore experiments Acetate Buffer (50 mM Acetate pH5.6, 150 mM NaCl, 0.1% surfactant p20) and HBS-EP+ (Biacore GE Healthcare) were used as the running buffer and regeneration buffer respectively. Sensorgrams were analyzed with Biacore T100 Evaluation software. Recombinant mouse FcRn was purchased from R&D Systems.

### Immobilization of FcRn

Recombinant FcRn proteins were immobilized covalently to carboxyl groups in the dextran layer on a Sensor Chip CM5. The chip surface was activated with EDC/NHS (N-ethyl-N'-(3-dimethylaminopropyl) carbodiimidehydrochloride and N-hydroxysuccinimide (Biacore GE Healthcare)). FcRn proteins were diluted to 10 µg/ml in coupling buffer (10 mM acetate, pH 5.6) and injected until the appropriate immobilization level was reached (i.e. 2500 RU). Deactivation of the remaining activated groups was performed using 100 mM ethanolamine pH 8 (Biacore GE Healthcare).

### Affinity study

Monovalent affinity study was conducted following the Single Cycle Kinetic (SCK) protocol. Five serial dilutions of soluble analytes (antibodies and bi-specific molecules) ranging from 41.5 to 660 nM were injected over the FcRn (without regeneration) and allowed to dissociate for 10 min before regeneration. For each analyte, the entire sensorgram was fitted using the 1:1 SCK binding model.

### Results

Anti-CD19-F1-Anti-CD3 having its CH2-CH3 domains placed between two antigen binding domains, particularly two scFvs, was evaluated to assess whether such bispecific monomeric Fc protein could retain binding to FcRn and possess an improved *in vivo* half-life compared to conventional bispecific antibodies. The results of these experiments showed that FcRn binding was retained, the model suggesting a 1:1 ratio (1 FcRn for each monomeric Fc) instead of a 2:1 ratio (2 FcRn for each antibody) for a regular or wild-type IgG.

The binding affinity of this multispecific protein was evaluated using SPR, and was compared to a chimeric full length antibody containing intact human IgG1 constant regions. The monomeric Fc retained significant monomeric binding to FcRn (monomeric Fc: affinity of KD=194 nM; full length antibody with bivalent binding: avidity of KD=15.4 nM).

### Example 3: Construction of multimeric bispecific proteins with monomeric-Fc

Activating receptors on effector cells such as NK cells can contribute to the activation of the effector cell and/or lysis of target cells, yet conventional antibodies can block the activating receptor, exemplifieid by anti-NKp46 antibodies which as full length IgG1 block NKp46 signalling. We therefore investigated whether the bispecific protein format could induce NKp46 triggering, without inducing NKp46 agonism in the absence of target cells, which could lead to inappropriate NK activation distant from the target and/or decreased overall activity toward target cells.

A new bispecific protein format was developed as a single chain protein which binds to FcRn but not FcyR. Additionally, multimeric proteins that comprise two or three polypeptide chains, wherein the Fc domain remains monomeric, were developed that are compatible for use with antibody variable regions that do not maintain binding to their target when converted to scFv format. The latter formats can be used conveniently for antibody screening; by incorporating at least one binding region as a F(ab) structure, any anti-target (e.g. anti-tumor) antibody variable region can be directly expressed in a bispecific construct as the F(ab) format within the bispecific protein and tested, irrespective of whether the antibody would retain binding as an scFv, thereby simplifying screening and enhancing the number of antibodies available. These formats in which the Fc domain remains monomeric have the advantage of maintaining maximum conformational flexibility and as shown *infra* may permit optimal binding to activating receptor (e.g. NKp46) or target antigens.

Different constructs were made for use in the preparation of bispecific antibodies using the variable domains from the scFv specific for tumor antigen CD19 described in Example 2-1, and different variable regions from antibodies specific for the NKp46 receptor identified in Example 1.

In order for the Fc domain to remain monomeric in single chain polypeptides or in multimers in which only one chain had an Fc domain, CH3-CH3 dimerization was prevented through two different strategies: (1) through the use of CH3 domain incorporating specific mutations (EU numbering), i.e., L351K, T366S, P395V, F405R, T407A and K409Y; or (2) through the use of a tandem CH3 domain in which the tandem CH3 domains are separated by a flexible linker associated with one another, which prevents interchain CH3-CH3 dimerization. The DNA and amino acid sequences for the monomeric CH2-CH3 Fc portion containing the above-identified point mutations were the same as in Example 2-1. The DNA and amino acid sequences for the monomeric CH2-CH3-linker-CH3 Fc portion with tandem CH3 domains are shown in **Figures 2A-2D****.**

The light chain and heavy chain DNA and amino acid sequences for the anti-CD19 scFv were also the same as in Example 2-1. Proteins were cloned, produced and purified as in Example 2-1. Shown below are an exemplary light chain and heavy chain DNA and amino acid sequences for an anti-NKp46 scFv referred to as "NKp46-3".

| scFv anti-N Kp46 | scFv sequence (VHVK) / - stop |
|---|---|
| NKp46-3 | STGSEVQLQQSGPELVKPGASVKISCKTSGYTFTEYTMHWVKQSHGKSLEWIGGI SPNIGGTSYNQKFKGKATLTVDKSSSTAYMELRSLTSEDSAVYYCARRGGSFDYW GQGTTLTVSSVEGGSGGSGGSGGSGGVDDIVMTQSPATLSVTPGDRVSLSCRASQ SISDYLHWYQQKSHESPRLLIKYASQSISGIPSRFSGSGSGSDFTLSINSVEPED VGVYYCQNGHSFPLTFGAGTKLELK- (SEQ ID NO: 10) |

### Format 1 (F1) (Anti-CD19-IgG1-Fcmono-Anti-NKp46 (scFv))

The domain structure of Format 1 (F1) is shown in **Figure 2A****.** A bispecific Fc-containing polypeptide was constructed based on a scFv specific for the tumor antigen CD19 (anti-CD19 scFv) and an scFV specific for the NKp46 receptor. The polypeptide is a single chain polypeptide having domains arranged (N- to C- termini) as follows:

(Vκ-V_{H})^{anti-CD19} - CH2 - CH3 - (V_{H}-Vκ)^{anti-NKp46}

A DNA sequence coding for a CH3/VH linker peptide having the amino acid sequence STGS was designed in order to insert a specific Sall restriction site at the CH3-VH junction. The domain arrangement of the final polypeptide in shown in Figure 2 (star in the CH2 domain indicates an optional N297S mutation). The (Vκ-V_{H}) units include a linker between the V_{H} and Vκ domains. Proteins were cloned, produced and purified as in Example 2-1. The amino acid sequence is shown as follows:

DIQLTQSPASLAVSLGQRATISCKASQSVDYDGDSYLNWYQQIPGQPPKLLIYDASNLVSGIPPRFSGSGSGTDF
TLNIHPVEKVDAATYHCQQSTEDPWTFGGGTKLEIKGGGGSGGGGSGGGGSQVQLQQSGAELVRPGSSVKISCKA
SGYAFSSYWMNWVKQRPGQGLEWIGQIWPGDGDTNYNGKFKGKATLTADESSSTAYMQLSSLASEDSAVYFCARR
ETTTVGRYYYAMDYWGQGTTVTVSSGGGSSAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQPREPQVYT
KPPSREEMTKNQVSLSCLVKGFYPSDIAVEWESNGQPENNYKTTVPVLDSDGSFRLASYLTVDKSRWQQGNVFSC
SVMHEALHNHYTQKSLSLSPGSTGSEVQLQQSGPELVKPGASVKISCKTSGYTFTEYTMHWVKQSHGKSLEWIGG
ISPNIGGTSYNQKFKGKATLTVDKSSSTAYMELRSLTSEDSAVYYCARRGGSFDYWGQGTTLTVSSVEGGSGGSG
GSGGSGGVDDIVMTQSPATLSVTPGDRVSLSCRASQSISDYLHWYQQKSHESPRLLIKYASQSISGIPSRFSGSG
SGSDFTLSINSVEPEDVGVYYCQNGHSFPLTFGAGTKLELK-

### Format 2 (F2) : CD19-F2-NKp46-3

The domain structure of F2 polypeptides is shown in **Figure 2A****.** The DNA and amino acid sequences for the monomeric CH2-CH3 Fc portion were as in Example 2-1 and it similarly contains CH3 domain mutations (the mutations (EU numbering) L351K, T366S, P395V, F405R, T407A and K409Y. The heterodimer is made up of:
(1) a first (central) polypeptide chain having domains arranged as follows (N- to C-termini):

   (Vκ-V_{H})^{anti-CD19} - CH2 - CH3 - V_{H}^{anti-NKp46} - CH1 and
(2) a second polypeptide chain having domains arranged as follows (N- to C- termini): VK^{anti-NKp46} - CK.

The (VK-VH) unit was made up of a VH domain, a linker and a VK unit (i.e. an scFv). As with other formats of the bispecific polypeptides, the DNA sequence coded for a CH3/VH linker peptide having the amino acid sequence STGS designed in order to insert a specific Sall restriction site at the CH3-VH junction. Proteins were cloned, produced and purified as in Example 2-1. The amino acid sequences for the CD19-F2-NKp46-3 Polypeptide chain 1 is shown in SEQ ID NO: 11 and CD19-F2-NKp46-3 Polypeptide chain 2 in SEQ ID NO: 12.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKGGGGSGGGGSGGGGSQVQLQQSGAE
LVRPGSSVKISCKASGYAFSSYWMNWVKQRPGQGLEWIGQIWPGDGDTNYNGKFKGKATLTADESSST
AYMQLSSLASEDSAVYFCARRETTTVGRYYYAMDYWGQGTTVTVSSGGGSSAPELLGGPSVFLFPPKP
KDTLMISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTKPPSREEMTKNQVSLSCLVKGFYPSDIAVEWES
NGQPENNYKTTVPVLDSDGSFRLASYLTVDKSRWQQGNVFSCSVMHEALHNHYTQKSLSLSPGSTGSE
VQLQQSGPELVKPGASVKISCKTSGYTFTEYTMHWVKQSHGKSLEWIGGISPNIGGTSYNQKFKGKAT
LTVDKSSSTAYMELRSLTSEDSAVYYCARRGGSFDYWGQGTTLTVSSASTKGPSVFPLAPSSKSTSGG
TAALGCLVKDYFPEPVTVSWNSGALTSGVHTFPAVLQSSGLYSLSSVVTVPSSSLGTQTYICNVNHKP
SNTKVDKRVEPKSCDKTH- (SEQ ID NO: 11)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC- (SEQ ID NO: 12)

### Format 8 (F8)

The domain structure of F8 polypeptides is shown in **Figure 2B****.** The DNA and amino acid sequences for the monomeric CH2-CH3 Fc portion were as in Format F2 and it similarly contains CH3 domain mutations (the mutations (EU numbering) L351K, T366S, P395V, F405R, T407A and K409Y, as well as a N297S mutation which prevents N-linked glycosylation and moreover abolishes FcyR binding. Three variants of F8 proteins were produced: (a) one wherein the cysteine residues in the hinge region were left intact (wild-type, referred to as F8A), (b) a second wherein the cysteine residues in the hinge region were replaced by serine residues (F8B), and (c) a third including a linker sequence GGGSS replacing residues DKTHTCPPCP in the hinge (F8C). Variants F8B and F8C provided production advantages as these versions avoided the formation of homodimers of the central chain. This heterotrimer is made up of;
(1) a first (central) polypeptide chain having domains arranged as follows (N- to C-termini):

   V_{H}^{anti-CD19} - CH1 - CH2 - CH3 - VH^{anti-NKP46} - Cκ and
(2) a second polypeptide chain having domains arranged as follows (N- to C- termini):

   VK^{anti-NKp46} - CH1

   and
(3) a third polypeptide chain having domains arranged as follows (N- to C- termini):

   Vκ ^{anti-CD19} - Cκ.

Proteins were cloned, produced and purified as in Example 2-1. Bispecific proteins was purified from cell culture supernatant by affinity chromatography using prot-A beads and analysed and purified by SEC. The protein showed a high production yield of 3.7 mg/L (F8C) and with a simple SEC profile. The amino acid sequences for the three F8 protein chains for the F8 "C" variant are shown in SEQ ID NOS 13, 14 and 15.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC- (SEQ ID NO: 13)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCGGGSSAPELLGGPSVFLFPPKPKDTLMISRTPEVTCVVVDVSHED
PEVKFNWYVDGVEVHNAKTKPREEQYSSTYRVVSVLTVLHQDWLNGKEYKCKVSNKALPAPIEKTISK
AKGQPREPQVYTKPPSREEMTKNQVSLSCLVKGFYPSDIAVEWESNGQPENNYKTTVPVLDSDGSFRL
ASYLTVDKSRWQQGNVFSCSVMHEALHNHYTQKSLSLSPGSTGSEVQLQQSGPELVKPGASVKISCKT
SGYTFTEYTMHWVKQSHGKSLEWIGGISPNIGGTSYNQKFKGKATLTVDKSSSTAYMELRSLTSEDSA
VYYCARRGGSFDYWGQGTTLTVSSRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGEC-
(SEQ ID NO: 14)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV

KDYFPEPVTVSWNSGALTSGVHTFPAVLQSSGLYSLSSVVTVPSSSLGTQTYICNVNHKPSNTKVDKR
VEPKSCDKTH- (SEQ ID NO: 15)

### Format 9 (F9): CD19-F9-NKp46-3

The F9 polypeptide is a trimeric polypeptide having a central polypeptide chain and two polypeptide chains each of which associate with the central chain via CH1-Cκ dimerization. The domain structure of the trimeric F9 protein is shown in **Figure 2B****,** wherein the bonds between the CH1 and Cκ domains are interchain disulfide bonds. The two antigen binding domains have a F(ab) structure permitting the use of these antibodies irrespective of whether they remain functional in a scFv format. The DNA and amino acid sequences for the CH2-CH3 Fc portion comprise a tandem CH3 domain as in Format F4 and comprise a CH2 domain comprising a N297S substitution. Three variants of F9 proteins were produced: (a) a first wherein the cysteine residues in the hinge region left intact (wild-type, referred to as F9A), (b) a second wherein the cysteine residues in the hinge region were replaced by serine residues (F9B), and (c) a third containing a linker sequence GGGSS which replaces residues DKTHTCPPCP in the hinge (F9C). Variants F9B and F9C provided advantages in production by avoiding the formation of homodimers of the central chain. The heterotrimer is made up of:
(1) a first (central) polypeptide chain having domains arranged as follows (N- to C-termini):

   V_{H}^{anti-CD19} - CH1 - CH2- CH3 - CH3 - V_{H}^{anti-NKp46} - Cκ

   and
(2) a second polypeptide chain having domains arranged as follows (N- to C- termini):

   Vκ^{anti-NKp46} - CH1

   and
(3) a third polypeptide chain having domains arranged as follows (N- to C- termini):

   VK ^{anti-CD19} - CK.

Proteins were cloned, produced and purified as in Example 2-1. Bispecific proteins was purified from cell culture supernatant by affinity chromatography using prot-A beads and analysed and purified by SEC. The protein showed a high production yield of 8.7 mg/L (F9A) and 3.0 mg/L (F9B), and with a simple SEC profile. The amino acid sequences for the three F9 protein chains for each of variants F9A, F9B and F9C are shown in the SEQ ID NOS listed in the table below.

| Protein | SEQ ID NOS |
|---|---|
| F9A | 16, 17, 18 |
| F9B | 19, 20, 21 |
| F9C | 22, 23, 24 |

F9A: 
DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC- (SEQ ID NO: 16)
 
QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGSGQPREPQV
YTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGSTGSEVQLQQSGPELVKPGASVKISCKTSGYTFTEYTM
HWVKQSHGKSLEWIGGISPNIGGTSYNQKFKGKATLTVDKSSSTAYMELRSLTSEDSAVYYCARRGGS
FDYWGQGTTLTVSSRTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC- (SEQ ID NO: 17)
 
DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH- (SEQ ID NO: 18)
F9B: 
DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC- (SEQ ID NO: 19)
 
QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTSPPSPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGSGQPREPQV
YTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGSTGSEVQLQQSGPELVKPGASVKISCKTSGYTFTEYTM
HWVKQSHGKSLEWIGGISPNIGGTSYNQKFKGKATLTVDKSSSTAYMELRSLTSEDSAVYYCARRGGS
FDYWGQGTTLTVSSRTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC- (SEQ ID NO: 20)
 
DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH- (SEQ ID NO: 21)
F9C:

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV

VCLLNNFYPREAKVQWKVDNALQSGNSQESVTEQDSKDSTYSLSSTLTLSKADYEKHKVYACEVTHQG
LSSPVTKSFNRGEC- (SEQ ID NO: 22)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCGGGSSAPELLGGPSVFLFPPKPKDTLMISRTPEVTCVVVDVSHED
PEVKFNWYVDGVEVHNAKTKPREEQYSSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGGGGGSGGGGSGGGGSGQPREPQVYTLPP
SREEMTKNQVSLTCLVKGFYPSDIAVEWESNGQPENNYKTTPPVLDSDGSFFLYSKLTVDKSRWQQGN
VFSCSVMHEALHNHYTQKSLSLSPGSTGSEVQLQQSGPELVKPGASVKISCKTSGYTFTEYTMHWVKQ
SHGKSLEWIGGISPNIGGTSYNQKFKGKATLTVDKSSSTAYMELRSLTSEDSAVYYCARRGGSFDYWG
QGTTLTVSSRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC- (SEQ ID NO: 23)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH- (SEQ ID NO: 24)

### Format 10 (F10): CD19-F10-NKp46-3

The F10 polypeptide is a dimeric protein having a central polypeptide chain and a second polypeptide chain which associates with the central chain via CH1-Cκ dimerization. The domain structure of the dimeric F10 protein is shown in **Figure 2B** wherein the bonds between the CH1 and Cκ domains are interchain disulfide bonds. One of the two antigen binding domains has a Fab structure, and the other is a scFv. The DNA and amino acid sequences for the CH2-CH3 Fc portion comprise a tandem CH3 domain as shown in Format F4 and comprise a CH2 domain containing a N297S substitution. Three variants of F10 proteins were also produced: (a) a first wherein the cysteine residues in the hinge region were left intact (wild-type, referred to as F10A), (b) a second wherein the cysteine residues in the hinge region were replaced by serine residues (F10B), and (c) a third containing a linker sequence GGGSS replacing residues DKTHTCPPCP in the hinge (F10C). Variants F10B and F10C provided advantages in production as they avoid the formation of homodimers of the central chain. The (Vκ-V_{H}) unit was made up of a V_{H} domain, a linker and a Vκ unit (scFv). The heterodimer is made up of:
(1) a first (central) polypeptide chain having domains arranged as follows (N- to C-termini):

   V_{H}^{anti-CD19} - CH1- CH2- CH3 - CH3 - (V_{H} - Vκ)^{anti-NKp46}

   and
(2) a second polypeptide chain having domains arranged as follows (N- to C- termini):

   Vκ ^{anti-CD19} - CK.

Proteins were cloned, produced and purified as in Example 2-1. Bispecific proteins was purified from cell culture supernatant by affinity chromatography using prot-A beads and analysed and purified by SEC. The protein showed a good production yield of 2 mg/L (F10A) and with a simple SEC profile. The amino acid sequences for the three F9 protein chains for each of variants F10A, F10B and F10C are shown in the SEQ ID NOS listed in the table below.

| Protein | SEQ ID NOS |
|---|---|
| F10A | 25, 26 |
| F10B | 27, 28 |
| F10C | 29, 30 |

F10A: 
DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC- (SEQ ID NO: 25)
 
QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGSGQPREPQV
YTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGSTGSEVQLQQSGPELVKPGASVKISCKTSGYTFTEYTM
HWVKQSHGKSLEWIGGISPNIGGTSYNQKFKGKATLTVDKSSSTAYMELRSLTSEDSAVYYCARRGGS
FDYWGQGTTLTVSSVEGGSGGSGGSGGSGGVDDIVMTQSPATLSVTPGDRVSLSCRASQSISDYLHWY
QQKSHESPRLLIKYASQSISGIPSRFSGSGSGSDFTLSINSVEPEDVGVYYCQNGHSFPLTFGAGTKL
ELK- (SEQ ID NO: 26)
F10B:
   - Fragl 
DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC-(SEQ ID NO: 27)
 
QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTSPPSPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGSGQPREPQV
YTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGSTGSEVQLQQSGPELVKPGASVKISCKTSGYTFTEYTM
 
HWVKQSHGKSLEWIGGISPNIGGTSYNQKFKGKATLTVDKSSSTAYMELRSLTSEDSAVYYCARRGGS
FDYWGQGTTLTVSSVEGGSGGSGGSGGSGGVDDIVMTQSPATLSVTPGDRVSLSCRASQSISDYLHWY
QQKSHESPRLLIKYASQSISGIPSRFSGSGSGSDFTLSINSVEPEDVGVYYCQNGHSFPLTFGAGTKL
ELK- (SEQ ID NO: 28)
F10C:

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC-  (SEQ ID NO: 29)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCGGGSSPAPELLGGPSVFLFPPKPKDTLMISRTPEVTCVVVDVSHE
DPEVKFNWYVDGVEVHNAKTKPREEQYSSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVLDSDGSFF
LYSKLTVDKSRWQQGNVFSCSVMHEALHNHYTQKSLSLSPGGGGGSGGGGSGGGGSGQPREPQVYTLP
PSREEMTKNQVSLTCLVKGFYPSDIAVEWESNGQPENNYKTTPPVLDSDGSFFLYSKLTVDKSRWQQG
NVFSCSVMHEALHNHYTQKSLSLSPGSTGSEVQLQQSGPELVKPGASVKISCKTSGYTFTEYTMHWVK
QSHGKSLEWIGGISPNIGGTSYNQKFKGKATLTVDKSSSTAYMELRSLTSEDSAVYYCARRGGSFDYW
GQGTTLTVSSVEGGSGGSGGSGGSGGVDDIVMTQSPATLSVTPGDRVSLSCRASQSISDYLHWYQQKS
HESPRLLIKYASQSISGIPSRFSGSGSGSDFTLSINSVEPEDVGVYYCQNGHSFPLTFGAGTKLELK-
(SEQ ID NO: 30)

### Format 11 (F11): CD19-F11-NKp46-3

The domain structure of F11 polypeptides is shown in **Figure 2C****.** The heterodimeric protein is similar to F10 except that the structures of the antigen binding domains are reversed. One of the two antigen binding domains has a Fab-like structure, and the other is a scFv. The heterodimer is made up of
(1) a first (central) polypeptide chain having domains arranged as follows (N- to C-termini):

   (Vκ - V_{H})^{anti-CD19} -CH2- CH3 - CH3 - VH^{anti-NKp46} - Cκ

   and
(2) a second polypeptide chain having domains arranged as follows (N- to C- termini):

   Vκ ^{anti-NKp46} - CH1.

Proteins were cloned, produced and purified as in Example 2-1. Bispecific proteins was purified from cell culture supernatant by affinity chromatography using prot-A beads and analysed and purified by SEC. The protein showed a good production yield of 2 mg/L and with a simple SEC profile. The amino acid sequences for the two chains of the F11 protein are shown in SEQ ID NOS 31 and 32.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKGGGGSGGGGSGGGGSQVQLQQSGAE

LVRPGSSVKISCKASGYAFSSYWMNWVKQRPGQGLEWIGQIWPGDGDTNYNGKFKGKATLTADESSST
AYMQLSSLASEDSAVYFCARRETTTVGRYYYAMDYWGQGTTVTVSSGGGSSAPELLGGPSVFLFPPKP
KDTLMISRTPEVTCVVVDVSHEDPEVKFNWYVDGVEVHNAKTKPREEQYSSTYRVVSVLTVLHQDWLN
GKEYKCKVSNKALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQKSLSLSPGGGGGS
GGGGSGGGGSGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGSTGSEVQLQQSGPELVKPGA
SVKISCKTSGYTFTEYTMHWVKQSHGKSLEWIGGISPNIGGTSYNQKFKGKATLTVDKSSSTAYMELR
SLTSEDSAVYYCARRGGSFDYWGQGTTLTVSSRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNR
GEC- (SEQ ID NO: 31)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH-(SEQ ID NO: 32)

### Format 12 (F12): CD19-F12-NKp46-3

The domain structure of the dimeric F12 polypeptides is shown in **Figure 2C****,** wherein the bonds between the CH1 and Cκ domains are disulfide bonds. The heterodimeric protein is similar to F11 but the CH1 and Cκ domains within the F(ab) structure are inversed. The heterodimer is made up of:
(1) a first (central) polypeptide chain having domains arranged as follows (N- to C-termini):

   (Vκ - V_{H})^{anti-CD19} -CH2- CH3 - CH3 - V_{H}^{anti-NKp46} - CH1

   and
(2) a second polypeptide chain having domains arranged as follows (N- to C- termini):

   VK ^{anti-NKp46} - CK.

Proteins were cloned, produced and purified as in Example 2-1. Bispecific proteins was purified from cell culture supernatant by affinity chromatography using prot-A beads and analysed and purified by SEC. The protein showed a good production yield of 2.8 mg/L and with a simple SEC profile. The amino acid sequences for the two chains of the F12 protein are shown in SEQ ID NOS: 33 and 34. 
DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKGGGGSGGGGSGGGGSQVQLQQSGAE
LVRPGSSVKISCKASGYAFSSYWMNWVKQRPGQGLEWIGQIWPGDGDTNYNGKFKGKATLTADESSST
AYMQLSSLASEDSAVYFCARRETTTVGRYYYAMDYWGQGTTVTVSSGGGSSAPELLGGPSVFLFPPKP
KDTLMISRTPEVTCVVVDVSHEDPEVKFNWYVDGVEVHNAKTKPREEQYSSTYRVVSVLTVLHQDWLN
GKEYKCKVSNKALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQKSLSLSPGGGGGS
GGGGSGGGGSGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGSTGSEVQLQQSGPELVKPGA
SVKISCKTSGYTFTEYTMHWVKQSHGKSLEWIGGISPNIGGTSYNQKFKGKATLTVDKSSSTAYMELR
 
SLTSEDSAVYYCARRGGSFDYWGQGTTLTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKRVEPKSCD
KTH- (SEQ ID NO: 33)
- Frag2

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC- (SEQ ID NO: 34)

### Format 17 (F17): CD19-F17-NKp46-3

The domain structure of the trimeric F17 polypeptides is shown in **Figure 2C****,** wherein the bonds between the CH1 and Cκ domains are disulfide bonds. The heterodimeric protein is similar to F9 but the V_{H} and Vκ domains, and the CH1 and Cκ, domains within the C-terminal F(ab) structure are each respectively inversed with their partner. The heterotrimer is made up of:
(1) a first (central) polypeptide chain having domains arranged as follows (N- to C-termini):

   V_{H}^{anti-CD19} - CH1 - CH2- CH3 - CH3 - Vκ^{anti-NKp46} - CH1

   and
(2) a second polypeptide chain having domains arranged as follows (N- to C- termini):

   V_{H} ^{anti-NKp46} - CK

   and
(3) a third polypeptide chain having domains arranged as follows (N- to C- termini):

   Vκ anti-CD19 - Cκ

Additionally, three variants of F17 proteins were produced: (a) a first where the cysteine residues in the hinge region were left intact (wild-type, referred to as F17A), (b) a second wherein the cysteine residues in the hinge region were replaced by serine residues (F10B, and (c) a third containing a linker sequence GGGSS which replaces residues DKTHTCPPCP in the hinge (F17C). Proteins were cloned, produced and purified as in Example 2-1.The amino acid sequences for the three chains of the F17B protein chains are shown in SEQ ID NOS: 35, 36 and 37.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC-(SEQ ID NO: 35)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTSPPSPAPELLGGPSVFLFPPKPKDTLMISRTPEVTCVVVD

VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGGGGGSGGGGSGGGGSGQPREPQV
YTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGSTGSDIVMTQSPATLSVTPGDRVSLSCRASQSISDYLH
WYQQKSHESPRLLIKYASQSISGIPSRFSGSGSGSDFTLSINSVEPEDVGVYYCQNGHSFPLTFGAGT
KLELKASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTH-(SEQ ID NO: 36)

EVQLQQSGPELVKPGASVKISCKTSGYTFTEYTMHWVKQSHGKSLEWIGGISPNIGGTSYNQKFKGKA
TLTVDKSSSTAYMELRSLTSEDSAVYYCARRGGSFDYWGQGTTLTVSSRTVAAPSVFIFPPSDEQLKS
GTASVVCLLNNFYPREAKVQWKVDNALQSGNSQESVTEQDSKDSTYSLSSTLTLSKADYEKHKVYACE
VTHQGLSSPVTKSFNRGEC-(SEQ ID NO: 37)

### Example 4: Bispecific NKp46 antibody formats with dimeric Fc domains

New protein constructions with dimeric Fc domains were developed that share many of the advantages of the monomeric Fc domain proteins of Example 3 but bind to FcRn with greater affinity. Different protein formats were produced that either had low or substantially lack of binding to FcyR (including CD16) or which had binding to FcyRs (including CD16), e.g. the binding affinity to human CD16 was within 1-log of that of wild-type human IgG1 antibodies, as assessed by SPR (e.g. see methods of Example 15. The different polypeptide formats were tested and compared to investigate the functionality of heterodimeric proteins comprising a central chain with a (V_{H}-(CH1/Cκ)-CH2-CH3-) unit or a (Vκ-(CH1 or Cκ)-CH2-CH3-) unit. One of both of the CH3 domains is fused, optionally via intervening amino acid sequences or domains, to a variable domain(s) (a single variable domain that associates with a variable domain on a separated polypeptide chain, a tandem variable domain (e.g., an scFv), or a single variable domain that is capable of binding antigen as a single variable domain).The two chains associate by CH1-Cκ dimerization to form disulfide linked dimers, or if associated with a third chain, to form trimers.

Different constructs were made for use in the preparation of a bispecific antibody using the variable domains DNA and amino acid sequences derived from the scFv specific for tumor antigen CD19 described in Example 2-1 and different variable regions from antibodies specific for NKp46 identified in Example 1. Proteins were cloned, produced and purified as in Example 2-1. Domains structures are shown in **Figures 2A-6D.**

### Format 5 (F5): CD19-F5-NKp46-3

The domain structure of the trimeric F5 polypeptide is shown in **Figure 2D****,** wherein the interchain bonds between hinge domains (indicated in the figures between CH1/Cκ and CH2 domains on a chain) and interchain bonds between the CH1 and Cκ domains are interchain disulfide bonds. The heterotrimer is made up of:
(1) a first (central) polypeptide chain having domains arranged as follows (N- to C-termini):

   V_{H}^{anti-CD19} - CH1 - CH2 - CH3 - V_{H}^{anti-NKp46} - Cκ

   and
(2) a second polypeptide chain having domains arranged as follows (N- to C- termini):

   Vκ^{anti-CD19} - CK - CH2 - CH3

   and
(3) a third polypeptide chain having domains arranged as follows (N- to C- termini):

   Vκ ^{anti-NKp46} - CH1.

Proteins were cloned, produced and purified as in Example 2-1. Bispecific proteins was purified from cell culture supernatant by affinity chromatography using prot-A beads and analysed and purified by SEC. The protein showed a high production yield of 37 mg/L and with a simple SEC profile. The amino acid sequences of the three chains are shown in SEQ ID NOS: 38, 39 and 40.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGECDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK- (SEQ ID NO: 38)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGSTGSEVQLQQSGPELVKPGASVK
ISCKTSGYTFTEYTMHWVKQSHGKSLEWIGGISPNIGGTSYNQKFKGKATLTVDKSSSTAYMELRSLT
SEDSAVYYCARRGGSFDYWGQGTTLTVSSRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC
- (SEQ ID NO: 39)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH- (SEQ ID NO: 40)

### Format 6 (F6): CD19-F6-NKp46-3

The domain structure of heterotrimeric F6 polypeptides is shown in Figure 2D. The F6 protein is the same as F5, but contains a N297S substitution to avoid N-linked glycosylation. Proteins were cloned, produced and purified as in Example 2-1. Bispecific proteins were purified from cell culture supernatant by affinity chromatography using prot-A beads and analyzed and purified by SEC. The protein showed a high production yield of 12 mg/L and the purified proteins exhibited a simple SEC profile. The amino acid sequences of the three chains of the F6 protein are shown in SEQ ID NOS: 41, 42 and 43.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGECDKTHTCPPCPAPELLGGPSVFLFPPKPKDTLMISRTPEVTCVVVDVSHEDPEVK
FNWYVDGVEVHNAKTKPREEQYSSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK-(SEQ ID NO: 41)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGSTGSEVQLQQSGPELVKPGASVK
ISCKTSGYTFTEYTMHWVKQSHGKSLEWIGGISPNIGGTSYNQKFKGKATLTVDKSSSTAYMELRSLT
SEDSAVYYCARRGGSFDYWGQGTTLTVSSRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC
– (SEQ ID NO: 42)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH-(SEQ ID NO: 43)

### Format 7 (F7) : CD19-F7-NKp46-3

The domain structure of heterotrimeric F7 polypeptides is shown in **Figure 2D****.** The F7 protein is the same as F6, except for cysteine to serine substitutions in the CH1 and Cκ domains that are linked at their C-termini to Fc domains, in order to prevent formation of a minor population of dimeric species of the central chain with the Vκ ^{anti-NKp46} - CH1 chain. Proteins were cloned, produced and purified as in Example 2-1. Bispecific proteins were purified from the cell culture supernatant by affinity chromatography using prot-A beads and analyzed and purified by SEC. The protein showed a high production yield of 11 mg/L and the purified proteins exhibited a simple SEC profile. The amino acid sequences of the three chains of the 76 protein are shown in SEQ ID NOS: 44, 45 and 46.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGESDKTHTCPPCPAPELLGGPSVFLFPPKPKDTLMISRTPEVTCVVVDVSHEDPEVK

FNWYVDGVEVHNAKTKPREEQYSSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK-(SEQ ID NO: 44)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSSDKTHTCPPCPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGSTGSEVQLQQSGPELVKPGASVK
ISCKTSGYTFTEYTMHWVKQSHGKSLEWIGGISPNIGGTSYNQKFKGKATLTVDKSSSTAYMELRSLT
SEDSAVYYCARRGGSFDYWGQGTTLTVSSRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC
- (SEQ ID NO: 45)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH- (SEQ ID NO: 46)

### Format 13 (F13): CD19-F13-NKp46-3

The domain structure of the dimeric F13 polypeptide is shown in **Figure 2D****,** wherein the interchain bonds between hinge domains (indicated between CH1/Cκ and CH2 domains on a chain) and interchain bonds between the CH1 and Cκ domains are interchain disulfide bonds. The heterodimer is made up of:
(1) a first (central) polypeptide chain having domains arranged as follows (N- to C-termini):

   V_{H}^{anti-CD19} - CH1 - CH2 - CH3 - (V_{H}-Vκ)^{anti-NKp46}

   and
(2) a second polypeptide chain having domains arranged as follows (N- to C- termini):

   V_{K}^{anti-CD19} - Cκ - CH2 - CH3.

The (V_{H}-Vκ) unit was made up of a V_{H} domain, a linker and a Vκ unit (scFv).

Proteins were cloned, produced and purified as in Example 2-1. Bispecific proteins were purified from the cell culture supernatant by affinity chromatography using prot-A beads and analyzed and purified by SEC. The protein showed a high production yield of 6.4 mg/L and the purified proteins exhibited a simple SEC profile. The amino acid sequences of the two chains of the F13 protein are shown in SEQ ID NOS: 47 and 48.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGECDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQ

PREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK- (SEQ ID NO: 47)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGSTGSEVQLQQSGPELVKPGASVK
ISCKTSGYTFTEYTMHWVKQSHGKSLEWIGGISPNIGGTSYNQKFKGKATLTVDKSSSTAYMELRSLT
SEDSAVYYCARRGGSFDYWGQGTTLTVSSVEGGSGGSGGSGGSGGVDDIVMTQSPATLSVTPGDRVSL
SCRASQSISDYLHWYQQKSHESPRLLIKYASQSISGIPSRFSGSGSGSDFTLSINSVEPEDVGVYYCQ
NGHSFPLTFGAGTKLELK- (SEQ ID NO: 48)

### Format 14 (F14): CD19-F14-NKp46-3

The domain structure of the dimeric F14 polypeptide is shown in **Figure 2E****.** The F14 polypeptide is a dimeric polypeptide which shares the structure of the F13 format, but instead of a wild-type Fc domain (CH2-CH3), the F14 bispecific format has CH2 domain mutations N297S to abolish N-linked glycosylation. Proteins were cloned, produced and purified as in Example 2-1. Bispecific proteins were purified from cell culture supernatant by affinity chromatography using prot-A beads and analyzed and purified by SEC. The protein showed a high production yield of 2.4 mg/L and the purified proteins exhibited a simple SEC profile.The amino acid sequences of the two chains of the F14 protein are shown in SEQ ID NOS: 49 and 50.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGECDKTHTCPPCPAPELLGGPSVFLFPPKPKDTLMISRTPEVTCVVVDVSHEDPEVK
FNWYVDGVEVHNAKTKPREEQYSSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK-(SEQ ID NO: 49)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGSTGSEVQLQQSGPELVKPGASVK
ISCKTSGYTFTEYTMHWVKQSHGKSLEWIGGISPNIGGTSYNQKFKGKATLTVDKSSSTAYMELRSLT
SEDSAVYYCARRGGSFDYWGQGTTLTVSSVEGGSGGSGGSGGSGGVDDIVMTQSPATLSVTPGDRVSL
SCRASQSISDYLHWYQQKSHESPRLLIKYASQSISGIPSRFSGSGSGSDFTLSINSVEPEDVGVYYCQ
NGHSFPLTFGAGTKLELK-(SEQ ID NO: 50)

### Format 15 (F15): CD19-F15-NKp46-3

The domain structure of the trimeric F15 polypeptides is shown in **Figure 2E****.** The F15 polypeptide is a dimeric polypeptide which shares the structure of the F6 format, but differs by inversion of the N-terminal V_{H}-CH1 and Vκ-Cκ units between the central and second chains. Proteins were cloned, produced and purified as in Example 2-1. Bispecific proteins were purified from the cell culture supernatant by affinity chromatography using prot-A beads and analyzed and purified by SEC. The protein showed a good production yield of 0.9 mg/L and the purified proteins possessed a simple SEC profile. The amino acid sequences of the three chains of the F15 protein are shown in SEQ ID NOS: 51, 52 and 53.

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK-(SEQ ID NO: 51)

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGECDKTHTCPPCPAPELLGGPSVFLFPPKPKDTLMISRTPEVTCVVVDVSHEDPEVK
FNWYVDGVEVHNAKTKPREEQSSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYSKLT
VDKSRWQQGNVFSCSVMHEALHNHYTQKSLSLSPGSTGSEVQLQQSGPELVKPGASVKISCKTSGYTF
TEYTMHWVKQSHGKSLEWIGGISPNIGGTSYNQKFKGKATLTVDKSSSTAYMELRSLTSEDSAVYYCA
RRGGSFDYWGQGTTLTVSSRTVAAPSVFIFPPSDEQLKSGTASVVCLLNNFYPREAKVQWKVDNALQS
GNSQESVTEQDSKDSTYSLSSTLTLSKADYEKHKVYACEVTHQGLSSPVTKSFNRGEC- (SEQ ID
NO: 52)

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTH-(SEQ ID NO: 53)

### Format 16 (F16): CD19-F16-NKp46-3

The domain structure of the trimeric F16 polypeptide is shown in **Figure 2E****.** The F16 polypeptide is a dimeric polypeptide which shares the structure of the F6 format, but differs by inversion of the C-terminal V_{H}-CK and Vκ-CH1 units between the central and second chains. Proteins were cloned, produced and purified as in Example 2-1. The amino acid sequences of the three chains of the F16 protein are shown in SEQ ID NOS: 54, 55 and 56.

DIQLTQSPASLAVSLGQRATISCKASQSVDYDGDSYLNWYQQIPGQPPKLLIYDASNLVSGIPPRFSG
SGSGTDFTLNIHPVEKVDAATYHCQQSTEDPWTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGECDKTHTCPPCPAPELLGGPSVFLFPPKPKDTLMISRTPEVTCVVVDVSHEDPEVK

FNWYVDGVEVHNAKTKPREEQYSSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK- (SEQ ID NO: 54)

QVQLQQSGAELVRPGSSVKISCKASGYAFSSYWMNWVKQRPGQGLEWIGQIWPGDGDTNYNGKFKGKA
TLTADESSSTAYMQLSSLASEDSAVYFCARRETTTVGRYYYAMDYWGQGTTVTVSSASTKGPSVFPLA
PSSKSTSGGTAALGCLVKDYFPEPVTVSWNSGALTSGVHTFPAVLQSSGLYSLSSVVTVPSSSLGTQT
YICNVNHKPSNTKVDKRVEPKSCDKTHTCPPCPAPELLGGPSVFLFPPKPKDTLMISRTPEVTCVVVD
VSHEDPEVKFNWYVDGVEVHNAKTKPREEQYSSTYRVVSVLTVLHQDWLNGKEYKCKVSNKALPAPIE
KTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGSTGSDIVMTQSPATLSVTPGDRV
SLSCRASQSISDYLHWYQQKSHESPRLLIKYASQSISGIPSRFSGSGSGSDFTLSINSVEPEDVGVYY
CQNGHSFPLTFGAGTKLELKASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCDKTH-(SEQ ID
NO: 55)

EVQLQQSGPELVKPGASVKISCKTSGYTFTEYTMHWVKQSHGKSLEWIGGISPNIGGTSYNQKFKGKA
TLTVDKSSSTAYMELRSLTSEDSAVYYCARRGGSFDYWGQGTTLTVSSRTVAAPSVFIFPPSDEQLKS
GTASVVCLLNNFYPREAKVQWKVDNALQSGNSQESVTEQDSKDSTYSLSSTLTLSKADYEKHKVYACE
VTHQGLSSPVTKSFNRGEC-(SEQ ID NO: 56)

### Format T5 (T5)

The domain structure of a trimeric T5 polypeptide is shown in Figure 2F. The T5 polypeptide is a trimeric polypeptide which shares the structure of the F5 format, but differs by fusion of an scFv unit at the C-terminus of the third chain (the chain lacking the Fc domain). This protein will therefore have two antigen binding domains for antigens of interest, and one for NKp46, and will bind CD16 via its Fc domain. Proteins were cloned, produced and purified as in Example 2-1. Two different T5 proteins were produced, as follows.

A first T5 protein had one antigen binding domain that binds human CD137, one antigen binding domain that binds human NKp46, and one antigen binding domain that binds CD19. The amino acid sequences of the three chains of the T5 CD137-T5-NKp46-CD19 protein are shown in below (anti-CD137 in bold and underlined, anti-CD19 underlined, anti-NKp46 in italics).

### CD137-T5-NKp46-CD19

### Polypeptide 1:

Residues 1-121 are anti-CD137 binding domain; 122-454 and 571-677 are T5 sequences; 455-570 are anti-NKp46 binding domain

QVQLQQWGAGLLKPSETLSLTCAVYGGSFSGYYWSWIRQSPEKGLEWIGEINHGGYVTYNPSLESRVT
ISVDTSKNQFSLKLSSVTAADTAVYYCARDYGPGNYDWYFDLWGRGTLVTVSSASTKGPSVFPLAPSS
KSTSGGTAALGCLVKDYFPEPVTVSWNSGALTSGVHTFPAVLQSSGLYSLSSVVTVPSSSLGTQTYIC
NVNHKPSNTKVDKRVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTPPVLDSDGSF

FLYSKLTVDKSRWQQGNVFSCSVMHEALHNHYTQKSLSLSPGSTGSEVQLQQSGPELVKPGASVKISC
KTSGYTFTEYTMHWVKQSHGKSLEWIGGISPNIGGTSYNQKFKGKATLTVDKSSSTAYMELRSLTSED
SAVYYCARRGGSFDYWGQGTTLTVSSRTVAAPSVFIFPPSDEQLKSGTASVVCLLNNFYPREAKVQWK
VDNALQSGNSQESVTEQDSKDSTYSLSSTLTLSKADYEKHKVYACEVTHQGLSSPVTKSFNRGEC-
(SEQ ID NO: 57)

### Polypeptide 2:

Residues 1-109 (anti-CD137); 110-443 are T5 sequences

EIVLTQSPATLSLSPGERATLSCRASQSVSSYLAWYQQKPGQAPRLLIYDASNRATGIPARFSGSGSG
TDFTLTISSLEPEDFAVYYCQQRSNWPPALTFCGGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEVTHQGLS
SPVTKSFNRGECDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGK-(SEQ ID NO: 58)

### Polypeptide 3:

Residues 1-107 are anti-NKp46 binding domain; 108-219 are T5 sequences; 220-469 are anti-CD19 binding domain

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTHGGSSSDIQLTQSPASLAVSLGQRATISCKASQSVDYDGDSYLNWYQQIPGQPPKLLIY
DASNLVSGIPPRFSGSGSGTDFTLNIHPVEKVDAATYHCQQSTEDPWTFGGGTKLEIKGGGGSGGGGS
GGGGSQVQLQQSGAELVRPGSSVKISCKASGYAFSSYWMNWVKQRPGQGLEWIGQIWPGDGDTNYNGK
FKGKATLTADESSSTAYMQLSSLASEDSAVYFCARRETTTVGRYYYAMDYWGQGTTVTVSS-
(SEQ ID NO: 59)

A second T5 protein had two antigen binding domains that bind human CD20, originating from different antibodies (and binding to different epitopes on CD20). The first anti-CD20 ABD contained the VH and VL of the parent antibody GA101 (GAZYVA@, Gazyvaro^{®}, obinutuzumab, Roche Pharmaceuticals). The second anti-CD20 ABD contained the VH and VL of the parent antibody rituximab (Rituxan^{®}, Mabthera^{®}, Roche Pharmaceuticals). The third antigen binding domain binds human NKp46. The amino acid sequences of the three chains of the T5 protein are shown in below (Rituximab sequences in bold and underlined, anti-GA101 sequences underlined, anti-NKp46 in italics).

### GA101-T5-Ritux-NKp46

### Polypeptide 1:

QVQLVQSGAEVKKPGSSVKVSCKASGYAFSYSWINWVRQAPGQGLEWMGRIFPGDGDTDYNGKFKGRV
TITADKSTSTAYMELSSLRSEDTAVYYCARNVFDGYWLVYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNV
NHKPSNTKVDKRVEPKSCDKTHTCPPCPAPELLGGPSVFLFPPKPKDTLMISRTPEVTCVVVDVSHED

PEVKFNWYVDGVEVHNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGSTGSQVQLQQPGAELVKPGASVKMSCKA
SGYTFTSYNMHWVKQTPGRGLEWIGAIYPGNGDTSYNQKFKGKATLTADKSSSTAYMQLSSLTSEDSA
VYYCARSTYYGGDWYFNVWGAGTTVTVSARTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC
-(SEQ ID NO: 60)

### Polypeptide 2:

DIVMTQTPLSLPVTPGEPASISCRSSKSLLHSNGITYLYWYLQKPGQSPQLLIYQMSNLVSGVPDRFS
GSGSGTDFTLKISRVEAEDVGVYYCAQNLELPYTFGGGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEVTHQ
GLSSPVTKSFNRGECDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPGK- (SEQ ID NO: 61)

### Polypeptide 3:

QIVLSQSPAILSASPGEKVTMTCRASSSVSYIHWFQQKPGSSPKPWIYATSNLASGVPVRFSGSGSGT
SYSLTISRVEAEDAATYYCQQWTSNPPTFGGGTKLEIKASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKRV
EPKSCDKTHGGSSSEVQLQQSGPELVKPGASVKISCKTSGYTFTEYTMHWVKQSHGKSLEWIGGISPN
IGGTSYNQKFKGKATLTVDKSSSTAYMELRSLTSEDSAVYYCARRGGSFDYWGQGTTLTVSSVEGGSG
GSGGSGGSGGVDDIVMTQSPATLSVTPGDRVSLSCRASQSISDYLHWYQQKSHESPRLLIKYASQSIS
GIPSRFSGSGSGSDFTLSINSVEPEDVGVYYCQNGHSFPLTFGAGTKLELK- (SEQ ID NO: 62)

### Format T6 (T6)

The domain structure of the trimeric T6 polypeptide is shown in Figure 2F. The T6 polypeptide is a trimeric polypeptide which shares the structure of the F6 format, but differs by fusion of an scFv unit at the C-terminus of the third chain (the chain lacking the Fc domain). This protein has two antigen binding domains for antigens of interest, and one for NKp46, but does not bind CD16 via its Fc domain due to the N297 substitution. Proteins were cloned, produced and purified as in Example 2-1. Two different T6 proteins were produced. A first T6 protein had one antigen binding domain that binds human CD137, one antigen binding domain that binds human NKp46, and one antigen binding domain that binds CD19. The second T6 protein had two antigen binding domains that bind human CD20 (CD137-T6-NKp46-CD19). The first anti-CD20 ABD contained the VH and VL of the parent antibody GA101 and the second anti-CD20 ABD contained the VH and VL of the parent antibody rituximab. The amino acid sequences of the three chains of the T6 proteins are shown in below.

### CD137-T6-NKp46-CD19

### Polypeptide 2:

EIVLTQSPATLSLSPGERATLSCRASQSVSSYLAWYQQKPGQAPRLLIYDASNRATGIPARFSGSGSG
TDFTLTISSLEPEDFAVYYCQQRSNWPPALTFCGGTKVEIKRTVAAPSVFIFPPSDEQLKSGTASVVC

LLNNFYPREAKVQWKVDNALQSGNSQESVTEQDSKDSTYSLSSTLTLSKADYEKHKVYACEVTHQGLS
SPVTKSFNRGECDKTHTCPPCPAPELLGGPSVFLFPPKPKDTLMISRTPEVTCVVVDVSHEDPEVKFN
WYVDGVEVHNAKTKPREEQYSSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGK- (SEQ ID NO: 63)

### Polypeptide 1:

QVQLQQWGAGLLKPSETLSLTCAVYGGSFSGYYWSWIRQSPEKGLEWIGEINHGGYVTYNPSLESRVT
ISVDTSKNQFSLKLSSVTAADTAVYYCARDYGPGNYDWYFDLWGRGTLVTVSSASTKGPSVFPLAPSS
KSTSGGTAALGCLVKDYFPEPVTVSWNSGALTSGVHTFPAVLQSSGLYSLSSVVTVPSSSLGTQTYIC
NVNHKPSNTKVDKRVEPKSCDKTHTCPPCPAPELLGGPSVFLFPPKPKDTLMISRTPEVTCVVVDVSH
EDPEVKFNWYVDGVEVHNAKTKPREEQYSSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGSTGSEVQLQQSGPELVKPGASVKISC
KTSGYTFTEYTMHWVKQSHGKSLEWIGGISPNIGGTSYNQKFKGKATLTVDKSSSTAYMELRSLTSED
SAVYYCARRGGSFDYWGQGTTLTVSSRTVAAPSVFIFPPSDEQLKSGTASVVCLLNNFYPREAKVQWK
VDNALQSGNSQESVTEQDSKDSTYSLSSTLTLSKADYEKHKVYACEVTHQGLSSPVTKSFNRGEC-
(SEQ ID NO: 64)

### Polypeptide 3:

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTHGGSSSDIQLTQSPASLAVSLGQRATISCKASQSVDYDGDSYLNWYQQIPGQPPKLLIY
DASNLVSGIPPRFSGSGSGTDFTLNIHPVEKVDAATYHCQQSTEDPWTFGGGTKLEIKGGGGSGGGGS
GGGGSQVQLQQSGAELVRPGSSVKISCKASGYAFSSYWMNWVKQRPGQGLEWIGQIWPGDGDTNYNGK
FKGKATLTADESSSTAYMQLSSLASEDSAVYFCARRETTTVGRYYYAMDYWGQGTTVTVSS-
(SEQ ID NO: 65)

### GA101-T6-Ritux-NKp46

### Polypeptide 2:

DIVMTQTPLSLPVTPGEPASISCRSSKSLLHSNGITYLYWYLQKPGQSPQLLIYQMSNLVSGVPDRFS
GSGSGTDFTLKISRVEAEDVGVYYCAQNLELPYTFGGGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEVTHQ
GLSSPVTKSFNRGECDKTHTCPPCPAPELLGGPSVFLFPPKPKDTLMISRTPEVTCVVVDVSHEDPEV
KFNWYVDGVEVHNAKTKPREEQYSSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPGK-
(SEQ ID NO: 66)

### Polypeptide 1:

QVQLVQSGAEVKKPGSSVKVSCKASGYAFSYSWINWVRQAPGQGLEWMGRIFPGDGDTDYNGKFKGRV
TITADKSTSTAYMELSSLRSEDTAVYYCARNVFDGYWLVYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNV
NHKPSNTKVDKRVEPKSCDKTHTCPPCPAPELLGGPSVFLFPPKPKDTLMISRTPEVTCVVVDVSHED
PEVKFNWYVDGVEVHNAKTKPREEQYSSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGSTGSQVQLQQPGAELVKPGASVKMSCKA
SGYTFTSYNMHWVKQTPGRGLEWIGAIYPGNGDTSYNQKFKGKATLTADKSSSTAYMQLSSLTSEDSA
VYYCARSTYYGGDWYFNVWGAGTTVTVSARTVAAPSVFIFPPSDEQLKSGTASVVCLLNNFYPREAKV

QWKVDNALQSGNSQESVTEQDSKDSTYSLSSTLTLSKADYEKHKVYACEVTHQGLSSPVTKSFNRGEC
(SEQ ID NO: 67)

### Polypeptide 3:

QIVLSQSPAILSASPGEKVTMTCRASSSVSYIHWFQQKPGSSPKPWIYATSNLASGVPVRFSGSGSGT
SYSLTISRVEAEDAATYYCQQWTSNPPTFGGGTKLEIKASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKRV
EPKSCDKTHGGSSSEVQLQQSGPELVKPGASVKISCKTSGYTFTEYTMHWVKQSHGKSLEWIGGISPN
IGGTSYNQKFKGKATLTVDKSSSTAYMELRSLTSEDSAVYYCARRGGSFDYWGQGTTLTVSSVEGGSG
GSGGSGGSGGVDDIVMTQSPATLSVTPGDRVSLSCRASQSISDYLHWYQQKSHESPRLLIKYASQSIS
GIPSRFSGSGSGSDFTLSINSVEPEDVGVYYCQNGHSFPLTFGAGTKLELK- (SEQ ID NO: 68)

### Format T9B (T9B)

The domain structure of the trimeric T9B polypeptide is shown in Figure 2F. The T9B polypeptide is a trimeric polypeptide which shares the structure of the F9 format (F9B variant), but differs by fusion of an scFv unit at the C-terminus of the free CH1 domain (on the third chain). This protein will therefore have two antigen binding domains for antigen of interest, and one for NKp46, but will not bind CD16 via its Fc domain due to the monomeric Fc domain and/or the N297 substitution. Proteins were cloned, produced and purified as in Example 2-1. Two different T9B proteins were produced. A first T9B protein had one antigen binding domain that binds human CD137, one antigen binding domain that binds human NKp46, and one antigen binding domain that binds CD19 (CD137-T9B-NKp46-CD19). The second T9B protein had two antigen binding domains that bind human CD20. The first anti-CD20 ABD contained the VH and VL of the parent antibody GA101 and the second anti-CD20 ABD contained the VH and VL of the parent antibody rituximab. The amino acid sequences of the three chains of the T9B proteins are shown in below.

### CD137-T9B-NKp46-CD19

### Polypeptide 2:

EIVLTQSPATLSLSPGERATLSCRASQSVSSYLAWYQQKPGQAPRLLIYDASNRATGIPARFSGSGSG
TDFTLTISSLEPEDFAVYYCQQRSNWPPALTFCGGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEVTHQGLS
SPVTKSFNRGEC- (SEQ ID NO: 69)

### Polypeptide 1:

QVQLQQWGAGLLKPSETLSLTCAVYGGSFSGYYWSWIRQSPEKGLEWIGEINHGGYVTYNPSLESRVT
ISVDTSKNQFSLKLSSVTAADTAVYYCARDYGPGNYDWYFDLWGRGTLVTVSSASTKGPSVFPLAPSS
KSTSGGTAALGCLVKDYFPEPVTVSWNSGALTSGVHTFPAVLQSSGLYSLSSVVTVPSSSLGTQTYIC
NVNHKPSNTKVDKRVEPKSCDKTHTSPPSPAPELLGGPSVFLFPPKPKDTLMISRTPEVTCVVVDVSH
EDPEVKFNWYVDGVEVHNAKTKPREEQYSSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGGGGGSGGGGSGGGGSGQPREPQVYTL
PPSREEMTKNQVSLTCLVKGFYPSDIAVEWESNGQPENNYKTTPPVLDSDGSFFLYSKLTVDKSRWQQ
GNVFSCSVMHEALHNHYTQKSLSLSPGSTGSEVQLQQSGPELVKPGASVKISCKTSGYTFTEYTMHWV

KQSHGKSLEWIGGISPNIGGTSYNQKFKGKATLTVDKSSSTAYMELRSLTSEDSAVYYCARRGGSFDY
WGQGTTLTVSSRTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC- (SEQ ID NO: 70)

### Polypeptide 3:

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTHGGSSSDIQLTQSPASLAVSLGQRATISCKASQSVDYDGDSYLNWYQQIPGQPPKLLIY
DASNLVSGIPPRFSGSGSGTDFTLNIHPVEKVDAATYHCQQSTEDPWTFGGGTKLEIKGGGGSGGGGS
GGGGSQVQLQQSGAELVRPGSSVKISCKASGYAFSSYWMNWVKQRPGQGLEWIGQIWPGDGDTNYNGK
FKGKATLTADESSSTAYMQLSSLASEDSAVYFCARRETTTVGRYYYAMDYWGQGTTVTVSS-
(SEQ ID NO: 71)

### GA 101-T9B-Ritux-NKp46

### Polypeptide 2:

DIVMTQTPLSLPVTPGEPASISCRSSKSLLHSNGITYLYWYLQKPGQSPQLLIYQMSNLVSGVPDRFS
GSGSGTDFTLKISRVEAEDVGVYYCAQNLELPYTFGGGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEVTHQ
GLSSPVTKSFNRGEC- (SEQ ID NO: 72)

### Polypeptide 1:

QVQLVQSGAEVKKPGSSVKVSCKASGYAFSYSWINWVRQAPGQGLEWMGRIFPGDGDTDYNGKFKGRV
TITADKSTSTAYMELSSLRSEDTAVYYCARNVFDGYWLVYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNV
NHKPSNTKVDKRVEPKSCDKTHTSPPSPAPELLGGPSVFLFPPKPKDTLMISRTPEVTCVVVDVSHED
PEVKFNWYVDGVEVHNAKTKPREEQYSSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGGGGGSGGGGSGGGGSGQPREPQVYTLPP
SREEMTKNQVSLTCLVKGFYPSDIAVEWESNGQPENNYKTTPPVLDSDGSFFLYSKLTVDKSRWQQGN
VFSCSVMHEALHNHYTQKSLSLSPGSTGSQVQLQQPGAELVKPGASVKMSCKASGYTFTSYNMHWVKQ
TPGRGLEWIGAIYPGNGDTSYNQKFKGKATLTADKSSSTAYMQLSSLTSEDSAVYYCARSTYYGGDWY
FNVWGAGTTVTVSARTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC- (SEQ ID NO: 73)

### Polypeptide 3:

QIVLSQSPAILSASPGEKVTMTCRASSSVSYIHWFQQKPGSSPKPWIYATSNLASGVPVRFSGSGSGT
SYSLTISRVEAEDAATYYCQQWTSNPPTFGGGTKLEIKASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKRV
EPKSCDKTHGGSSSEVQLQQSGPELVKPGASVKISCKTSGYTFTEYTMHWVKQSHGKSLEWIGGISPN
IGGTSYNQKFKGKATLTVDKSSSTAYMELRSLTSEDSAVYYCARRGGSFDYWGQGTTLTVSSVEGGSG
GSGGSGGSGGVDDIVMTQSPATLSVTPGDRVSLSCRASQSISDYLHWYQQKSHESPRLLIKYASQSIS
GIPSRFSGSGSGSDFTLSINSVEPEDVGVYYCQNGHSFPLTFGAGTKLELK- (SEQ ID NO: 74)

### Format T11 (T1): CD19-T11-NKp46-3

The domain structure of the dimeric T11 polypeptide is shown in Figure 2F. The T11 polypeptide is a trimeric polypeptide which shares the structure of the F11 format, but differs by fusion of an scFv unit at the C-terminus of the free CH1 domain. This protein therefore has antigen binding domains for antigen of interest, and one for NKp46, but does not bind CD16 via its Fc domain due to the monomeric Fc domain and/or the N297 substitution. Proteins were cloned, produced and purified as in Example 2-1. Two different T11B proteins were produced. A first T11B protein had one antigen binding domain that binds human CD137, one antigen binding domain that binds human NKp46, and one antigen binding domain that binds CD19 (CD137-T9B-NKp46-CD19). The second T9B protein had two antigen binding domains that bind human CD20. The first anti-CD20 ABD contained the VH and VL of the parent antibody GA101 and the second anti-CD20 ABD contained the VH and VL of the parent antibody rituximab. The amino acid sequences of the three chains of the T11 protein are shown in below.

### CD137-T11-NKp46-CD19

### Polypeptide 1:

EIVLTQSPATLSLSPGERATLSCRASQSVSSYLAWYQQKPGQAPRLLIYDASNRATGIPARFSGSGSG
TDFTLTISSLEPEDFAVYYCQQRSNWPPALTFCGGTKVEIKGGGGSGGGGSGGGGSQVQLQQWGAGLL
KPSETLSLTCAVYGGSFSGYYWSWIRQSPEKGLEWIGEINHGGYVTYNPSLESRVTISVDTSKNQFSL
KLSSVTAADTAVYYCARDYGPGNYDWYFDLWGRGTLVTVSSGGGSSAPELLGGPSVFLFPPKPKDTLM
ISRTPEVTCVVVDVSHEDPEVKFNWYVDGVEVHNAKTKPREEQYSSTYRVVSVLTVLHQDWLNGKEYK
CKVSNKALPAPIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGGGGGSGGGGS
GGGGSGQPREPQVYTLPPSREEMTKNQVSLTCLVKGFYPSDIAVEWESNGQPENNYKTTPPVLDSDGS
FFLYSKLTVDKSRWQQGNVFSCSVMHEALHNHYTQKSLSLSPGSTGSEVQLQQSGPELVKPGASVKIS
CKTSGYTFTEYTMHWVKQSHGKSLEWIGGISPNIGGTSYNQKFKGKATLTVDKSSSTAYMELRSLTSE
DSAVYYCARRGGSFDYWGQGTTLTVSSRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSPVTKSFNRGEC-
(SEQ ID NO: 75)

### Polypeptide 2:

DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKR
VEPKSCDKTHGGSSSDIQLTQSPASLAVSLGQRATISCKASQSVDYDGDSYLNWYQQIPGQPPKLLIY
DASNLVSGIPPRFSGSGSGTDFTLNIHPVEKVDAATYHCQQSTEDPWTFGGGTKLEIKGGGGSGGGGS
GGGGSQVQLQQSGAELVRPGSSVKISCKASGYAFSSYWMNWVKQRPGQGLEWIGQIWPGDGDTNYNGK
FKGKATLTADESSSTAYMQLSSLASEDSAVYFCARRETTTVGRYYYAMDYWGQGTTVTVSS-
(SEQ ID NO: 76)

### GA101-T11-Ritux-NKp46

### Polypeptide 1:

DIVMTQTPLSLPVTPGEPASISCRSSKSLLHSNGITYLYWYLQKPGQSPQLLIYQMSNLVSGVPDRFS
GSGSGTDFTLKISRVEAEDVGVYYCAQNLELPYTFGGGTKVEIKGGGGSGGGGSGGGGSQVQLVQSGA
EVKKPGSSVKVSCKASGYAFSYSWINWVRQAPGQGLEWMGRIFPGDGDTDYNGKFKGRVTITADKSTS
TAYMELSSLRSEDTAVYYCARNVFDGYWLVYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKV
DKRVEPKSCDKTHTSPPSPAPELLGGPSVFLFPPKPKDTLMISRTPEVTCVVVDVSHEDPEVKFNWYV
DGVEVHNAKTKPREEQYSSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFLYSKLTVDKS

RWQQGNVFSCSVMHEALHNHYTQKSLSLSPGGGGGSGGGGSGGGGSGQPREPQVYTLPPSREEMTKNQ
VSLTCLVKGFYPSDIAVEWESNGQPENNYKTTPPVLDSDGSFFLYSKLTVDKSRWQQGNVFSCSVMHE
ALHNHYTQKSLSLSPGSTGSQVQLQQPGAELVKPGASVKMSCKASGYTFTSYNMHWVKQTPGRGLEWI
GAIYPGNGDTSYNQKFKGKATLTADKSSSTAYMQLSSLTSEDSAVYYCARSTYYGGDWYFNVWGAGTT
VTVSARTVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC- (SEQ ID NO: 77)

### Polypeptide 2:

QIVLSQSPAILSASPGEKVTMTCRASSSVSYIHWFQQKPGSSPKPWIYATSNLASGVPVRFSGSGSGT
SYSLTISRVEAEDAATYYCQQWTSNPPTFGGGTKLEIKASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKRV
EPKSCDKTHGGSSSEVQLQQSGPELVKPGASVKISCKTSGYTFTEYTMHWVKQSHGKSLEWIGGISPN
IGGTSYNQKFKGKATLTVDKSSSTAYMELRSLTSEDSAVYYCARRGGSFDYWGQGTTLTVSSVEGGSG
GSGGSGGSGGVDDIVMTQSPATLSVTPGDRVSLSCRASQSISDYLHWYQQKSHESPRLLIKYASQSIS
GIPSRFSGSGSGSDFTLSINSVEPEDVGVYYCQNGHSFPLTFGAGTKLELK- (SEQ ID NO: 78)

### Example 5: NKp46 binding affinity by bispecific proteins detected by Surface Plasmon Resonance (SPR)

### Biacore T100 general procedure and reagents

SPR measurements were performed on a Biacore T100 apparatus (Biacore GE Healthcare) at 25°C. In all Biacore experiments HBS-EP+ (Biacore GE Healthcare) and NaOH 10mM served as running buffer and regeneration buffer respectively. Sensorgrams were analyzed with Biacore T100 Evaluation software. Protein-A was purchased from (GE Healthcare). Human NKp46 recombinant proteins were cloned, produced and purified at Innate Pharma.

### Immobilization of Protein-A

Protein-A proteins were immobilized covalently to carboxyl groups in the dextran layer on a Sensor Chip CM5. The chip surface was activated with EDC/NHS (N-ethyl-N'-(3-dimethylaminopropyl) carbodiimidehydrochloride and N-hydroxysuccinimide (Biacore GE Healthcare)). Protein-A was diluted to 10 µg/ml in coupling buffer (10 mM acetate, pH 5.6) and injected until the appropriate immobilization level was reached (i.e. 2000 RU). Deactivation of the remaining activated groups was performed using 100 mM ethanolamine pH 8 (Biacore GE Healthcare).

### Binding study

Antibodies were tested as different formats F5, F6, F9, F10, F11, F13, F14 and compared to the single chain format (F1), and an anti-NKp46 antibody as a full-length human IgG1.

Bispecific proteins at 1 µg/mL were captured onto Protein-A chip and recombinant human NKp46 proteins were injected at 5 µg/mL over captured bispecific antibodies. For blank subtraction, cycles were performed again replacing NKp46 proteins with running buffer.

### Affinity study

Monovalent affinity study was conducted following a regular Capture-Kinetic protocol recommended by the manufacturer (Biacore GE Healthcare kinetic wizard). Seven serial dilutions of human NKp46 recombinant proteins, ranging from 6.25 to 400 nM were sequentially injected over the captured Bi-Specific antibodies and allowed to dissociate for 10 min before regeneration. The entire sensorgram sets were fitted using the 1:1 kinetic binding model.

### Results

SPR showed that the bispecific polypeptides of formats F1, F5, F6, F9, F10, F11, F13, F14 retained binding to NKp46. Monovalent affinities and kinetic association and dissociation rate constants are shown below in the Table 3 below.

**Table 3**

| Bispecific mAb | ka (1/Ms) | kd (1/s) | KD (M) |
|---|---|---|---|
| CD19-F1-NKp46-3 | 7.05E+04 | 6.44E-04 | 9.14E-09 |
| CD19-F5-NKp46-3 | 7.555E+4 | 0.00510 | 67E-09 |
| CD19-F6-NKp46-3 | 7.934E+4 | 0.00503 | 63E-09 |
| CD19-F9A-NKp46-3 | 2.070E+5 | 0.00669 | 32E-09 |
| CD19-F10A-NKp46-3 | 2.607E+5 | 0.00754 | 29E-09 |
| CD19-F11A-NKp46-3 | 3.388E+5 | 0.01044 | 30E-09 |
| CD19-F13-NKp46-3 | 8.300E+4 | 0.00565 | 68E-09 |
| CD19-F14-NKp46-3 | 8.826E+4 | 0.00546 | 62E-09 |
| NKp46-3 IgG1 | 2.224E+5 | 0.00433 | 20E-09 |

### Example 6: Engagement of NK cells against Daudi tumor target with Fc-containing NKp46 x CD19 bispecific protein

Bispecific antibodies having a monomeric Fc domain and a domain arrangement according to the single chain F1 or dimeric F2 formats described in Example 3, and a NKp46 binding region based on different anti-NKp46 variable domains (NKp46-1, NKp46-2, NKp46-3 or NKp46-4) were tested for functional ability to direct NK cells to lyse CD19-positive tumor target cells (Daudi, a well characterized B lymphoblast cell line). The F2 proteins additionally included variable regions of a further antibody (NKp46-9) which lost binding to NKp46 in the scFv format but which retained binding in the F(ab)-like format of F2.

Briefly, the cytolytic activity of each of (a) resting human NK cells, and (b) human NK cell line KHYG-1 transfected with human NKp46, was assessed in a classical 4-h ⁵¹Cr-release assay in U-bottom 96 well plates. Daudi cells were labelled with ⁵¹Cr (50 µCi (1.85 MBq)/1 x 10⁶ cells), then mixed with KHYG-1 transfected with hNKp46 at an effector/target ratio equal to 50 for KHYG-1, and 10 (for F1 proteins) or 8.8 (for F2 proteins) for resting NK cells, in the presence of monomeric bi-specific antibodies at different concentrations. After brief centrifugation and 4 hours of incubation at 37°C, samples of supernatant were removed and transferred into a LumaPlate (Perkin Elmer Life Sciences, Boston, MA), and ⁵¹Cr release was measured with a TopCount NXT beta detector (PerkinElmer Life Sciences, Boston, MA). All experimental conditions were analyzed in triplicate, and the percentage of specific lysis was determined as follows: 100 x (mean cpm experimental release - mean cpm spontaneous release)/ (mean cpm total release - mean cpm spontaneous release). Percentage of total release is obtained by lysis of target cells with 2% Triton X100 (Sigma) and spontaneous release corresponds to target cells in medium (without effectors or Abs).

### Results

In the KHYG-1 hNKp46 NK experimental model, each bi-specific antibody NKp46-1, NKp46-2, NKp46-3, NKp46-4 or NKp46-9 induced specific lysis of Daudi cells by human KHYG-1 hNKp46 NK cell line compared to negative controls (Human IgG1 isotype control (IC) and CD19/CD3 bi-specific antibodies), thereby showing that these antibodies induce Daudi target cell lysis by KHYG-1 hNKp46 through CD19/NKp46 cross-linking.

When resting NK cells were used as effectors, each bi-specific antibody NKp46-1, NKp46-2, NKp46-3, NKp46-4 or NKp46-9 again induced specific lysis of Daudi cells by human NK cells compared to negative control (Human IgG1 isotype control (IC) antibody), thereby showing that these antibodies induce Daudi target cell lysis by human NK cells through CD19/NKp46 cross-linking. Rituximab (RTX, chimeric IgG1) was used as a positive control of ADCC (Antibody-Dependent Cell Cytotoxicity) by resting human NK cells. The maximal response obtained with RTX (at 10 µg/ml in this assay) was 21.6% specific lysis illustrating that the bispecific antibodies have high target cell lysis activity. Results for experiments with resting NK cells are shown in **Figure 3A** for the single chain F1 proteins and **3B** for the dimeric F2 proteins.

### Example 7: Comparison with full length anti-NKp46 mAbs and depleting anti-tumor mAbs: only NKp46 x CD19 bispecific proteins prevent non-specific NK activation

In these experiments bispecific antibodies possessing a specific bispecific format were produced in order to assess whether such bispecific antibodies can mediate NKp46-mediated NK activation toward cancer target cells without triggering non-specific NK cell activation.

Particularly, NKp46 x CD19 bispecific proteins having an arrangement according to the F2 format described in Example 3 with anti-NKp46 variable domains from NKp46-1, NKp46-2, NKp46-3, NKp46-4 or NKp46-9 were compared to:
(a) full-length monospecific anti-NKp46 antibodies (NKp46-3 as human IgG1), and
(b) the anti-CD19 antibody as a full-length human IgG1 as ADCC inducing antibody control comparator.

The experiments further included as controls: rituximab, an anti-CD20 ADCC inducing antibody control for a target antigen with high expression levels; anti-CD52 antibody alemtuzumab, a human IgG1, binds CD52 target present on both targets and NK cells; and negative control isotype control therapeutic antibody (a human IgG1 that does not bind a target present on the target cells (HUG1-IC).

The different proteins were tested in order to assess their relative functional effects on NK cell activation in the presence of CD19-positive tumor target cells (Daudi cells), in the presence of CD19-negative, CD16-positive target cells (HUT78 T-lymphoma cells), and in the absence of target cells.

Briefly, NK activation was tested by assessing CD69 and CD107 expression on NK cells by flow cytometry. The assay was carried out in 96 U well plates in completed RPMI, 150µL final/well. Effector cells were fresh NK cells purified from donors. Target cells were Daudi (CD19-positive), HUT78 (CD19-negative) or K562 (NK activation control cell line). In addition to K562 positive control, three conditions were tested, as follows:
> NK cell alone
> NK cells vs Daudi (CD19+)
> NK cells vs HUT78 (CD19-)

Effector :Target (E :T) ratio was 2.5 : 1 (50 000 E : 20 000 T), with an antibody dilution range starting to 10 µg/mL with 1/4 dilution (n=8 concentrations). Antibodies, target cells and effector cells were mixed; spun 1 min at 300g; incubated 4h at 37°C; spun 3 min at 500g; washed twice with Staining Buffer (SB); added 50µL of staining Ab mix; incubated 30 min at 300g; washed twice with SB resuspended pellet with CellFix ; stored overnight at 4°C; and fluorescence detected with Canto II (HTS).

### Results

### 1. NK cells alone

Results of these experiments are shown in **Figure 4A****.** In the absence of target-antigen expressing cells, none of the bispecific anti-NKp46 x anti-CD19 antibodies (including each of the NKp46-1, NKp46-2, NKp46-3, NKp46-4 and NKp46-9 variable regions) activated NK cells as assessed by CD69 or CD107 expression. The full-length anti-CD19 also did not activate NK cells. However, the full-length anti-NKp46 antibodies did cause detectable activation of NK cells. Alemtuzumab also induced activation of NK cells, at a very high level. The isotype control antibody did not induce activation.

### 2. NK cells vs Daudi (CD19⁺)

Results of these experiments are shown in **Figure 4B****.** In the presence of target-antigen expressing cells, each of the bispecific anti-NKp46 x anti-CD19 antibodies (including each of the NKp46-1, NKp46-2, NKp46-3, NKp46-4 and NKp46-9 binding domains) activated NK cells. The full-length anti-CD19 antibody showed at best only very low activation of NK cells. Neither full-length anti-NKp46 antibodies nor alemtuzumab showed a substantial increase in activation beyond what was observed in presence of NK cells alone. The data in **Figure 4** shows that full-length anti-NKp46 antibodies elicited a similar level of baseline activation as was observed in the presence of NK cells alone. Alemtuzumab also induced the activation of NK cells at a similar level of activation to what was observed in the presence of NK cells alone, and at higher antibody concentrations in this setting (ET 2.5 : 1) the activation was greater than with the bispecific anti-NKp46 x anti-CD19 antibody. The isotype control antibody did not induce activation.

### 3. NK cells vs HUT78 (CD19-)

Results of these experiments are shown in **Figure 4C****.** In the presence of target-antigen-negative HUT78 cells, none of the bispecific anti-NKp46 x anti-CD19 antibody (including each of the NKp46-1, NKp46-2, NKp46-3, NKp46-4 and NKp46-9 variable regions) activated NK cells. However, the full-length anti-NKp46 antibodies and alemtuzumab caused detectable activation of NK cells at a similar level observed in presence of NK cells alone. Isotype control antibody did not induce activation.

The foregoing results indicate that the inventive bispecific anti-NKp46 proteins are able to activate NK cells in a target-cell specific manner, unlike full-length monospecific anti-NKp46 antibodies and further unlike full-length antibodies of depleting IgG isotypes which also activate NK cells in the absence of target cells. The NK cell activation achieved with anti-NKp46 bispecific proteins remarkably was higher than that observed with full length anti-CD19 IgG1 antibodies. Therefore these bispecific antibodies should elicit less non-specific cytotoxicity and may be more potent when used in therapy.

### Example 8: Comparative efficacy with depleting anti-tumor mAbs: NKp46 x CD19 bispecific proteins at low ET ratio

These studies aimed to investigate whether bispecific antibodies can mediate NKp46-mediated NK cell activation toward cancer target cells at lower effector:target ratios. The ET ratio used in this Example was 1:1 which is believed to be closer to the setting that would be encountered in vivo than the 2.5:1 ET ratio used in Example 7 or the 10:1 ET ratio of Example 6.

NKp46 x CD19 bispecific proteins having an arrangement according to the F2 format described in Example 3 with anti-NKp46 variable domains from NKp46-1, NKp46-2, NKp46-3, NKp46-4 or NKp46-9 were compared to:
(a) full-length monospecific anti-NKp46 antibodies (NKp46-3 as human IgG1), and
(b) the anti-CD19 antibody as a full-length human IgG1 as ADCC inducing antibody control comparator.

The experiments further included as controls: rituximab (an anti-CD20 ADCC inducing antibody control for a target antigen with high expression levels); anti-CD52 antibody alemtuzumab (a human IgG1, binds CD52 target present on both targets and NK cells), and negative control isotype control therapeutic antibody (a human IgG1 that does not bind a target present on the target cells (HUG1-IC). The different proteins were tested for functional effect on NK cell activation as assessed by CD69 or CD107 expression in the presence of CD19-positive tumor target cells (Daudi cells), in the presence of CD19-negative, CD16-positive target cells (HUT78 T-lymphoma cells), and in the absence of target cells. The experiments were carried out as in Example 7 except that the ET ratio was 1:1.

### Results

### Results

The results of the above experiments are shown in Figure 5 (5A: CD107 and 5B: CD69). In the presence of target-antigen expressing cells, each of the bispecific anti-NKp46 x anti-CD19 antibodies (respectively including NKp46-1, NKp46-2, NKp46-3, NKp46-4 or NKp46-9 variable regions) activated NK cells in the presence of Daudi cells.

The activation induced by bispecific anti-NKp46 x anti-CD19 antibody in the presence of Daudi cells was far more potent than that elicited by the full-length human IgG1 anti-CD19 antibody. This ADCC inducing antibody had low activity in this setting. Furthermore, in this low E:T ratio setting the activation induced by the bispecific anti-NKp46 x anti-CD19 antibody was as potent as the anti-CD20 antibody rituximab, with a difference being observed only at the highest concentrations that were 10 fold higher than concentrations in which differences were observed at the 2.5:1 ET ratio.

In the absence of target cells or in the presence of target antigen-negative HUT78 cells, full-length anti-NKp46 antibodies and alemtuzumab showed a similar level of baseline activation as was observed in the presence of Daudi cells. Anti-NKp46 x anti-CD19 antibody did not activate NK cells in presence of HUT78 cells.

The foregoing results indicate that the bispecific anti-NKp46 proteins of the invention are able to activate NK cells in a target-cell specific manner and at lower effector: target ratios and are more effective in mediating NK cell activation that traditional human IgG1 antibodies.

### Example 9:

### Mechanism of action studies

NKp46 x CD19 bispecific proteins having an arrangement according to the F2, F3, F5 or F6 formats described in Examples 3 or 4 with anti-NKp46 variable domains from NKp46-3 were compared to rituximab (anti-CD20 ADCC inducing antibody), and to a human IgG1 isotype control antibody for their functional ability to direct CD16-/NKp46+ NK cell lines to lyse CD19-positive tumor target cells.

Briefly, the cytolytic activity of the CD16-/NKp46+ human NK cell line KHYG-1 was assessed in a classical 4-h ⁵¹Cr-release assay in U-bottom 96 well plates. Daudi or B221 cells were labelled with ⁵¹Cr (50 µCi (1.85 MBq)/1 x 10⁶ cells), then mixed with KHYG-1 at an effector/target ratio equal to 50:1, in the presence of test antibodies at dilution ranges starting from 10⁻⁷ mol/L with 1/5 dilution (n=8 concentrations).

After a brief centrifugation and 4 hours of incubation at 37°C, 50µL of the supernatant were removed and transferred into a LumaPlate (Perkin Elmer Life Sciences, Boston, MA), and ⁵¹Cr release was measured with a TopCount NXT beta detector (PerkinElmer Life Sciences, Boston, MA). All experimental conditions were analyzed in triplicate, and the percentage of specific lysis was determined as follows: 100 x (mean cpm experimental release - mean cpm spontaneous release)/ (mean cpm total release - mean cpm spontaneous release). Percentage of total release is obtained by lysis of target cells with 2% Triton X100 (Sigma) and spontaneous release corresponds to target cells in medium (without effectors or Abs).

### Results

The results of the above experiments are shown in **Figure 6A** (KHYG-1 vs Daudi) and 6B (KHYG-1 vs B221). In the KHYG-1 hNKp46 NK experimental model, each NKp46 x CD19 bispecific protein (Format F2, F3, F5 and F6) induced specific lysis of Daudi or B221 cells by human KHYG-1 hNKp46 NK cell line, while rituximab and the human IgG1 isotype control (IC) antibodies did not.

### Example 10 Anti-KIR3DL2 bispecific proteins

Bispecific proteins targeting human KIR3DL2 (KIR3DL2 x NKp46 bispecific) were constructed as F6 formats and tested for activity. KIR3DL2 (CD158k; killer cell immunoglobulin like receptor, three Ig domains and long cytoplasmic tail 2) is a disulphide-linked homodimer of three-Ig domain molecules of about 140 kD, described in Pende et al. (1996) J. Exp. Med. 184: 505-518. Several allelic variants have been reported for KIR3DL2 polypeptides, each of these are encompassed by the term KIR3DL2. The amino acid sequence of the mature human KIR3DL2 (allele *002) is shown in Genbank accession no. AAB52520. Briefly, the cytolytic activity of NK cells from Buffy coat from donors was assessed in a classic 4-h ⁵¹Cr-release assay in U-bottom 96 well plates. HUT78 tumor cells (CTCL) that express KIR3DL2 were labelled with ⁵¹Cr, then mixed with NK cells at an effector/target ratio equal to 10:1 (25 000:2500), in the presence of test antibodies at dilution ranges starting from 10 µg/mL (or 100 µg/mL) with 1/10 dilution (n=8). Assays were in cRPMI, 150 µL final/well, in triplicates.

Results are shown in **Figure 6C****.** Despite its Fc domain not binding to CD16 in this format, the F6 protein structure produced as an NKp46 x KIR3DL2 bispecific protein surprisingly exhibited comparable ability to lyse target cells as a known anti-KIR3DL2 human IgG1 antibody that contained the same variable regions and which binds KIR3DL2 bivalently.

### Example 11 Effect of intrachain domain motion within multimeric proteins

It was theorized by the inventors that the ability of NKp46 bispecific proteins to promote NKp46-mediated lysis of target cells may be affected by the distance between the two antigen binding domains in the bispecific protein which may impact the ability of one or both of the NKp46 antigen binding domain and the antigen binding domain which interacts with an antigen of interest to interact with their respective targets. Also, it was further theorized that NKp46 mediated lysis of target cells may be impacted by the structure of the two antigen binding domains and/or their respective conformation, freedom of motion or flexibility which may be impacted by the structure of the two antigen binding domains as well as the manner by which they are associated with each other, e.g., by a linker peptide and its particular length and chemical composition. Particularly, it was theorized that a lytic NKp46-target cell synapse may vary as a function of the size and structure of the bispecific protein. Therefore, the inventors posited that bispecific proteins wherein the antigen binding domains are in a format whereby the antigen binding domains more closely mimics or approximates the conformation, spacing and flexibility of the antigen binding domains of

This was theorized because conformational flexibility, notably intrachain domain motion or movement, may for example affect the effective distance between NKp46 and antigen-of-interest binding sites, which in turn might have an effect on the NKp46-target cell synapse and the ability of a multimeric bispecific protein to mediate NKp46-mediated signaling and lysis. Based on these suppositions the inventors evaluated the lytic function of multimeric proteins of different bispecific protein formats and which comprise more or less freedom of motion of the antigen binding domains based on the structure of the antigen binding sites and the specific linkers separating these antigen binding sites.

Specifically, different NKp46 x tumor antigen bispecific proteins of different formats such as the F3, F4, F9, F10 and F11 format that bound different tumor antigens were evaluated for their relative ability to induce NKp46- mediated lysis of tumor target cells by KHYG-1 NK cells (NKp46⁺CD16⁻). F5 and F6 bispecific protein formats have distances between the NKp46 binding site and the antigen of interest binding site that are less than that of full-length antibodies. By contrast bispecific proteins targeting human CD19 (CD19 x NKp46 bispecific) in F9 format have binding sites that are spaced farther apart, similar to distances in the two binding sites in conventional full-length antibodies. Bispecific proteins were therefore constructed as F9 formats and compared to F10 and F11 formats. Structurally speaking, format F9, F10 and F11 are very close to one another, however formats F10 and F11 are characterized by one antigen binding domain with a Fab structure and the other antigen binding domain with a tandem variable domain structure (two variable domains separated by a flexible linker). F10 and F11 therefore have greater intrachain domain motion and/or less local steric hindrance, as well as possibly less distance between binding sites than in the F9 proteins.

The cytolytic activity of the CD16-/NKp46+ human NK cell line KHYG-1 was assessed in a classical 4-h ⁵¹Cr-release assay in U-bottom 96 well plates. Daudi or B221 cells were labelled with ⁵¹Cr (50 µCi (1.85 MBq)/1 x 10⁶ cells), then mixed with KHYG-1 at an effector/target ratio equal to 50:1, in the presence of test antibodies at dilution range starting from 10⁻⁷ mol/L with 1/5 dilution (n=8 concentrations). The results showed that formats F10 and F11 were both more potent than format F9 in inducing Daudi cell lysis by NK cells. As noted above F9 format proteins have distances between the NKp46 binding site and the antigen of interest binding site which is similar to full-length antibodies or about 80 Å, and the F10 and F11 proteins comprise a single chain domain connected to the Fc by a flexible linker and have substantially less than 80Å between the antigen binding sites (in the case of F10, about 55 Å).

Based thereon we studied the effects of even further shortened distances between the NKp46 and antigen of interest binding domains using other CD19 x NKp46 bispecific proteins. In these experiments F3, F4 protein formats were selected for comparison with protein formats F10 and F11. Each of these proteins have distances between antigen binding sites of less than 80Å, however, F3 and F4 are shorter than F10 and F11, and F3 and F4 have distances between antigen binding sites that are equivalent to F11 but 25Å less than that of F10. The results of these experiments indicated that the F3, F4, F10 and F11 formats did not significantly differ in their ability to induce Daudi cell lysis by NK cells. These results would suggest that there may be an optimal minimal spacing between the antigen binding domains that improves potency and/or that potency is affected by a combination of the spacing between the antigen binding domains and the flexibility and/or conformation of the antigen binding domains.

### Example 12: Combining NKp46 and CD16 triggering

NKp46 x CD19 bispecific proteins that bind human CD16 having an arrangement according to the F5 format with anti-NKp46 variable domains from NKp46-3 were compared to the same bispecific antibody in a F6 format (which lacks CD16 binding), and to a human IgG1 isotype anti-CD19 antibody, as well as to a human IgG1 isotype control antibody for functional ability to direct purified NK cells to lyse CD19-positive Daudi tumor target cells.

Briefly, the cytolytic activity of fresh human purified NK cells from EFS Buffy Coat was assessed in a classical 4-h ⁵¹Cr-release assay in U-bottom 96 well plates. Daudi or HUT78 cells (negative control cells that do not express CD19) were labelled with ⁵¹Cr and then mixed with NK cells at an effector/target ratio equal to 10:1, in the presence of test antibodies at dilution range starting from 10 µg/ml with 1/10 dilution (n=8 concentrations).

After brief centrifugation and 4 hours of incubation at 37°C, 50µL of supernatant were removed and transferred into a LumaPlate (Perkin Elmer Life Sciences, Boston, MA), and ⁵¹Cr release was measured with a TopCount NXT beta detector (PerkinElmer Life Sciences, Boston, MA). All experimental conditions were analyzed in triplicate, and the percentage of specific lysis was determined as follows: 100 x (mean cpm experimental release - mean cpm spontaneous release)/ (mean cpm total release - mean cpm spontaneous release). Percentage of total release is obtained by lysis of target cells with 2% Triton X100 (Sigma) and spontaneous release corresponds to target cells in medium (without effectors or Abs).

The results of these experiments are shown in **Figure 7****.** The CD19-F6-NKp46 (bispecific protein in F6 format) whose Fc domain does not bind CD16 due to a N297 substitution was as potent in mediating NK cell lysis of Daudi target cells as the full-length IgG1 anti-CD19 antibody. This result is remarkable especially considering that the control IgG1 anti-CD19 antibody binds CD19 bivalently and further since the anti-CD19 antibody is bound by CD16. The F6 protein was also compared to a protein CD19-F5-NKp46 that was identical to the CD19-F6-NKp46 protein with the exception of an asparagine at Kabat residue 297. Surprisingly, despite the strong NK activation mediated by CD16 triggering by the CD19-F5-NKp46 (F5 format protein) whose Fc domain binds CD16, the F5 format was far more potent in mediating Daudi target cell lysis that the full-length IgG1 anti-CD19 antibody or the F6 format bispecific protein. This would suggest that NKp46 can enhance target cell lysis even when CD16 is triggered. In fact, at comparable levels of target cell lysis, the CD19-F5-NKp46 was at least 1000 times more potent than the full-length anti-CD19 IgG1. These potency results would suggest that the inventive multispecific NKp46 antibodies should be well suited for use in human therapy, e.g., in treating cancer or infectious diseases.

### Example 13: Mechanisms of action of CD16-binding NKp46 x CD19 bispecific

Lysis of Daudi cells by NKp46 x CD19 bispecific F5 and F6 were compared to a conventional human IgG1 antibody. As a control, lysis was also tested on HUT78 cells that lack CD19; positive control for HUT78 cell lysis was an anti-KIR3DL2 of human IgG1 isotype (HUT78 are KIR3DL2-positive). Cytotoxicity assays were carried out as in Example 10. Flow cytometry staining of NK cell surface markers was carried out as in Example 7.

Results for the cytotoxicity assays are shown in **Figure 8** (Daudi cell in the right hand panel and HUT78 cells in the left hand panel). the CD19-F6-NKp46-3 whose Fc domain does not bind CD16 due to a N297 substitution has as mode of action NKp46 triggering when NK cells encounter the target cell, while the CD19-F5-NKp46-3 bispecific protein demonstrated a far higher potency in mediating cytotoxicity toward Daudi cells. Neither the F5 nor the F6 protein mediated any NK cell cytotoxicity towards HUT78 cells.

The results of flow cytometry staining of NK cell surface markers showed a strong upregulation of CD137 on the surface of NK cells by F5 proteins. These results are shown in **Figure 9** (Left-most panel: NK cells vs. Daudi; middle panel: NK cells vs. HUT78; right-most panel: NK cells alone). The CD19-F5-NKp46-3 whose Fc domain binds CD16 demonstrated the highest CD137 upregulation. The full-length anti-CD19 IgG1 antibody that binds CD16 also elicited CD137 upregulation, but to a far lesser extent than CD19-F5-NKp46-3. The CD19-F6-NKp46-3 which functions via NKp46 but not via CD16 did not elicit any detectable CD137 upregulation. It is hypothesized that the remarkable potency of the F5 format may arise from a particularly strong CD137 upregulation on NK cells which may be mediated by the dual targeting of NKp46 and CD16.

### Example 14: Fc-engineered CD16-binding NKp46 x CD20 bispecific

New bispecific proteins were further constructed in an attempt to generate an agent that could improve on the most potent new generation of Fc enhanced antibodies. In these experiments as the comparison antibody we selected the commercial antibody GA101 (GAZYVA@, Gazyvaro^{®}, obinutuzumab, Roche Pharmaceuticals), which is an Fc-modified human IgG1 antibody having enhanced CD16A binding as a result of hypofucosylated N-linked glycosylation.

NKp46 x CD20 bispecific proteins were produced as proteins without CD16 binding (F6 format), with CD16 binding (F5 format), or as Fc-engineered format based on F5 but comprising two amino acid substitutions in the CH2 domain of the heavy chain that increase binding affinity for human CD16A (referred to as "F5+"). In these constructs the anti-CD20 ABDs comprise the V_{H} and V_{L} of GA101.

Lysis of Daudi cells by NKp46 x CD20 bispecific F5, F5+ and F6 antibodies were compared to the commercial antibody GA101 (GAZYVA^{®}). Cytotoxicity assays were carried out as in Example 10.

Results for the cytotoxicity assays are shown in **Figure 10****.** As shown therein the GA101-F5+-NKp46-1 bispecific protein demonstrated a far higher potency (approximately 10-fold increase in EC₅₀) in mediating cytotoxicity toward Daudi cells that GA101.

Moreover, when ADCC optimized Fc are used for the bispecific format (F5⁺) a significant difference was observed between F5+-BS lacking the Nkp46 arm (GA101-F5+-IC; black diamond) and F5+-BS co-engaging CD16+NKp46 (GA101-F5⁺-NKp46-1; black square) confirming the contribution of NKp46 in GA101-F5⁺-NKp46-1 activity. Surprisingly, despite the high affinity of GA101-F5⁺-NKp46-1 for CD16 and the presumable maximum NK-cell mediated lysis, NKp46 nevertheless elicited a substantial further increase in cytotoxic activity. These results would suggest that agents capable of inducing ADCC via CD16, can be improved by further conferring on them the ability to induce NKp46-mediated lysis, and also that the potency of bispecific anti-NKp46 agents can be improved by enhancing affinity for CD16 via Fc engineering.

### Example 15: Binding of different bispecific formats to FcRn

The affinity of different antibody formats for human FcRn was studied by Surface Plasmon Resonance (SPR) by immobilizing recombinant FcRn proteins covalently to carboxyl groups in the dextran layer on a Sensor Chip CM5, as described in Example 2-6.

A chimeric full length anti-CD19 antibody having intact human IgG1 constant regions and NKp46 x CD19 bispecific proteins having an arrangement according to the F3, F4, F5, F6, F9, F10, F11, F13 or F14 formats described in Examples 3 or 4 with anti-NKp46 variable domains from NKp46-3 (NKp46-2 for F2) were tested; for each analyte, the entire sensorgram was fitted using the steady state or 1:1 SCK binding model.

The results of these experiments are shown in Table 4 below. The bispecific proteins having dimeric Fc domains (formats F5, F6, F13, F14) bound to FcRn with affinity similar to that of the full-length IgG1 antibody. The bispecific proteins with monomeric Fc domains (F3, F4, F9, F10, F11) also displayed binding affinity to FcRn, however with lower affinity that the bispecific proteins having dimeric Fc domains.

**Table 4**

| **Antibody/Bispecific** | **SPR method** | **KD nM** |
|---|---|---|
| Human IgG1/K Anti-CD19 | SCK / Two state reaction | 7.8 |
| CD19-F5-NKp46-3 | SCK / Two state reaction | 2.6 |
| CD19-F6- NKp46-3 | SCK / Two state reaction | 6.0 |
| CD19-F13- NKp46-3 | SCK / Two state reaction | 15.2 |
| CD19-F14- NKp46-3 | SCK / Two state reaction | 14.0 |
| CD19-F3- NKp46-3 | Steady State | 474.4 |
| CD19-F4- NKp46-3 | Steady State | 711.7 |
| CD19-F9A- NKp46-3 | Steady State | 858.5 |
| CD19-F10A- NKp46-3 | Steady State | 432.8 |
| CD19-F11- NKp46-3 | Steady State | 595.5 |

### Example 16: Binding to FcγR

Different multimeric Fc proteins were evaluated to assess whether such bispecific monomeric Fc proteins could retain binding to Fcy receptors.

SPR measurements were performed on a Biacore T100 apparatus (Biacore GE Healthcare) at 25°C. In all Biacore experiments HBS-EP+ (Biacore GE Healthcare) and 10 mM NaOH, 500mM NaCl served as running buffer and regeneration buffer respectively. Sensorgrams were analyzed with Biacore T100 Evaluation software. Recombinant human FcR's (CD64, CD32a, CD32b, CD16a and CD16b) were cloned, produced and purified.

F5 and F6 bispecific antibodies CD19-F5-NKp46-3 or CD19-F6-NKp46-3 were immobilized covalently to carboxyl groups in the dextran layer on a Sensor Chip CM5. The chip surface was activated with EDC/NHS (N-ethyl-N'-(3-dimethylaminopropyl) carbodiimidehydrochloride and N-hydroxysuccinimide (Biacore GE Healthcare)). Bispecific antibodies were diluted to 10 µg/ml in coupling buffer (10 mM acetate, pH 5.6) and injected until the appropriate immobilization level was reached (i.e. 800 to 900 RU). Deactivation of the remaining activated groups was performed using 100 mM ethanolamine pH 8 (Biacore GE Healthcare).

Monovalent affinity study was assessed following a classical kinetic wizard (as recommended by the manufacturer). Serial dilutions of soluble analytes (FcRs) ranging from 0.7 to 60 nM for CD64 and from 60 to 5000 nM for all the other FcRs were injected over the immobilized bispecific antibodies and allowed to dissociate for 10 min before regeneration. The entire sensorgram sets were fitted using the 1:1 kinetic binding model for CD64 and with the Steady State Affinity model for all the other FcRs.

The results showed that while full length wild type human IgG1 bound to all cynomolgus and human Fcy receptors, the CD19-F6-NKp46-3 bi-specific antibodies did not bind to any of the receptors. The CD19-F5-NKp46-3, on the other hand, bound to each of the human receptors CD64 (KD=0.7 nM), CD32a (KD=846 nM), CD32b (KD=1850 nM), CD16a (KD=1098 nM) and CD16b (KD=2426 nM). Conventional human anti-lgG1 antibodies have comparable binding to these Fc receptors (KD shown in the table below).

| Human Fcγ receptor | CD19-F5-NKp46-3 **KD (nM)** | Full length human IgG1 antibody **KD (nM)** |
|---|---|---|
| CD64 | 0.7 | 0.24 |
| CD32a | 846 | 379 |
| CD32b | 1850 | 1180 |
| CD16a | 1098 | 630 |
| CD16b | 2426 | 2410 |

### Example 17: Improved product profile and yield of different bispecific formats compared to existing formats

Blinatumomab and two bispecific antibodies having NKp46 and CD19 binding regions based on F1 to F17 formats and NKp46-3, and blinatumomab, respectively were cloned and produced under format 6 (F6), DART and BITE formats following the same protocol and using the same expression system. F6, DART and BITE bispecific proteins were purified from cell culture supernatant by affinity chromatography using prot-A beads for F6 or Ni-NTA beads for DART and BITE. Purified proteins were further analyzed and purified by SEC. BITE and DART showed a very low production yield compared to F6 and the purified proteins have a very complex SEC profile. DART and BITE are barely detectable by SDS-PAGE after Coomassie staining in the expected SEC fractions (3 and 4 for BITE and 4 and 5 for DART), whereas the F6 format showed a clear and simple SEC and SDS-PAGE profiles with a major peak (fraction 3) containing the multimeric bispecific proteins. The major peak for the F6 format corresponded to about 30% of the total proteins. These results are consistent for those seen with the F1 to F17 proteins (data not shown) indicating that the Fc domain (or Fc-derived domain) present in those formats facilitates the production and improves the quality and solubility of bispecific proteins.

Moreover, the Fc domains present in proteins F1 to F17 have the advantage of being suitable for usage in affinity chromatography without the need for the incorporation of peptide tags. This is desirable as such tags are undesirable in a therapeutic product as they may potentially elicit undesired immunogenicity. By contrast, BiTe and DART antibodies cannot be purified using protein A, whereas F1 to F17 antibodies are all bound by protein A. Table 6 below shows the productivity of different formats.

| **Format** | **SEC** | **SDS PAGE** | | **Final « productivity » yield** |
|---|---|---|---|---|
| | | **Reduced** | **Non Reduced** | |
| F5 | √ | √ | √ | 37mg/L |
| F6 | √ | √ | √ | 12mg/L |
| F7 | √ | √ | √ | 11mg/L |
| F8C | √ | √ | √ | *3,7mg*/*L* |
| F9A | √ | √ | √ | 8,7mg/L |
| F9B | √ | √ | √ | *3,0mg*/*L* |
| F10A | √ | √ | √ | 2,0mg/L |
| F11 | √ | √ | √ | 2,0mg/L |
| F12 | √ | √ | √ | *2,8mg*/*L* |
| F13 | √ | √ | √ | 6,4mg/L |
| F14 | √ | √ | √ | 2,4mg/L |
| F15 | √ | √ | √ | 0,9mg/L |
| BiTe | - | - | - | - |
| DART | - | - | - | - |

## Claims

1. A multispecific protein consisting of three polypeptide chains, each chain comprise a variable domain fused to a CH1 or Cκ domain (a V-(CH1/Cκ) unit), wherein a first (central) chain comprises two V-(CH1/Cκ) units and a human Fc domain interposed between the units, the second chain comprises one V-(CH1/Cκ) unit and a human Fc domain, and the third chain comprises one V-(CH1/Cκ) unit, wherein one of the V-(CH1/Cκ) units of the central chain is bound, by CH1-Cκ dimerization, to the V-(CH1/Cκ) unit of the second chain thereby forming a first antigen binding domain and a dimeric Fc domain, and wherein the other of the V-(CH1/Cκ) units of the central chain is bound, by CH1-Cκ dimerization, to the V-(CH1/Cκ) unit of the third chain thereby forming a second antigen binding domain, and wherein the Fc domain comprises N-linked glycosylation at residue N297 (Kabat EU numbering) and binds to a human CD16 polypeptide,
wherein the multispecific protein is a trimer with a dimeric Fc domain having the domain arrangement: 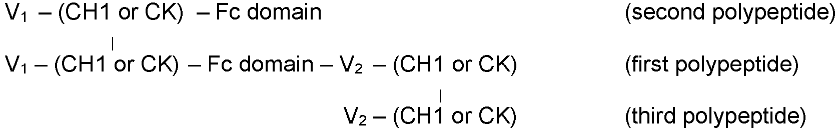
wherein one V₁ is a light chain variable domain and the other V₁ is a heavy chain variable domain, wherein one V₂ is a light chain variable domain and the other V₂ is a heavy chain variable domain, wherein the V₁ pair will form a first ABD, and the V₂ pair will form a second ABD and wherein an Fc domain is fused to a CK domain via a hinge region.

2. The protein of claim 1, wherein the Fc domain(s) comprises a human CH2 domain comprising an amino acid substitution to increase binding to a human Fcy receptor.

3. The protein of any of the above claims, wherein one ABD binds an activating receptor expressed at the surface of an effector cell, and one ABD binds a cancer, viral or bacterial antigen.

4. The protein of any of the above claims, wherein at least one ABD binds an a cancer, viral or bacterial antigen that is known to be capable of undergoing inducing or increase in intracellular internalization upon being bound by a full-length human IgG1 antibody.

5. A protein of any one of the above claims for use for the treatment of a cancer.

6. A method of making a heterotrimeric protein, comprising:
(a) providing a first nucleic acid encoding a first polypeptide chain according to claims 1-4;
(b) providing a second nucleic acid encoding a second polypeptide chain according to claims 1-4;
(c) providing a third nucleic acid comprising a third polypeptide chain according to claims 1-4; and
(d) expressing said first, second and third nucleic acids in a host cell to produce a protein comprising said first, second and third polypeptide chains, respectively; loading the protein produced onto an affinity purification support, optionally a Protein-A support, and recovering a heterotrimeric protein.

7. A method for identifying or evaluating a multimeric polypeptide, comprising the steps of:
(a) providing nucleic acids encoding the polypeptide chains of any of claims 1-4;
(b) expressing said nucleic acids in a host cell to produce said polypeptide chains, respectively; and recovering a multimeric protein comprising said polypeptide chains; and
(c) evaluating the polypeptide produced for a biological activity of interest.

## Patentansprüche

1. Multispezifisches Protein, bestehend aus drei Polypeptidketten, wobei jede Kette eine variable Domäne umfasst, die an eine CH1- oder C_{K}-Domäne (eine V-(CH1/C_{K})-Einheit) fusioniert ist, wobei eine erste (zentrale) Kette zwei V-(CH1/C_{K})-Einheiten und eine zwischen den Einheiten angeordnete menschliche Fc-Domäne, die zweite Kette eine V-(CH1/C_{K})-Einheit und eine menschliche Fc-Domäne umfasst und die dritte Kette eine V-(CH1/C_{K})-Einheit umfasst, wobei eine von den V-(CH1/C_{K})-Einheiten der zentralen Kette durch CH1-C_{K}-Dimerisierung an die V-(CH1/C_{K})-Einheit der zweiten Kette gebunden ist, wodurch sie eine erste Antigenbindungsdomäne und eine dimere Fc-Domäne bildet, und wobei die andere der V-(CH1/C_{K})-Einheiten der zentralen Kette durch CH1-C_{K}-Dimerisierung an die V-(CH1/C_{K})-Einheit der dritten Kette gebunden wird, wodurch sie eine zweite Antigenbindungsdomäne bildet, und wobei die Fc-Domäne eine N-verknüpfte Glykosylierung am Rest N297 (Kabat EU-Nummerierung) umfasst und an ein menschliches CO16-Polypeptid bindet,
wobei das multispezifische Protein ein Trimer mit einer dimeren Fe-Domäne ist, die die Domänen-Anordnung aufweist: 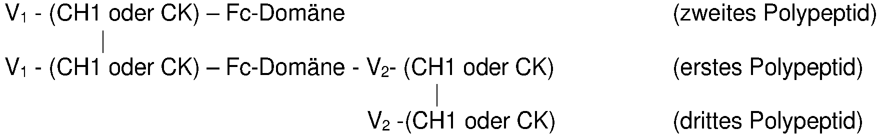
wobei ein V₁ eine variable Domäne der leichten Kette und das andere V₁ eine variable Domäne der schweren Kette ist, wobei ein V₂ eine variable Domäne der leichten Kette und das andere V₂ eine variable Domäne der schweren Kette ist, wobei das V₁ Paar einen ersten ABD bildet und das V₂ Paar einen zweiten ABD bildet und wobei eine Fc Domäne über eine Gelenkregion mit einer CK-Domäne fusioniert ist.

2. Das Protein nach Anspruch 1, wobei die Fc-Domäne(n) eine menschliche CH2-Domäne umfasst, die eine Aminosäuresubstitution umfasst, um die Bindung an einen menschlichen Fcγ-Rezeptor zu erhöhen.

3. Das Protein nach einem der oben genannten Ansprüche, wobei ein ABD einen aktivierenden Rezeptor bindet, der auf der Oberfläche einer Effektorzelle exprimiert wird, und ein ABD ein Krebs-, virales oder bakterielles Antigen bindet.

4. Das Protein nach einem der oben genannten Ansprüche, wobei mindestens ein ABD ein Krebs-, virales oder bakterielles Antigen bindet, von dem bekannt ist, dass es in der Lage ist, eine intrazelluläre Internalisierung zu induzieren oder zu steigern, wenn es an einen menschlichen IgG1-Antikörper voller Länge gebunden wird.

5. Das Protein nach einem der oben genannten Ansprüche zur Verwendung zur Behandlung von Krebs.

6. Verfahren zur Herstellung eines heterotrimeren Proteins, umfassend:
(a) Bereitstellen einer ersten Nukleinsäure, die eine erste Polypeptidkette gemäß den Ansprüchen 1-4 kodiert;
(b) Bereitstellen einer zweiten Nukleinsäure, die eine zweite Polypeptidkette gemäß den Ansprüchen 1-4 kodiert;
(c) Bereitstellen einer dritten Nukleinsäure, die eine dritte Polypeptidkette gemäß den Ansprüchen 1-4 umfasst; und
(d) Exprimieren dieser ersten, zweiten und dritten Nukleinsäure in einer Wirtszelle, um ein Protein zu produzieren, das diese erste, zweite bzw. dritte Polypeptidkette umfasst; Laden des produzierten Proteins auf einen Affinitätsreinigungsträger, optional einen Protein-A-Träger, und Gewinnen eines heterotrimeren Proteins.

7. Verfahren zur Identifizierung oder Bewertung eines multimeren Polypeptids, umfassend die Schritte:
(a) Bereitstellen von Nukleinsäuren, die die Polypeptidketten nach einem der Ansprüche 1-4 kodieren;
(b) Exprimieren dieser Nukleinsäuren in einer Wirtszelle, um diese Polypeptidketten jeweils zu produzieren; und Gewinnen eines multimeren Proteins, das diese Polypeptidketten umfasst; und
(c) Bewerten des Polypeptids, das im Hinblick auf eine interessierende biologische Aktivität produziert wurde.

## Revendications

1. Protéine multispécifique consistant en trois chaînes polypeptidiques, chaque chaîne comprenant un domaine variable fusionné à un domaine CH1 ou C_{κ} (un motif V-(CH1/C_{κ}), dans laquelle une première chaîne (centrale) comprend deux motifs V-(CH1/C_{κ}) et un domaine Fc humain interposé entre les motifs, la deuxième chaîne comprend un motif V-(CH1/C_{κ}) et un domaine Fc humain, et la troisième chaîne comprend un motif V-(CH1/C_{κ}), dans laquelle l'un des motifs V-(CH1/C_{κ}) de la chaîne centrale est lié, par dimérisation CH1-C_{κ}, au motif V-(CH1/C_{κ}) de la deuxième chaîne en formant ainsi un premier domaine se liant à un antigène et un domaine Fc dimère, et dans laquelle l'autre des motifs V-(CH1/C_{κ}) de la chaîne centrale est lié, par dimérisation CH1-C_{κ}, au motif V-(CH1/C_{κ}) de la troisième chaîne en formant ainsi un deuxième domaine se liant à un antigène, et dans laquelle le domaine Fc comprend une glycosylation N-liée au résidu N297 (numérotation Kabat EU) et se lie à un polypeptide de CD 16 humain,
laquelle protéine multispécifique est un trimère avec un domaine Fc dimère ayant l'agencement de domaines suivants : 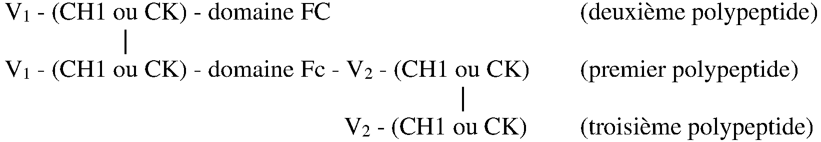
dans lequel un V₁ est un domaine variable de chaîne légère et l'autre V₁ est un domaine variable de chaîne lourde, dans lequel un V₂ est un domaine variable de chaîne légère et l'autre V₂ est un domaine variable de chaîne lourde, dans lequel la paire de Vi va former un premier ABD, et la paire de V₂ va former un deuxième ABD, et dans lequel un domaine Fc est fusionné à un domaine CK via une région de charnière.

2. Protéine selon la revendication 1, dans laquelle le ou les domaines Fc comprennent un domaine CH2 humain comprenant une substitution d'acide aminé pour augmenter la liaison à un récepteur Fcy humain.

3. Protéine selon l'une quelconque des revendications précédentes, dans laquelle un ABD se lie à un récepteur d'activation exprimé à la surface d'une cellule effectrice, et un ABD se lie à un antigène cancéreux, viral ou bactérien.

4. Protéine selon l'une quelconque des revendications précédentes, dans laquelle au moins un ABD se lie à un antigène cancéreux, viral ou bactérien qui est connu pour être capable de subir une induction ou pour augmenter l'internalisation intracellulaire après qu'il a été lié à un anticorps IgG1 humain de pleine longueur.

5. Protéine selon l'une quelconque des revendications précédentes, pour une utilisation dans le traitement d'un cancer.

6. Méthode de production d'une protéine hétérotrimère, comprenant :
(a) la fourniture d'un premier acide nucléique codant pour une première chaîne polypeptidique selon les revendications 1 à 4 ;
(b) la fourniture d'un deuxième acide nucléique codant pour une deuxième chaîne polypeptidique selon les revendications 1 à 4 ;
(c) la fourniture d'un troisième acide nucléique codant pour une troisième chaîne polypeptidique selon les revendications 1 à 4 ; et
(d) l'expression desdits premier, deuxième et troisième acides nucléiques dans une cellule hôte pour produire une protéine comprenant lesdites première, deuxième et troisième chaînes polypeptidiques, respectivement ; le chargement de la protéine produite sur un support de purification par affinité, éventuellement un support de protéine A, et la récupération d'une protéine hétérotrimère.

7. Méthode pour identifier ou évaluer un polypeptide multimère, comprenant les étapes de :
(a) fourniture d'acides nucléiques codant pour les chaînes polypeptidiques de l'une quelconque des revendications 1 à 4 ;
(b) expression desdits acides nucléiques dans une cellule hôte pour produire lesdites chaînes polypeptidiques, respectivement ; et récupération d'une protéine multimère comprenant lesdites chaînes polypeptidiques ; et
(c) évaluation du polypeptide produit pour une activité biologique présentant un intérêt.
